(19) **Europäisches Patentamt**
**European Patent Office**
**Office européen des brevets**

(11) **EP 4 613 871 A1**

(12) **EUROPEAN PATENT APPLICATION**

(43) Date of publication:
**10.09.2025   Bulletin 2025/37**

(21) Application number: **24177784.6**

(22) Date of filing: **23.05.2024**

(51) International Patent Classification (IPC):
**C12Q 1/6804** (2018.01)

(52) Cooperative Patent Classification (CPC):
(C-Sets available)
**C12Q 1/6804**                                              (Cont.)

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **07.03.2024   EP 24162157**

(71) Applicant: **Diagenode S.A.
4102 Seraing (BE)**

(72) Inventors:
• **SONDERMANN, Nathalie Allegra Faye
4102 Seraing (Ougree) (BE)**

• **PANTELEEVA, Irina
4102 Seraing (Ougree) (BE)**
• **CALAY, Damien Jean-Pol Bernard Ghislain
4102 Seraing (Ougree) (BE)**
• **SABATEL, Céline
4550 Villers-le-Temple (BE)**

(74) Representative: **Maiwald GmbH
Elisenhof
Elisenstraße 3
80335 München (DE)**

Remarks:
The complete document including Reference
Table(s) and the Sequence Listing(s) can be
downloaded from the EPO website

(54) **METHODS FOR CUT&TAG**

(57)     The present invention provides a novel method for determining at least one chromatin binding site of a chromatin-associated factor of interest in a cell, comprising:
(i) permeabilizing the cell;
(ii) contacting the permeabilized cell with a first antibody that specially binds the chromatin-associated factor of interest and with a second antibody that specifically binds to the first antibody;
(iii) contacting the permeabilized cell with a transposome that is linked to a specific binding agent that specifically binds the first and/or second antibody wherein the transposome comprises a transposase and a first and second DNA molecule;
(iv) activating the transposase, thereby excising a DNA segment comprising at least one chromatin binding site of a chromatin-associated factor of interest and tagging the DNA with the first and second DNA molecule;
(v) determining the sequence of the excised and tagged DNA segment; and
(vi) based on the determined sequence of the excised and tagged DNA segment, determining the at least one chromatin binding site of the chromatin-associated factor of interest in the cell;
wherein the steps of contacting the permeabilized cell with the transposome and activating the transposase are performed in presence a crowding agent.

EP 4 613 871 A1

(52) Cooperative Patent Classification (CPC): (Cont.)

C-Sets
**C12Q 1/6804, C12Q 2535/122, C12Q 2521/507,**
**C12Q 2521/301, C12Q 2525/191**

**Description**

**TECHNICAL FIELD**

The disclosure relates to improved methods that profile chromatin-associated proteins for their chromatin binding sites. The methods described herein allow for reliable analysis of a broad range of cell numbers and/or protein-chromatin interactions.

**BACKGROUND OF THE INVENTION**

Profiling of chromatin-associated proteins to their binding sites on chromatin is of major interest in basic research, applied science and the therapeutic field.

A common method for mapping chromatin-associated factors is chromatin immunoprecipitation (ChIP), which, in conjunction with downstream sequencing methods, enables genome-wide mapping of binding sites of the chromatin-associated factor of interest. In a conventional ChIP workflow, the harvested cells are cross-linked with formaldehyde to ensure covalent, reversible cross-linking of the proteins with the DNA. The cells are lysed and the chromatin is sheared off by sonication. Antibodies are added that bind against the chromatin-associated protein of interest as well as to magnetic beads coated with protein A, allowing the chromatin-bound fraction being retained during washing steps, while the non-target chromatin portion can be removed without centrifugation. The immunoprecipitated DNA is then eluted from the beads with SDS-containing buffer, the cross-linking is relieved by heating to 65°C in the presence of SDS and sodium chloride. The recovered DNA is purified and used for enrichment testing by qPCR, library preparation and sequencing.

However, conventional ChIP requires a high number of cells as starting material, typically $10^6$ to $10^7$ cells, and a low starting quantity correlates with lower data quality. In addition, harsh crosslinking conditions can mask the epitope, resulting in loss of signal, and chromatin that does not become soluble after shearing is lost in the process. In addition, chromatin preparation is time consuming, has a high variability between batches of sheared chromatin, and results in high sequencing background.

Cleavage Under Targets and Tagmentation (CUT&Tag) is a method for chromatin profiling that does not require chromatin preparation. In CUT&Tag, cells are bound to magnetic concanavalin A-coated beads during the process up to DNA extraction and purification. An antibody against the desired chromatin mark enters the permeabilized cells and marks the site in the genome. A subsequently added fusion protein consisting of protein A and transposase Tn5 is bound to the antibody. After binding of the protein A part to the antibody and activation of Tn5, the latter cuts the DNA and incorporates sequencing adapters at the site. These can be sequenced after extraction and PCR amplification.

It has been shown that CUT&Tag can successfully detect abundant proteins with strong interactions with DNA, such as histone proteins. However, proteins that are less abundant or have a weaker, more dynamic interaction with DNA and therefore detach more easily from DNA require a higher number of cell inputs and cannot be reliably detected with CUT&Tag. Since CUT&Tag usually involves the use of high salt concentrations to remove unbound pA-Tn5 during the washing steps which is necessary to decrease off-target tagmentation, proteins tightly bound to DNA may remain bound to their target site in chromatin, while weaker interacting proteins could be removed under these harsh washing conditions. In ChIP, these weaker interactions with DNA are counteracted by strong crosslinking with 1% formaldehyde for about 8 minutes for histone modifications and about 15 minutes for non-histone proteins, depending on the protocol version and context. In CUT&Tag, such strong crosslinking would mask epitopes, making them less accessible to antibodies and pA-Tn5.

There is a need for methods that profile chromatin-associated proteins including less abundant non-histone proteins and weakly interacting transcriptional factors for their chromatin binding sites, which allow for reliable analysis of a broad range of cell numbers and/or protein-chromatin interactions.

**SUMMARY OF THE INVENTION**

In one aspect of the invention, a method for determining at least one chromatin binding site of a chromatin-associated factor of interest in a cell is disclosed, the method comprising:

(a) permeabilizing the cell;
(b) contacting the permeabilized cell with a first antibody that specially binds the chromatin-associated factor of interest and with a second antibody that specifically binds to the first antibody;
(c) contacting the permeabilized cell with a transposome that is linked to a specific binding agent that specifically binds the first and/or second antibody wherein the transposome comprises a transposase and a first and second DNA molecule;
(d) activating the transposase, thereby excising a DNA segment comprising at least one chromatin binding site of a chromatin-associated factor of interest and tagging the DNA with the first and second DNA molecule;

(e) determining the sequence of the excised and tagged DNA segment; and

(f) based on the determined sequence of the excised and tagged DNA segment, determining the at least one chromatin binding site of the chromatin-associated factor of interest in the cell; wherein at least the steps of contacting the permeabilized cell with the transposome and activating the transposase are performed in presence a crowding agent.

The transposome comprises a transposase and a first and second DNA molecule. The first DNA molecule may comprise a first transposase recognition site and the second DNA molecule may comprise a second transposase recognition site.

In one embodiment of the invention, at least the steps of contacting the permeabilized cell with the transposome and activating the transposase are performed in presence of a Good's buffer.

In another embodiment of the invention, the Good's buffer is selected from Tricine, MES, ADA, PIPEs, ACES, MPOSO, Cholamine chloride, MOPS, BES, TES, DIPSO, TASO, Acetamidoglycine, POPSO, Acetamidoglycine, POPSO, HEPPSO, HEPPS, Tris, Glycinamide, Glycylglycine, Bicine and TAPS, optionally wherein the Good's buffer is Tricine.

In a further embodiment of the invention, the steps of contacting the permeabilized cell with the transposome and activating the transposase are performed at a salt concentration of ≤ 300 mM NaCl.

In another embodiment of the invention, prior to the permeabilization step the cells are subjected to mild cross-linking conditions. Optionally, the mild cross-linking conditions are 0.1% formaldehyde for ≤ 15 min, ≤ 10 min or ≤ 5 min.

In another embodiment of the invention, $MgCl_2$ is not added prior to activating the transposase.

In another embodiment of the invention, the chromatin-associated factor of interest is a non-histone protein, optionally a transcription factor or a histone reader protein.

In a further embodiment of the invention, wherein the number of cells is ≤ 100,000, optionally ≤ 50,000, ≤ 10,000, ≤ 5,000 or ≤ 1,000.

In another embodiment of the invention, the cell is permeabilized using Triton-X-1 00, optionally at a concentration of 0.1%.

In another embodiment of the invention, the crowding agent is selected from a group comprising sucrose, hexylene glycol and glycerol, optionally wherein the crowding agent is sucrose.

In another embodiment of the invention, prior to the permeabilization step, the cells are bound to beads, optionally magnetic beads,

In another embodiment of the invention, all method steps are performed using total buffer volumes of ≤ 200 μl.

In another embodiment of the invention, the amount of transposase that is linked to a specific binding agent is ≤ 1:250 of the reaction volume.

In yet another embodiment of the invention, the amount of first and/or second antibody per reaction is ≤ 1 μg.

Another embodiment of the invention comprises performing steps (a) to (f) in a first replicate and the following steps in a second replicate:

- purifying DNA from the cell,
- contacting the purified DNA with the transposome;
- activating the transposase;
- determining the sequence of the excised and tagged DNA in the second replicate;

wherein the sequence of the excised and tagged DNA segment determined in the second replicate is used as a normalization control in the step of determining the at least one chromatin binding site of the chromatin-associated factor of interest in the cell determined in step (f) in the first replicate.

In a further embodiment of the invention, an input sample is set aside prior to the permeabilization step and subjected to method steps (d) to (f), and wherein the input sample is used for normalization in the step of determining the at least one chromatin binding site of the chromatin-associated factor of interest in the cell based on the determined sequence of the excised and tagged DNA.

In another embodiment of the invention, for a first portion of the cell population an antibody targeting the chromatin-associated factor of interest is used as first antibody and for a second portion of the cell population IgG is used as first antibody, and wherein the sequence of the excised DNA determined for the portion of the cell population using IgG as first antibody is used for normalization in the step of determining the sequence of the excised DNA for the portion of cell population using the antibody targeting the chromatin-associated factor of interest as first antibody.

Another aspect of the invention relates to a method for generating a normalization control suitable for use in the methods described above, comprising the following steps:

- purifying DNA from the cell;

- contacting the purified DNA with a transposome that is linked to a specific binding agent suitable for binding a first and/or second antibody wherein the transposome comprises a transposase and a first and second DNA molecule;
- activating the transposase; and
- determining the sequence of the excised and tagged DNA.

In another aspect of the invention, a method for assessing the quality of the methods described above by determining the percentage of false positives resulting from off-target transposase activity is provided which comprises the following steps:

(i) conducting steps (a) to (f) of the above described methods for determining at least one chromatin binding site of a chromatin-associated factor of interest in a cell at least twice,
(ii) identifying the chromatin binding sites that overlap for the at least one chromatin binding site determined in each step (f);
(iii) identifying a subset of the chromatin binding sites identified in step (ii) that does not overlap with a reference set of chromatin binding sites determined for the chromatin-associated factor of interest identified using ChIP-seq,
(iv) identifying a subset of the subset of chromatin binding sites identified in step (iii) that overlaps with a reference set of chromatin sites identified using ATAC-seq; and
(v) determining the number of chromatin binding sites identified in step (iv) and dividing said number with the number of chromatin binding sites identified in step (ii) in order to obtain the percentage of false positives resulting from off-target transposase activity.

In one embodiment of the method for assessing the quality, conducting steps (a) to (f) of the above described methods for determining at least one chromatin binding site of a chromatin-associated factor of interest in a cell at least twice comprises conducting steps (a) to (f) of the above described methods for determining at least one chromatin binding site of a chromatin-associated factor of interest in a first replicate and repeating steps (a) to (f) of the above described method for determining at least one chromatin binding site of a chromatin-associated factor of interest in at least a second replicate. Alternatively, conducting steps (a) to (f) of the above described methods for determining at least one chromatin binding site of a chromatin-associated factor of interest in a cell at least twice comprises conducting steps (a) to (f) of the above described methods for determining at least one chromatin binding site of a chromatin-associated factor of interest in a first replicate and repeating steps (a) to (f) of the above described methods for determining at least one chromatin binding site of a chromatin-associated factor of interest at least once in the same replicate.
In another embodiment of the method for assessing the quality, the chromatin binding site of the chromatin-associated factor of interest is present in closed chromatin or heterochromatin.
In another aspect of the invention, a method for determining at least one chromatin binding site of a chromatin-associated factor of interest in a cell is disclosed which comprises the following steps:

(a) permeabilizing the cell in a first replicate;
(b) purifying DNA from the cell in a second replicate;
(c) contacting the permeabilized cell in the first replicate with a first antibody that specially binds the chromatin-associated factor of interest and with a second antibody that specifically binds to the first antibody;
(d) contacting both the permeabilized cell in the first replicate and the purified DNA in the second replicate with a transposome that is linked to a specific binding agent that specifically binds the first and/or second antibody wherein the transposome comprises a transposase and a first and second DNA molecule;
(e) activating the transposase in both the first and second replicate, thereby excising a DNA segment comprising at least one chromatin binding site of a chromatin-associated factor of interest and tagging the DNA with the first and second DNA molecule;
(f) determining the sequence of the excised and tagged DNA segment in both the first and second replicate; and
(g) based on the determined sequence of the excised and tagged DNA segment in the first replicate, determining the at least one chromatin binding site of the chromatin-associated factor of interest in the cell, wherein the sequence of the excised and tagged DNA segment determined in the second replicate is used as a normalization control, optionally wherein the steps of contacting both the permeabilized cell in the first replicate and the purified DNA in the second replicate with the transposome and activating the transposase in both the first and second replicate are performed in presence a crowding agent.

In another aspect of the invention, a method for determining at least one chromatin binding site of a chromatin-associated factor of interest in a cell is disclosed, the method comprising:

(a) permeabilizing the cell with a non-saponin permeabilization reagent, optionally selected from Triton X-100 and Tween-20.

(b) contacting the permeabilized cell with a first antibody that specially binds the chromatin-associated factor of interest and with a second antibody that specifically binds to the first antibody;

(c) contacting the permeabilized cell with a transposome that is linked to a specific binding agent that specifically binds the first and/or second antibody wherein the transposome comprises a transposase and a first and second DNA molecule;

(d) activating the transposase, thereby excising a DNA segment comprising at least one chromatin binding site of a chromatin-associated factor of interest and tagging the DNA with the first and second DNA molecule;

(e) determining the sequence of the excised and tagged DNA segment; and

(f) based on the determined sequence of the excised and tagged DNA segment, determining the at least one chromatin binding site of the chromatin-associated factor of interest in the cell.

**BRIEF DESCRIPTION OF THE DRAWINGS**

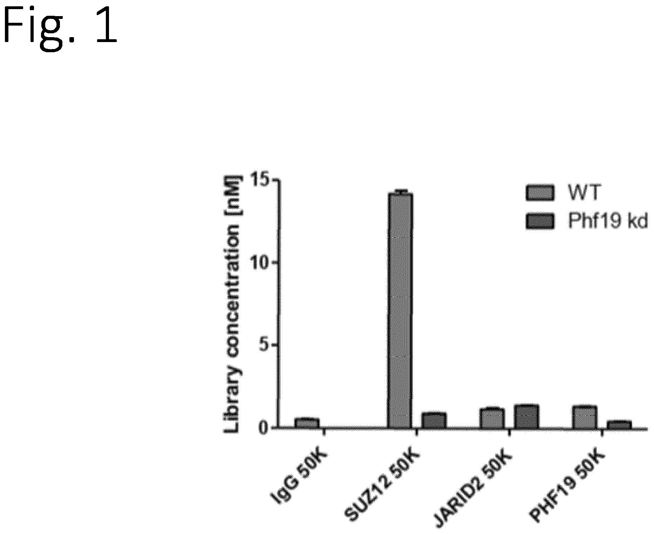

**Figure 1:** Concentration of sequencing libraries obtained with the conventional CUT&Tag protocol using 50,000 HPC7 wild-type (WT) or Phf19 knockdown (kd) cells and primary antibodies against SUZ12, JARID2 and PHF19. IgG was used as control. The bars represent the mean of library concentration $\pm$ SD of duplicates.

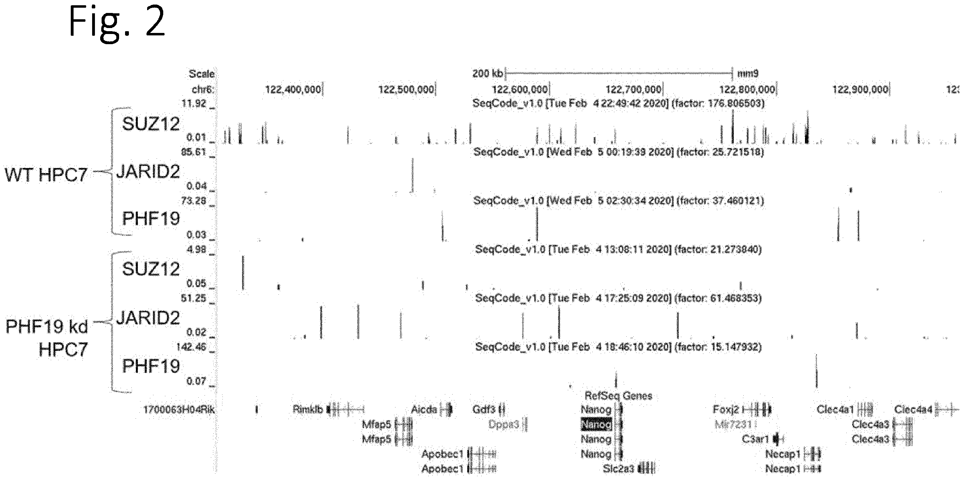

**Figure 2:** Genomic tracks at the *Nanog* gene locus showing data obtained with the conventional CUT&Tag protocol in 50,000 wild-type (WT) or Phf19 knockdown (kd) HPC7 cells using primary antibodies against SUZ12, JARID2 and PHF19.

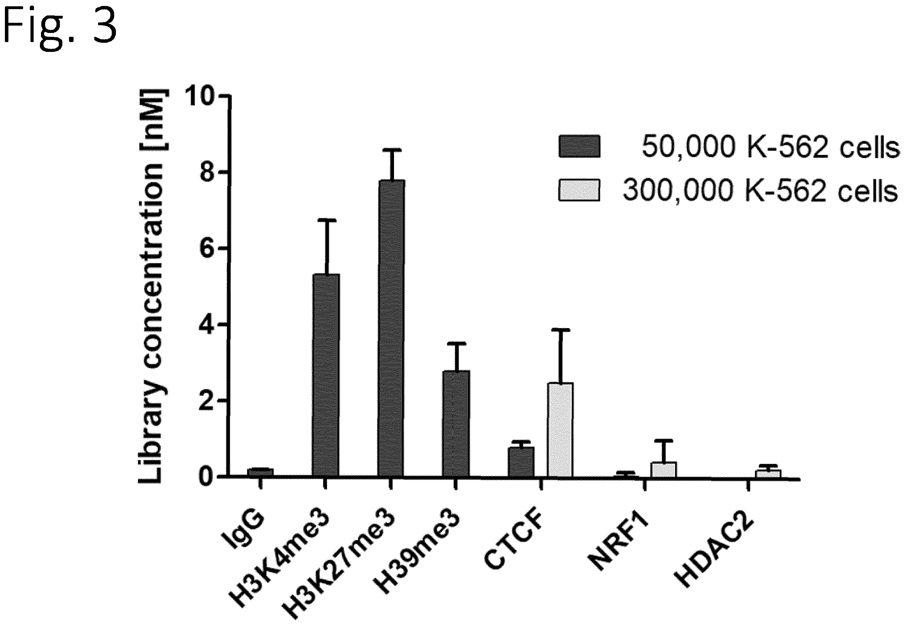

**Figure 3:** Concentration of sequencing libraries obtained with the conventional CUT&Tag protocol using 50,000 or 300,000 K-562 cells and primary antibodies against H3K4me3, H3K27me3, H3K9me3, CTCF, NRF1 and HDAC2. IgG was used as control. The bars represent the mean of library concentration $\pm$ SD of duplicates.

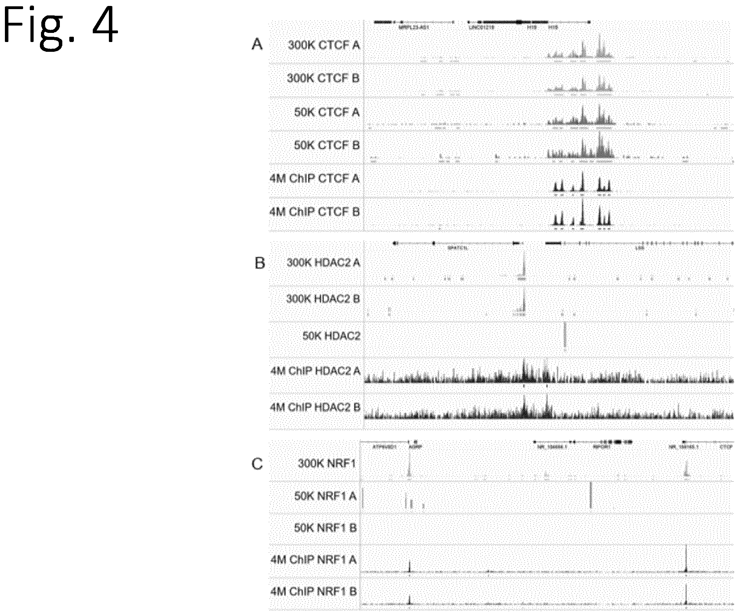

**Figure 4:** Genome tracks showing CTCF, HDAC2 and NRF1 obtained with 50,000 or 300,000 K-562 cells using the conventional CUT&Tag protocol or 4 million K-562 cells using ChiP. (A) CTCF samples at the H19 gene. (B) HDAC2 samples at the *Spatc1l* and *Lss* genes. (C) NRF1 samples at the *Agrp* and Ctcf genes.

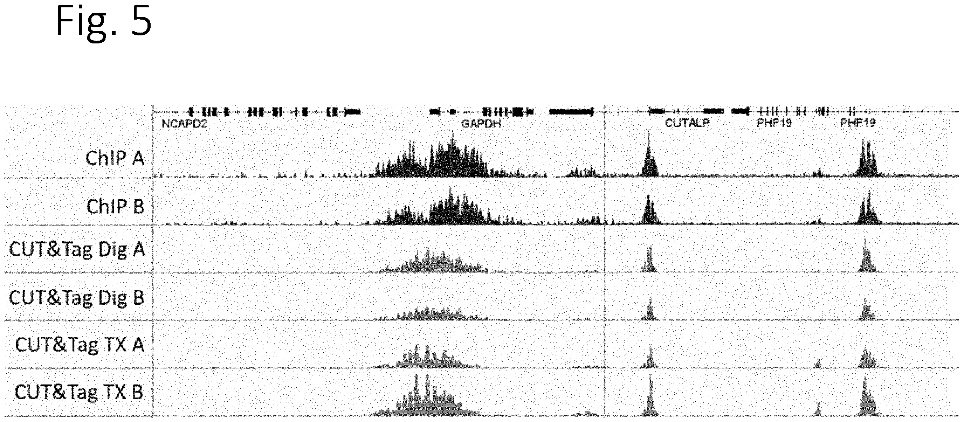

**Figure 5:** Genome tracks of CUT&Tag experiments on H3K4me3 using Triton X-100 (TX) and digitonin (Dig) for cell permeabilisation at the genomic locus of *Gapdh* and *Phf19.* CUT&Tag experiments were performed using 50,000 K-562 cells. For comparison, ChIP-seq data with 50,000 K-562 cells are shown.

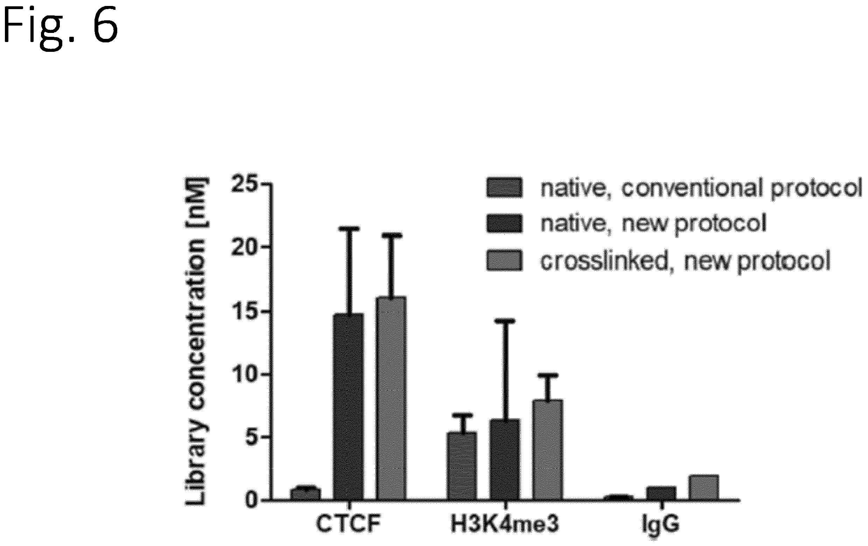

**Figure 6:** Concentration of sequencing libraries obtained with the improved CUT&Tag protocol with or without crosslinking and with the conventional CUT&Tag protocol using 50,000 K-562 cells and targeting CTCF, H3K3me3 or IgG (control). The bars represent the mean of library concentration $\pm$ SD of duplicates.

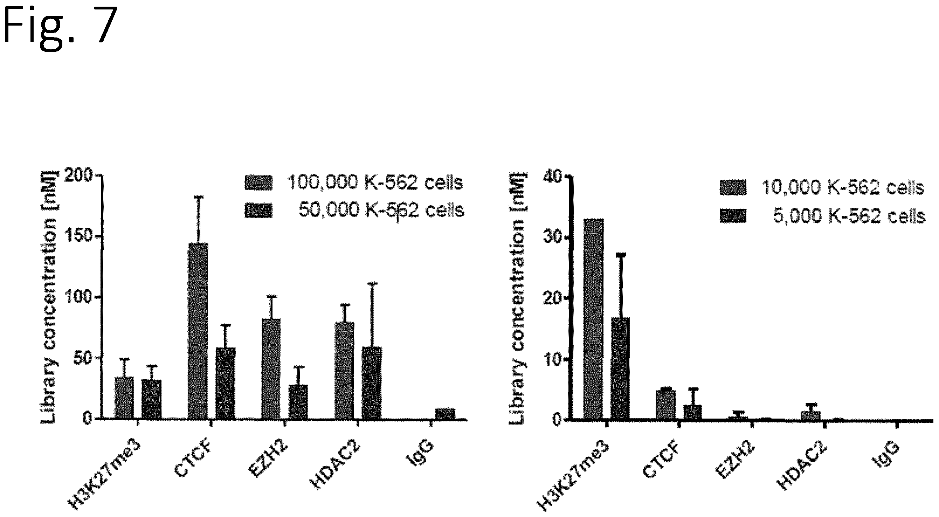

**Figure 7:** Concentration of sequencing libraries obtained with the improved CUT&Tag protocol targeting CTCF, EZH2, HDAC2, H3K27me3 and IgG using either 100,000, 50,000, 10,000 or 5,000 K-562 cells. Bars represent library concentration in mean $\pm$ SD of duplicates.

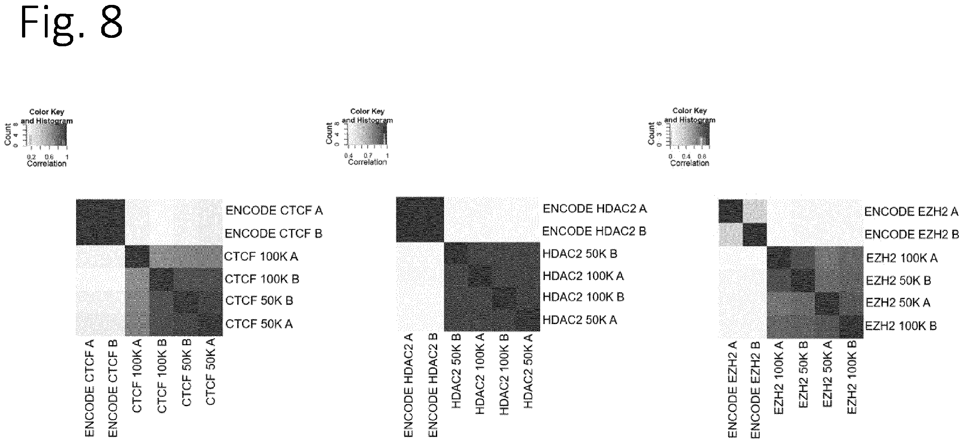

**Figure 8:** Peak correlation of CUT&Tag samples assessing CTCF, EZH2, HDAC2 using 50,000 or 100,000 cells (duplicates) and compared to ENCODE ChIP-seq data assessing the same chromatin-binding proteins. CUT&Tag samples obtained with the protocol disclosed herein are highly correlated with each other based on binding site occupancy, but not with ENCODE ChIP-seq data.

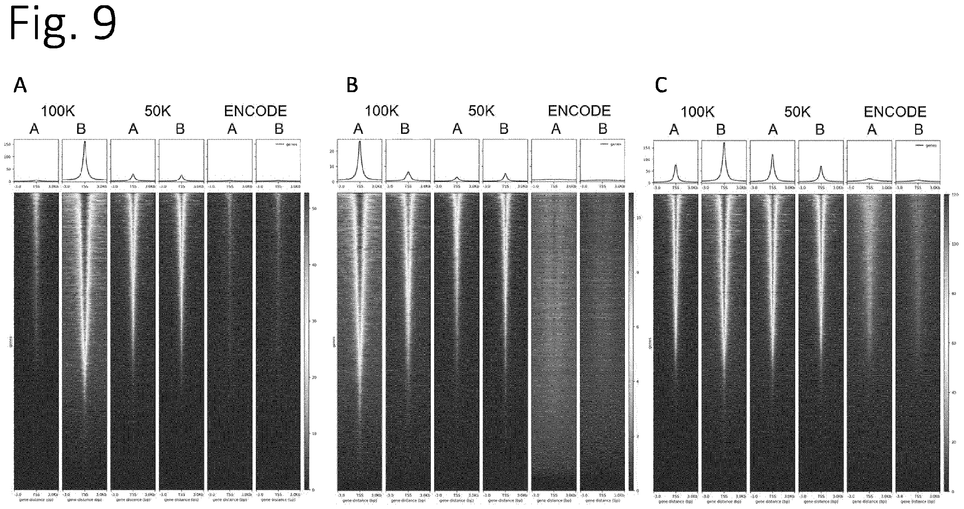

**Figure 9:** Heatmaps of (A) CTCF, (B) EZH2 and (C) HDAC2 CUT&Tag samples generated using the improved protocol and ENCODE ChIP-seq samples 3 kb upstream and downstream of the TSS. Despite their heterogeneity, most of the CUT&Tag samples show a higher signal than the ENCODE ChIP samples.

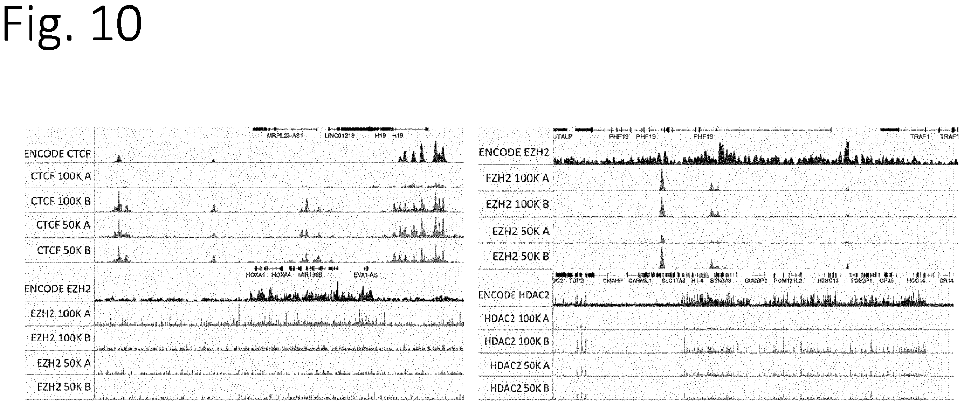

**Figure 10:** Genome tracks of CTCF, EZH2 and HDAC2 CUT&Tag samples and their respective ENCODE ChIP-seq reference data. CTCF: H19 locus. EZH2: *HoxA* locus and *Phf19* locus. HDAC2: *Btn3a3* locus.

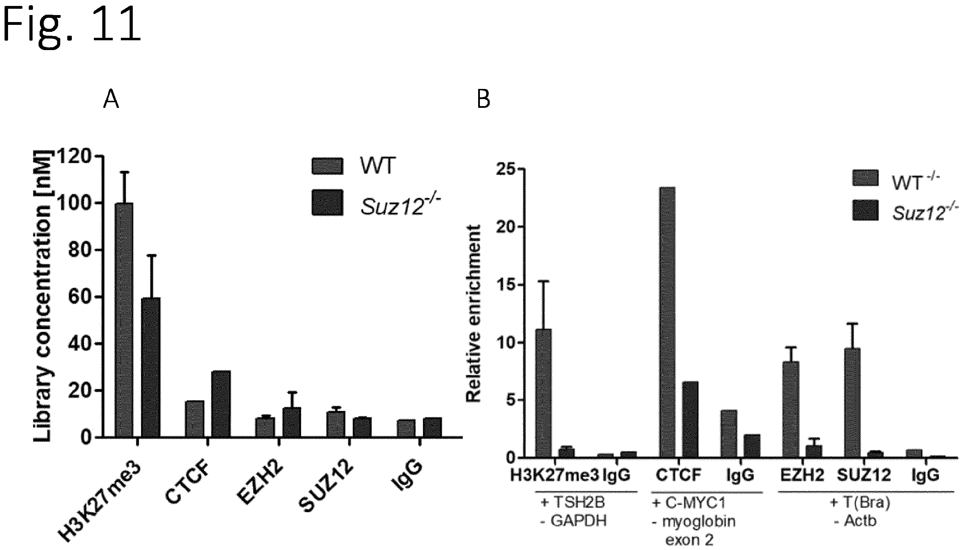

**Figure 11:** (A) Concentration of sequencing libraries normalised for 14 PCR cycles to be comparable obtained with the improved CUT&Tag protocol assessing H3K27me3, CTCF, EZH2, SUZ12 and IgG (control) using 50,000 cells in WT and SUZ12$^{-/-}$ mESCs. Bars represent library concentration in mean $\pm$ SD of duplicates. (B) Enrichment of H3K27me3, CTCF, EZH2 and SUZ12 on target loci relative to negative control loci. IgG was used as control. Bars represent relative enrichment in mean $\pm$ SD of duplicates.

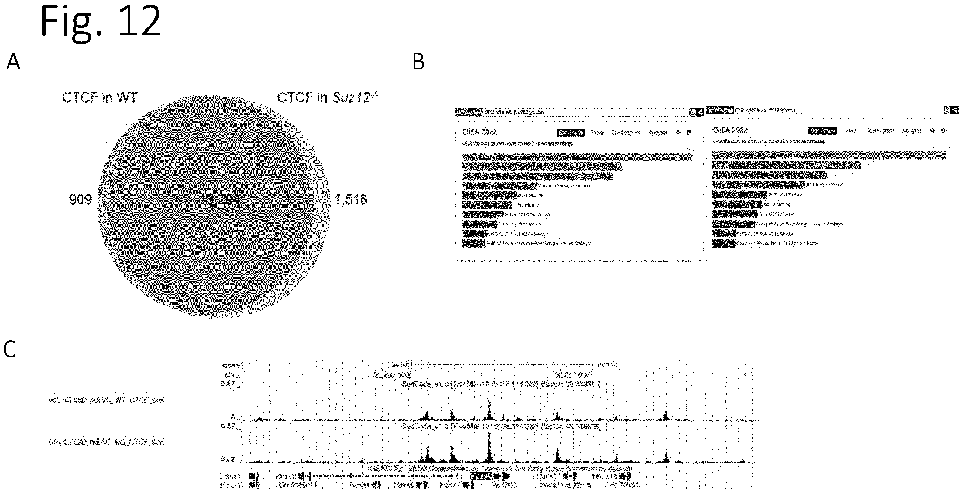

**Figure 12:** CUT&Tag for CTCF in WT and Suz12$^{-/-}$ mESCs as Suz12 KO-independent control. (A) Overlap of gene sets; (B) Functional analysis using Enrichr; (C) Genome tracks of CTCF at the *HoxA* cluster.

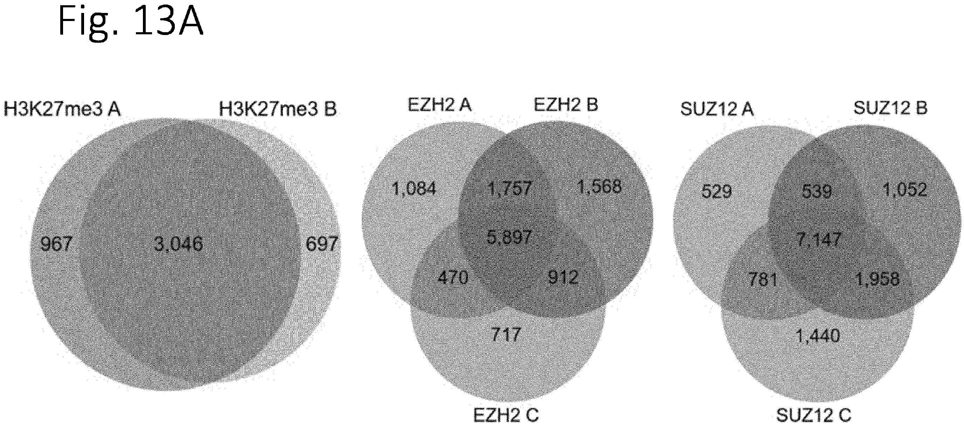

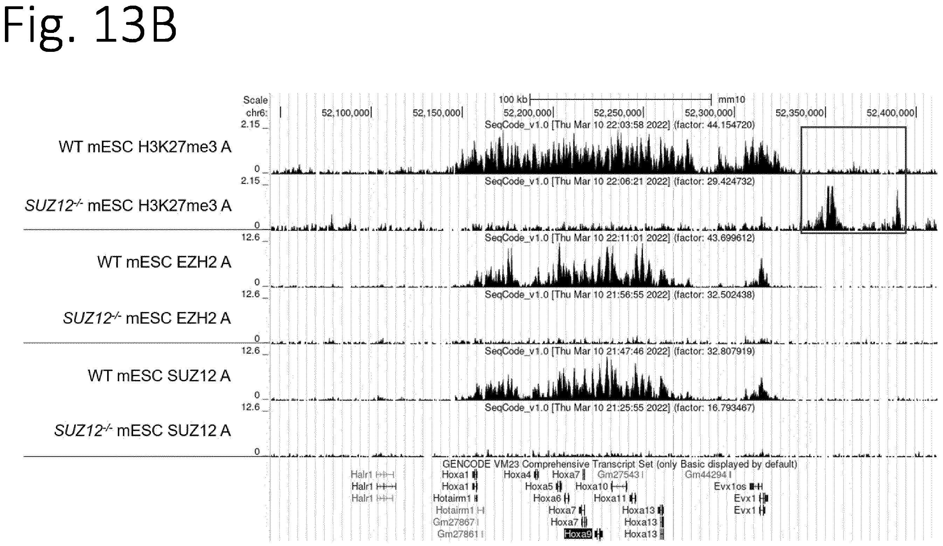

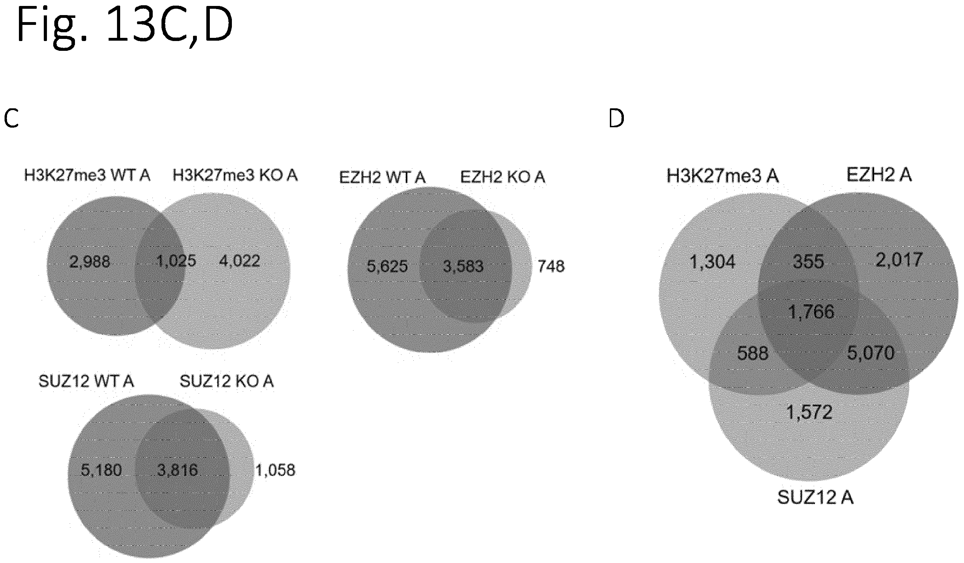

**Figure 13:** CUT&Tag for H3K27me3, EZH2, SUZ12 in WT and Suz12$^{-/-}$ mESCs. (A) Common and specific gene sets between H3K27me3 duplicates, EZH2 triplicates and SUZ12 triplicates, respectively; (B) Genome tracks of one replicate for H3K27me3, EZH2 and SUZ12 in WT and Suz12$^{-/-}$ mESCs using the same respective scale; (C) Common and specific genes between the respective WT and Suz12$^{-/-}$ samples for H3K27me3, EZH2 and SUZ12; (D) Overlap of gene sets between one replicate of H3K27me3, EZH2 and SUZ12.

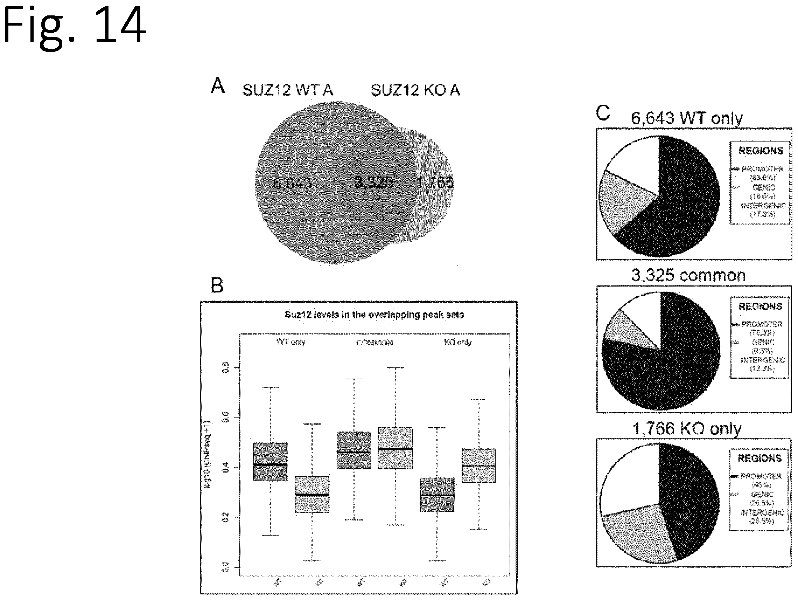

**Figure 14:** Comparison of SUZ12 CUT&Tag in WT and Suz12$^{-/-}$ cells at peak level. (A) Number of common and specific peaks between WT and Suz12$^{-/-}$; (B) Signal intensity at peaks; (C) Genomic distribution of peaks.

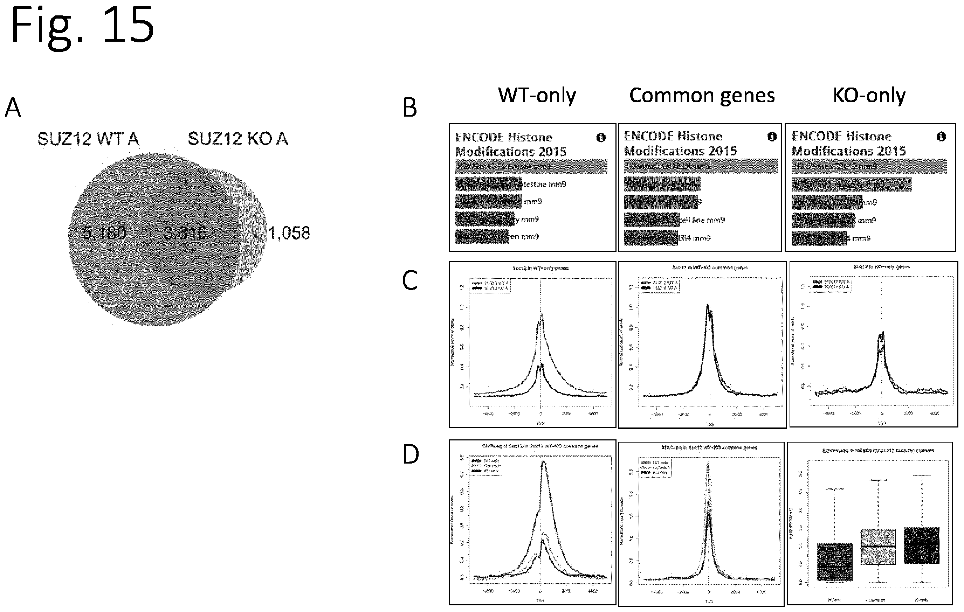

**Figure 15:** Characteristics of WT-only, common and KO-only genes identified using SUZ12 CUT&Tag in WT and Suz12$^{-/-}$ cells. (A) Overlap of the genes underlying peaks identified in SUZ12 CUT&Tag in WT and Suz12$^{-/-}$ cells. (B) Functional analysis of the gene subsets. (C) Count of reads at TSS of gene subsets identified by SUZ12 CUT&Tag in WT and Suz12$^{-/-}$ samples. (D) Signal of SUZ12 ChIP-seq and ATAC-seq data at common genes identified in SUZ12 CUT&Tag in WT and Suz12$^{-/-}$ cells, as well as RNA-seq for SUZ12 CUT&Tag subsets.

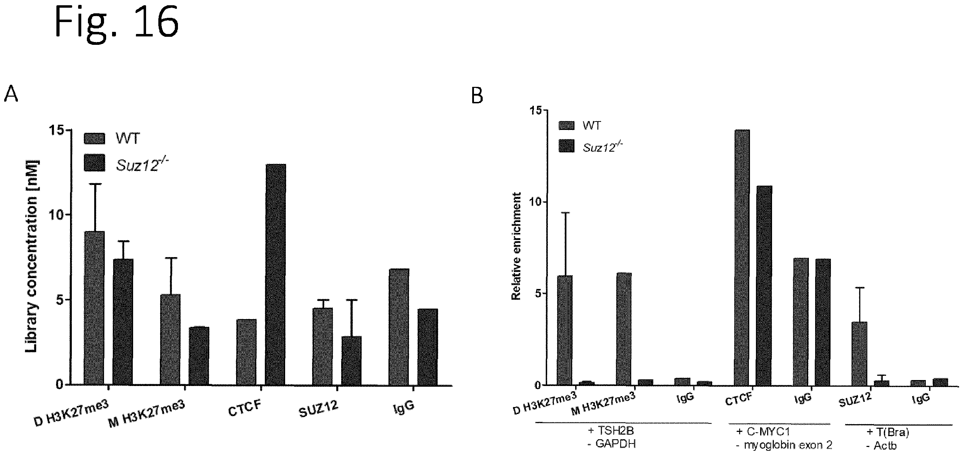

**Figure 16:** (A) Concentration of sequencing libraries obtained with the improved CUT&Tag protocol targeting H3K27me3 using two different antibodies (Diagenode, D; Merck Millipore; M), CTCT and SUZ12 using 50,000 mESCs. IgG was used as control. Bars represent library concentration in mean $\pm$ SD of duplicates. (B) Enrichment of H3K27me3, CTCF and SUZ12 on target loci relative to negative control loci. IgG was used as control. Bars represent relative enrichment in mean $\pm$ SD of duplicates.

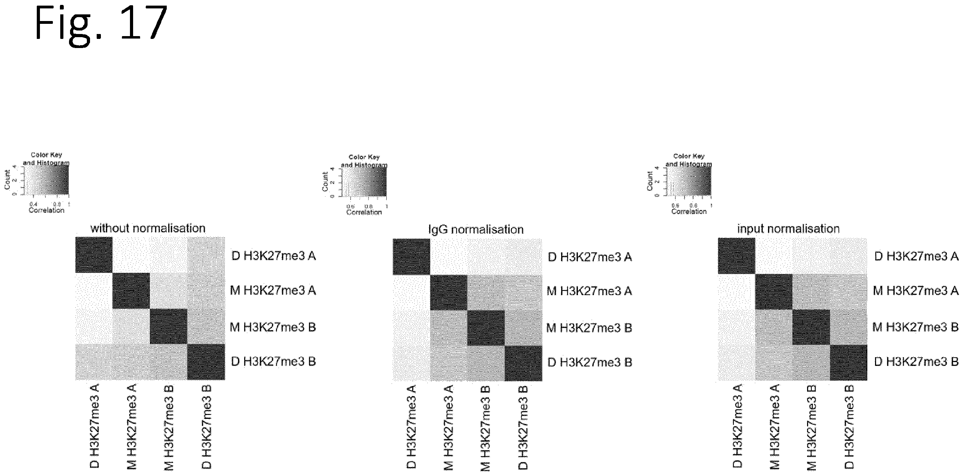

**Figure 17:** Peak correlation of CUT&Tag samples assessing H3K27me3 using two different antibodies (Diagenode, D; Merck Millipore; M) using 50,000 SUZ12 WT mESCs. Normalisation with either IgG or input increases correlation among H3K27me3 replicates.

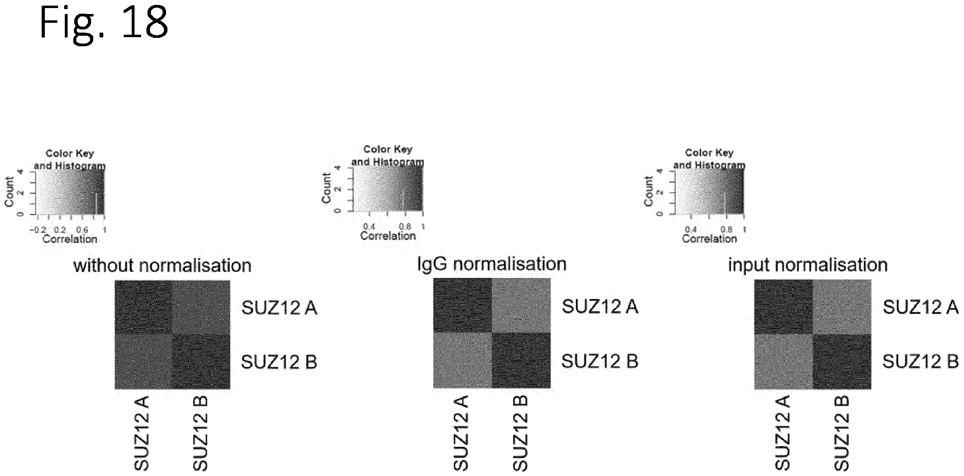

**Figure 18:** Peak correlation of CUT&Tag samples assessing SUZ12 using 50,000 SUZ12 WT mESCs. Normalisation with either IgG or input.

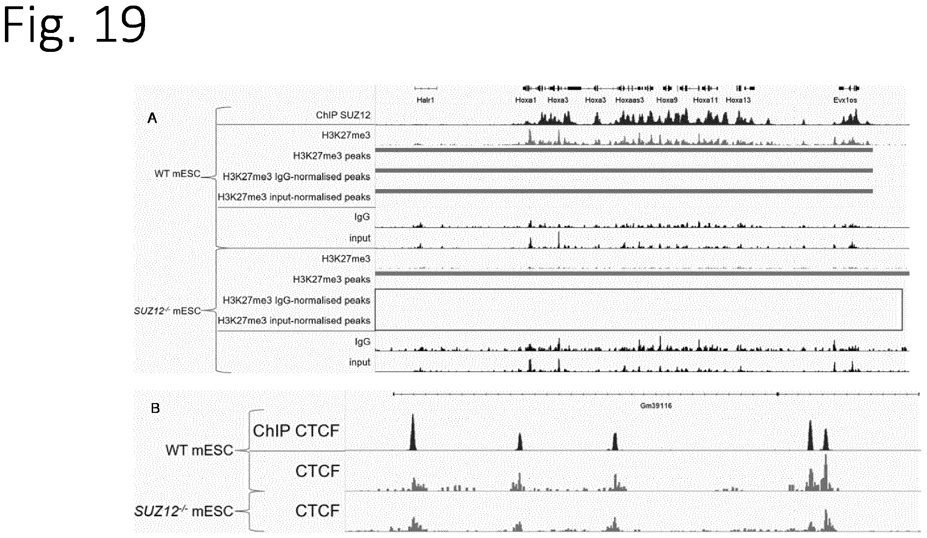

**Figure 19:** Genome tracks of CUT&Tag in WT and Suz12$^{-/-}$ mESCs. (A) H3K27me3 CUT&Tag in WT and Suz12$^{-/-}$ mESCs. SUZ12 ChIP (red), IgG and input tracks are shown as controls. Normalization with IgG or input (black) leads to the disappearance of the H3K27me3 peaks in the KO sample (circled in red), while the broad peak called at the *HoxA* cluster in the H3K27me3 WT sample (green) remains; (B) CTCF CUT&Tag in WT and Suz12$^{-/-}$ mESCs. CTCF ChIP-seq data from ENCODE are shown as controls.

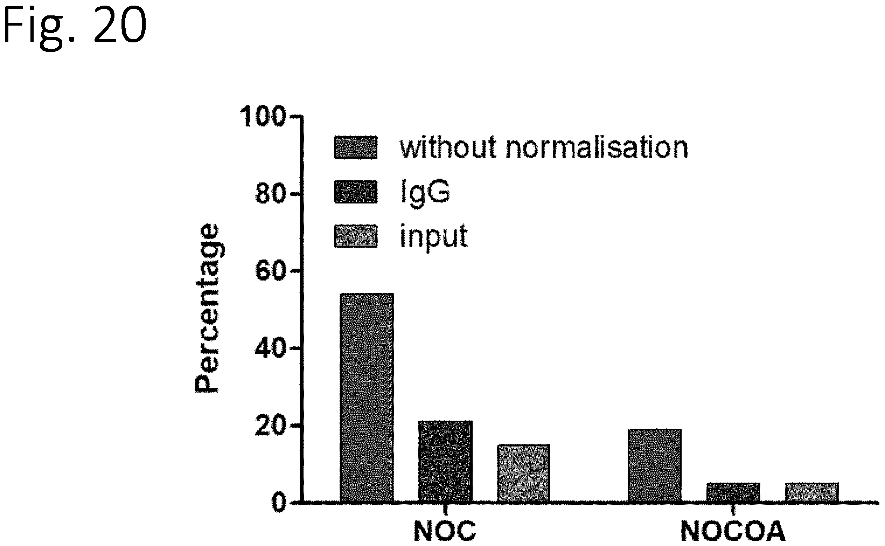

**Figure 20:** Percentage of peaks not overlapping with reference SUZ12 ChIP-seq data (NOC) and percentage of peaks not overlapping with reference SUZ12 ChIP-seq peaks but with ATAC-seq peaks (NOCOA) of SUZ12 CUT&Tag without normalisation or upon normalisation with IgG and input, respectively. Bars represent mean of duplicates.

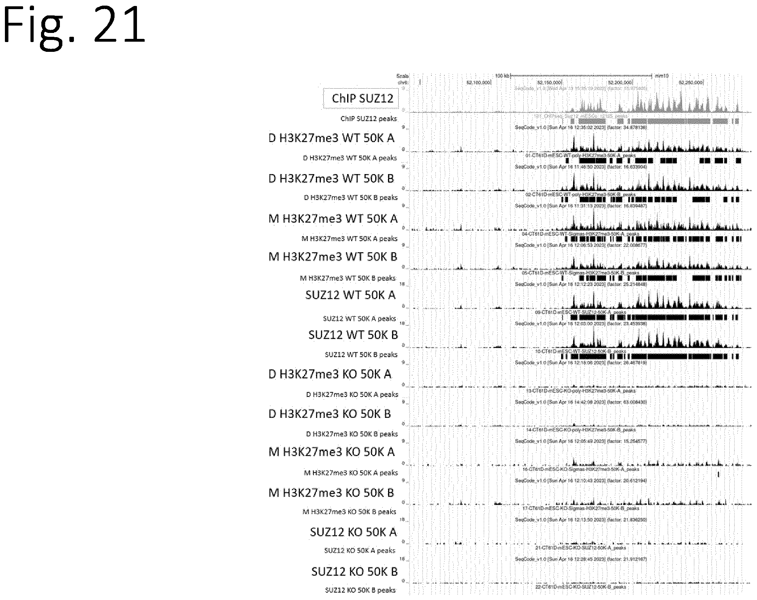

**Figure 21:** Genome tracks of CUT&Tag samples assessing H3K27me3 and SUZ12 at the *HoxA* cluster in 50,000 WT and Suz12$^{-/-}$ mESCs. Peaks were IgG-normalised.

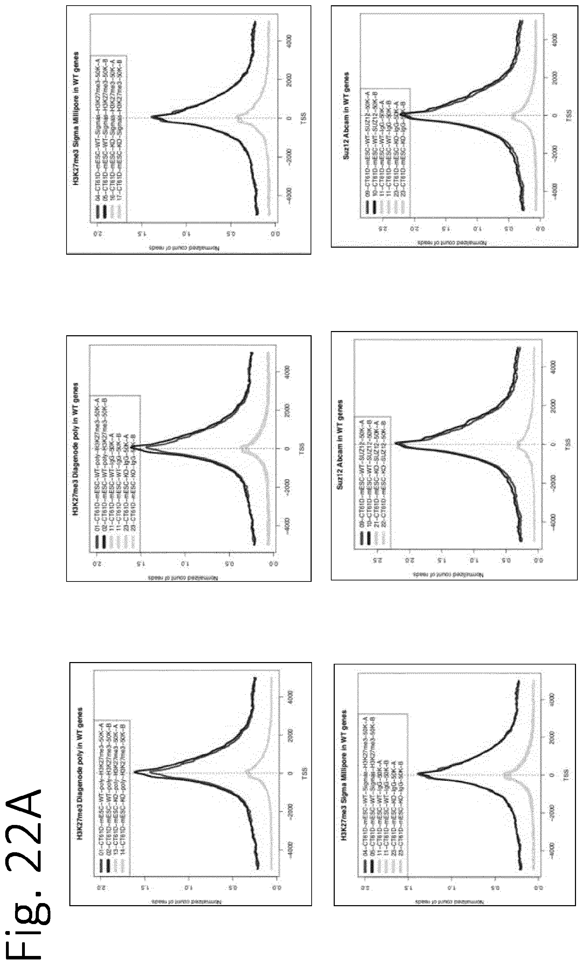

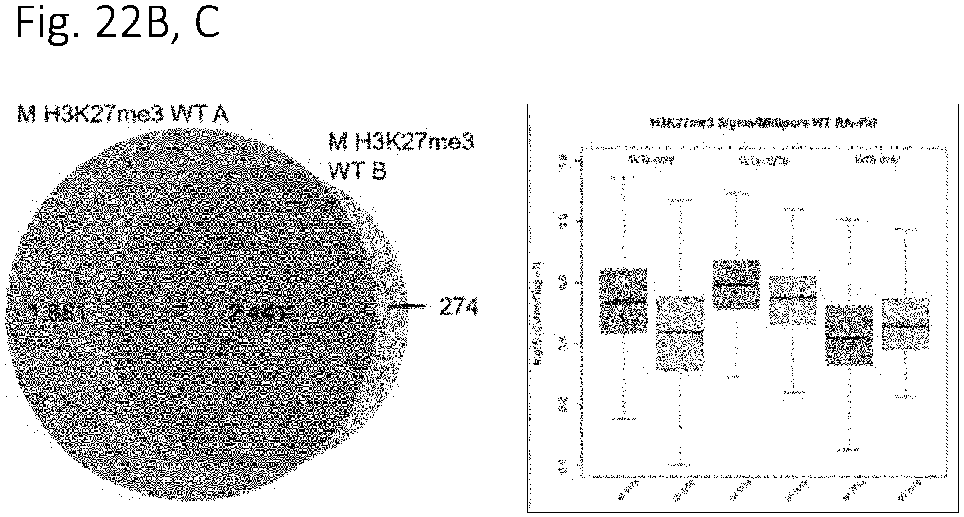

**Figure 22:** The comparison of CUT&Tag in WT and Suz12$^{-/-}$ mESCs shows the successful application of the CUT&Tag protocol described herein and a large overlap of peaks in the M H3K27me3 replicates. (A) Metaplots of CUT&Tag samples using antibodies against H3K27me3 by Diagenode and Merck Millipore, respectively, or against SUZ12 in WT and Suz12$^{-/-}$ mESCs, respectively, as well as obtained with IgG in the same cells. (B) Overlap of IgG-normalised peaks between H3K27me3 replicates obtained using the Merck Millipore (M) antibody. (C) Signal at sites of common and specific peaks for H3K27me3 replicates obtained using the Merck Millipore (M) antibody.

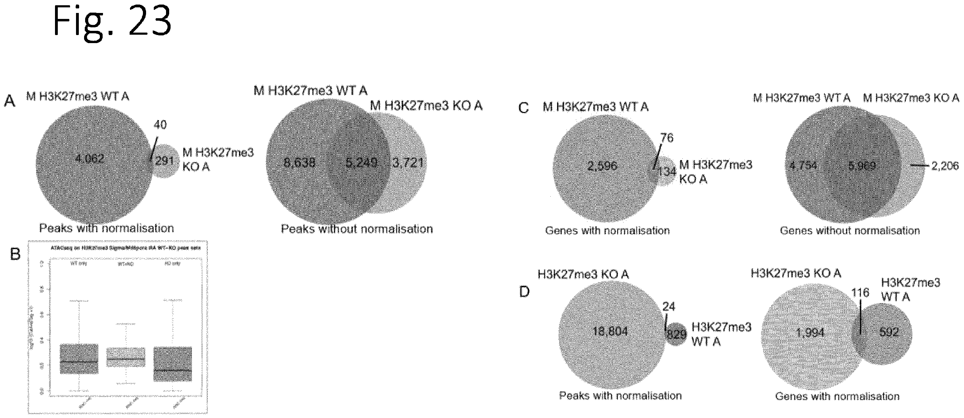

**Figure 23:** Normalisation decreases detected peaks and genes in Suz12$^{-/-}$ cells and reduces the overlap with WT data sets. (A) Overlap of H3K27me3 (Merck Millipore, M) peaks obtained by CUT&Tag in WT and Suz12$^{-/-}$ cells upon normalization with IgG (left) and without normalization (right). (B) ATAC-seq signal in IgG-normalised H3K27me3 (Merck Millipore, M) WT and Suz12$^{-/-}$ peak sets. Overlap of gene sets correspond to H3K27me3 (Merck Millipore, M) peaks obtained by CUT&Tag in WT and Suz12$^{-/-}$ cells upon normalization with IgG (left) and without normalization (right). (D) Overlap of H3K27me3 peaks obtained by CUT&Tag in WT and Suz12$^{-/-}$ cells in Example 3, after normalization with IgG (left) and without normalization (right).

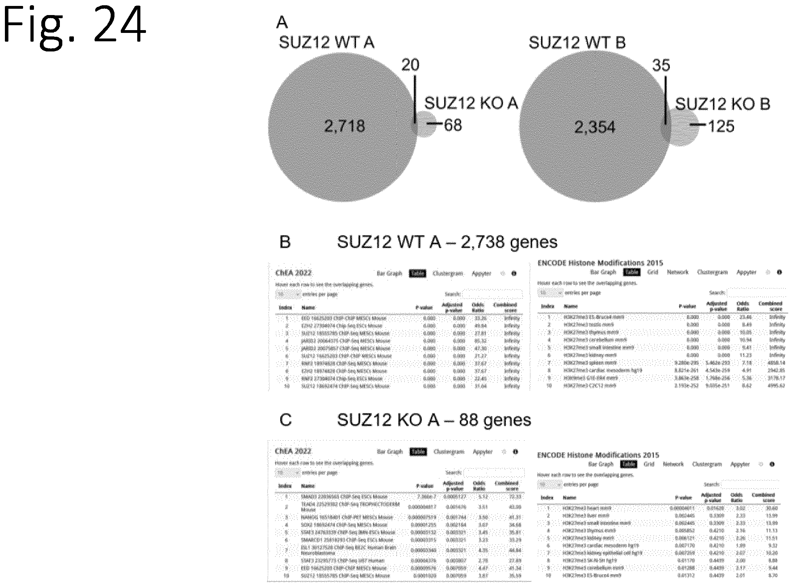

**Figure 24:** (A) Overlap of genes corresponding to peaks obtained by SUZ12 CUT&Tag in WT and Suz12$^{-/-}$ cells in replicate A (left) and replicate B (right). (B) Functional analysis of the 2738 genes obtained by SUZ12 CUT&Tag replicate A in SUZ12 WT cells and (C) of the 88 genes obtained in Suz12$^{-/-}$ cells.

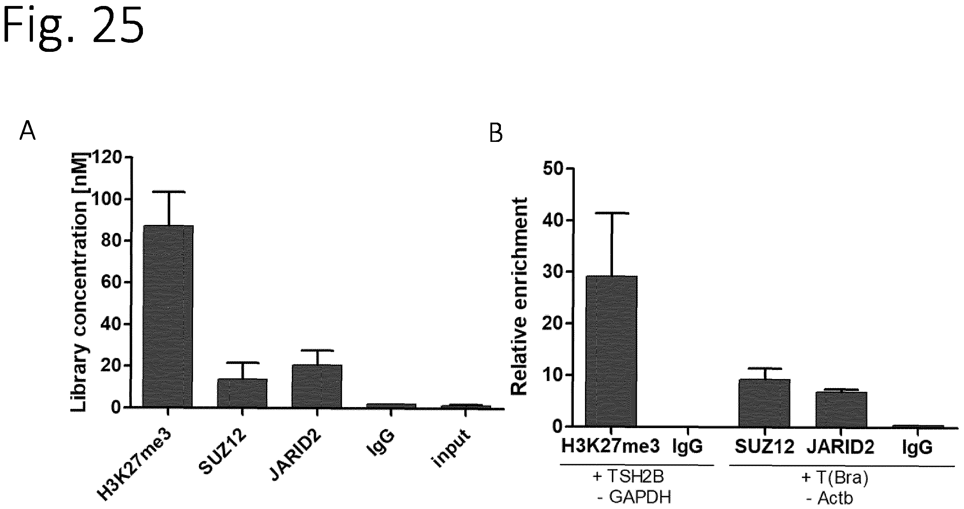

**Figure 25:** CUT&Tag protocol described herein achieves higher library concentrations and enrichment at target loci compared to previous protocol versions. (A) Concentration of sequencing libraries obtained with the improved CUT&Tag protocol targeting H3K27me3, SUZ12 and JARID2 using 5,000 mESCs. IgG and input were used as controls. Bars represent library concentration in mean $\pm$ SD of duplicates. (B) Enrichment of H3K27me3, SUZ12 and JARID2 on target loci relative to negative control loci. IgG was used as control. Bars represent relative enrichment in mean $\pm$ SD of duplicates.

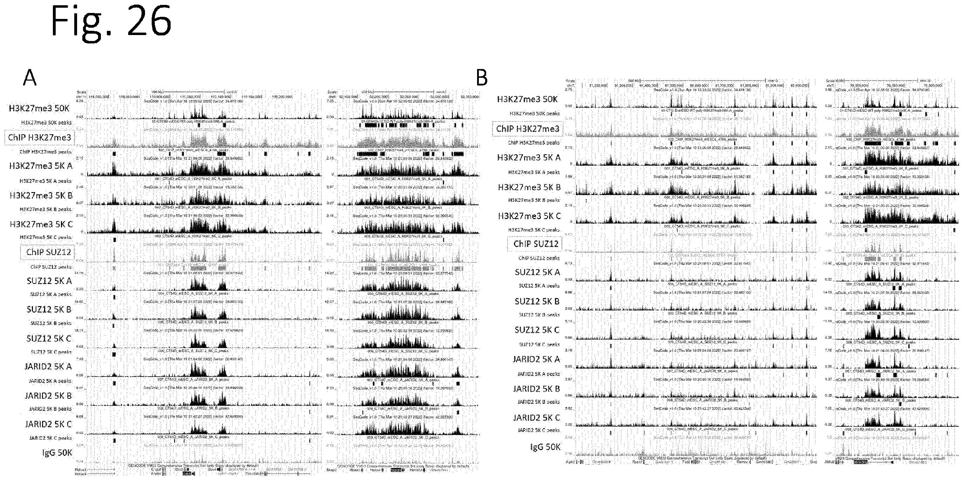

**Figure 26:** Genome tracks obtained with the improved CUT&Tag protocol targeting H3K27me3, SUZ12 and JARID2 using 5,000 mESCs showing four different loci: (A) *Cbx2,4,8* (left), *HoxA* cluster (right). (B) *Fsd2* (left), *Mir9-3hg* (right). ChIP-Seq data (gold), and IgG and H3K27me3 CUT&Tag data obtained using 50,000 mESCs are shown as controls.

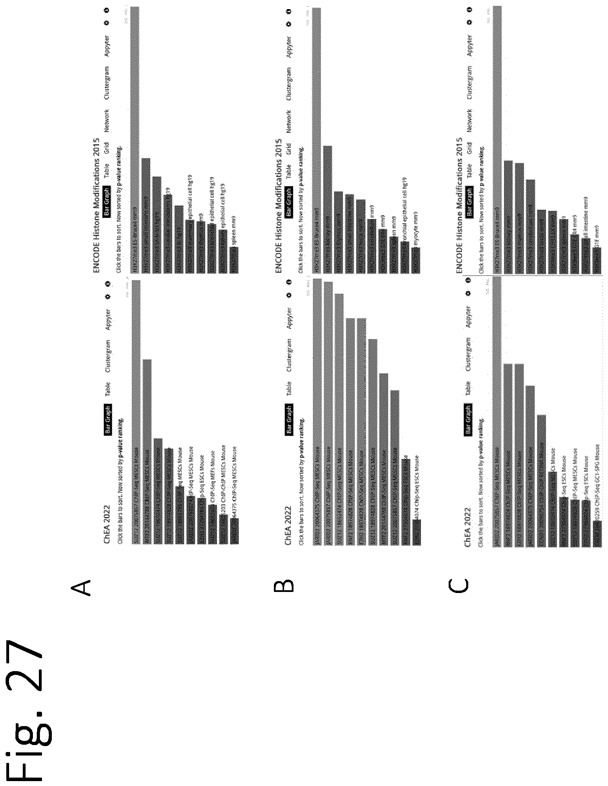

**Figure 27:** Functional analysis of CUT&Tag assessing H3K27me3 (A), SUZ12 (B) and JARID2 (C) in 5,000 mESCs using Enrichr.

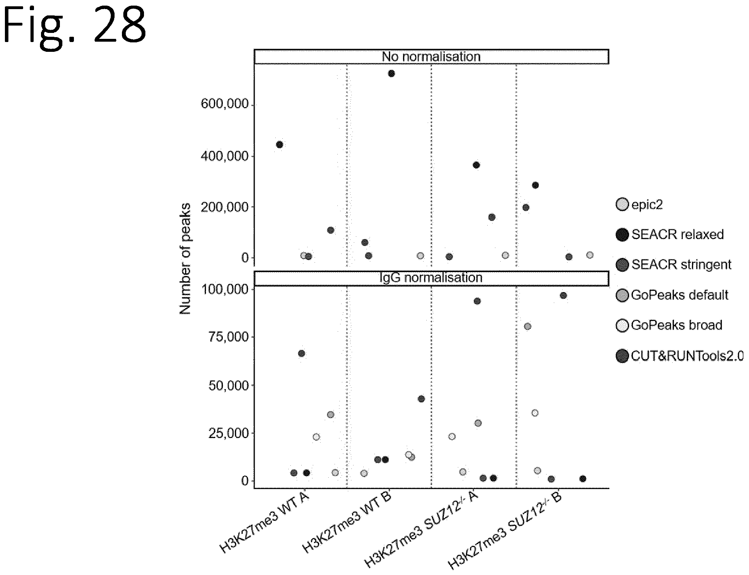

**Figure 28:** Number of peaks obtained for H3K27me3 in WT and SUZ12$^{-/-}$ mESCs with different peak callers and/or settings without normalization and after normalization with IgG, respectively.

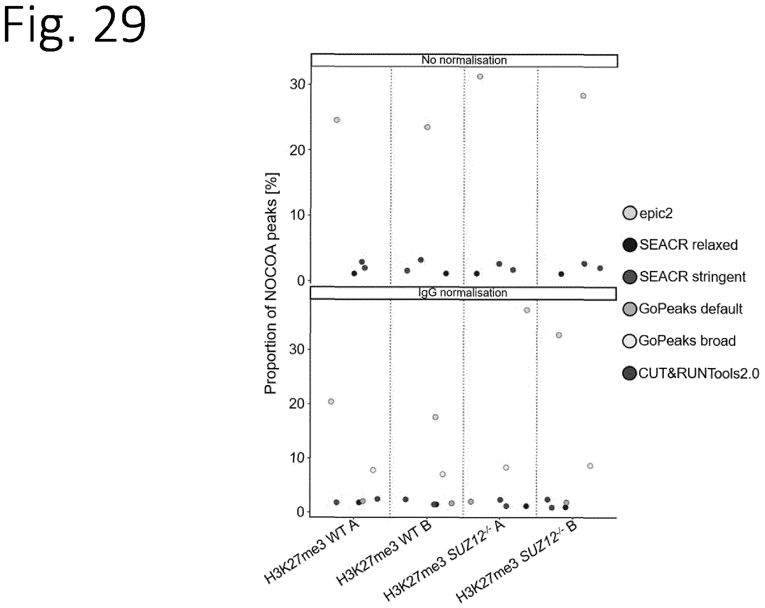

**Figure 29:** NOCOA proportions obtained for H3K27me3 in WT and SUZ12$^{-/-}$ mESCs with different peak callers and/or settings without normalization and after normalization with IgG, respectively.

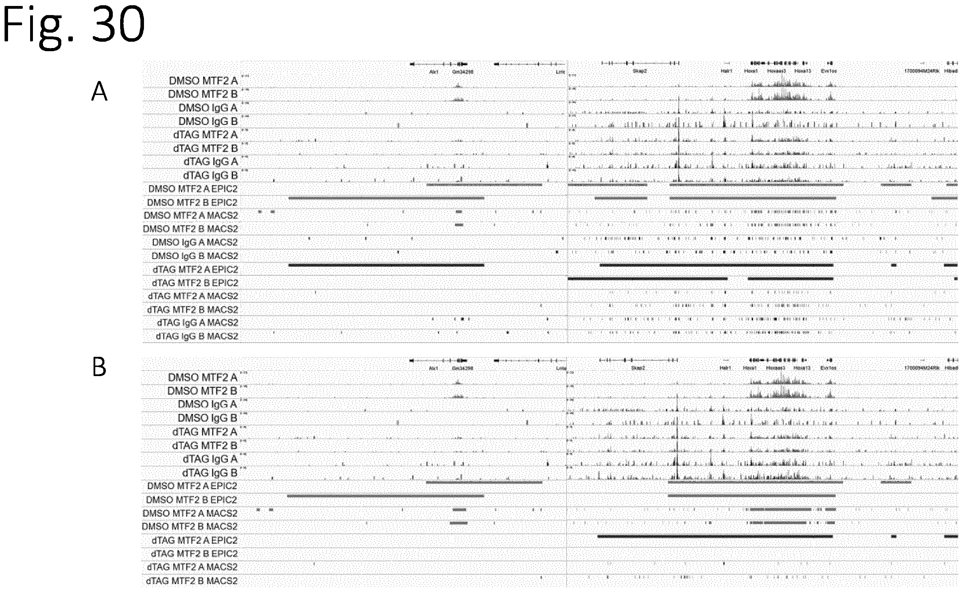

Figure **30:** Genome tracks and peaks of MTF2 in WT-like mES cells (DMSO, green) and mESCs with an MTF2-KO (dTAG, red) using standard settings of peak callers epic2 and MACS2 at the HoxA cluster (right) and *Alx1* gene (left). Blue: IgG samples. (A) without normalisation. (B) with IgG normalisation.

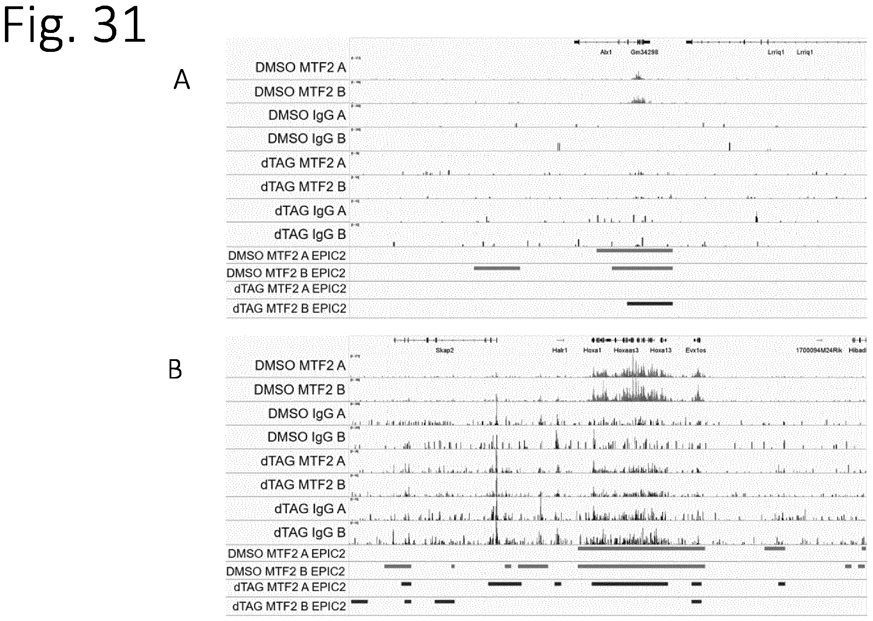

Figure **31:** Genome tracks and peaks of MTF2 in WT-like mES cells (DMSO, green) and mESCs with an MTF2-KO (dTAG, red) using optimized epic2 parameters, i.e. FDR (q-value) of 0.01, window size of 5,000 and gap of 1 using IgG normalisation. (A) *Alx1* gene (B) *HoxA* gene cluster.

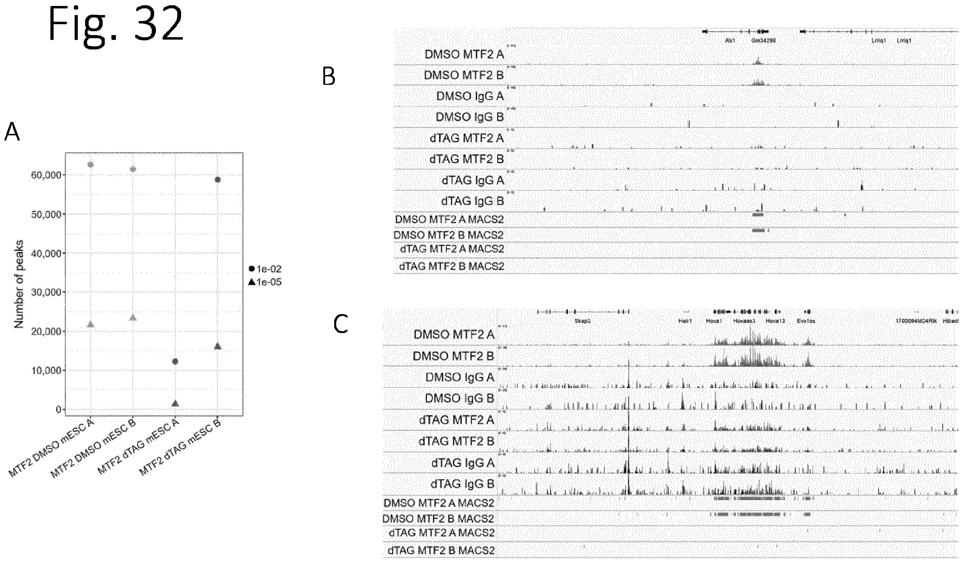

Figure **32:** Number of peaks, genome tracks and peaks of MTF2 in WT-like mES cells (DMSO) and MTF2-KO mESCs (dTAG) using optimized MACS2 peak calling parameters (non-broad option, q-value of 1 e-05, IgG normalisation. (A) Number of peaks using q-values of 1e-02 and 1 e-05. (B) Genome track at Alx1 gene. (C) Genome track at *HoxA* gene cluster.

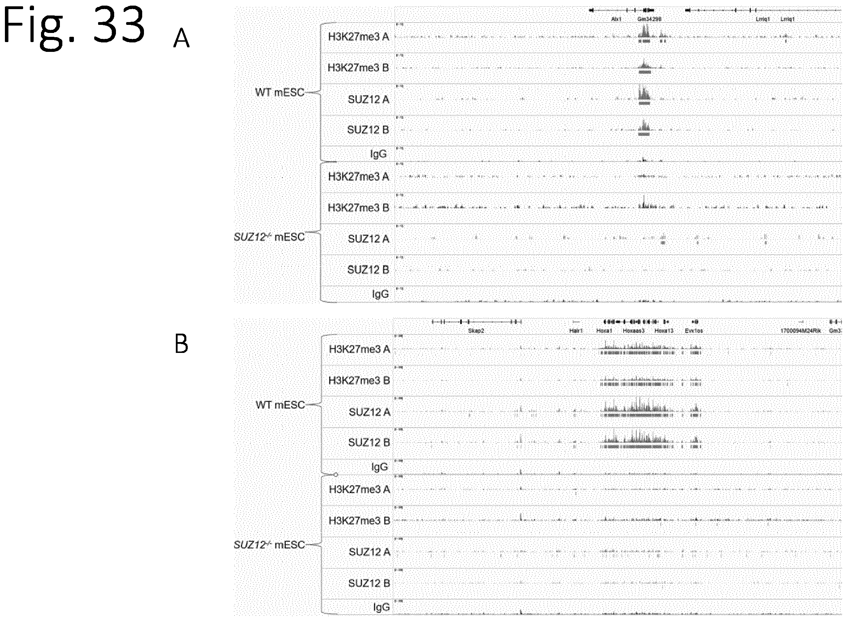

Figure **33:** Genome tracks and peaks of H3K27me3 and SUZ12 in WT and Suz12$^{-/-}$ mESCs using optimized MACS2 peak calling parameters (non-broad option, q-value of 1e-5, IgG normalisation). (A) *Alx1* gene. (B) HoxA gene cluster.

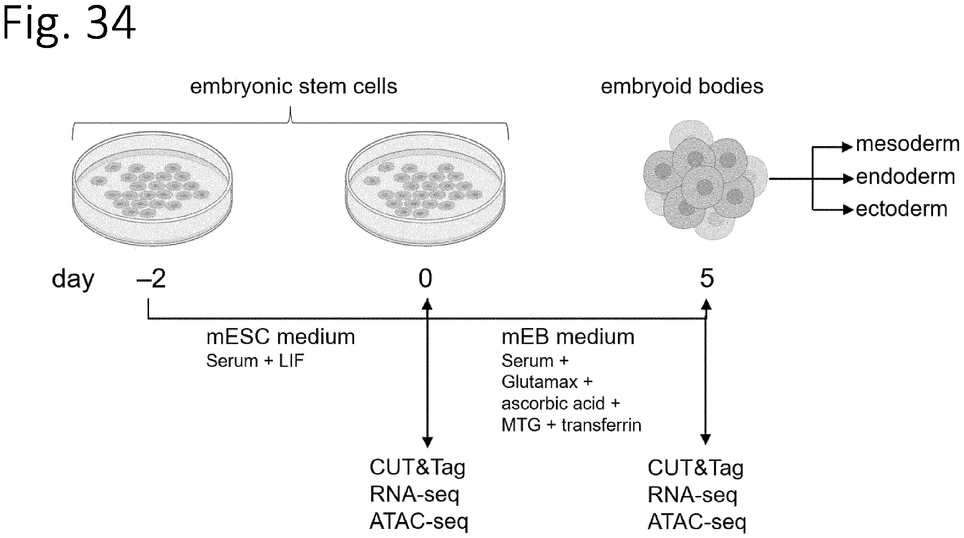

Figure **34:** Schematic overview of the protocol used for differentiation from mouse embryonic stem cells (mESCs) to mouse embryoid bodies (mEBs). Created with BioRender.com.

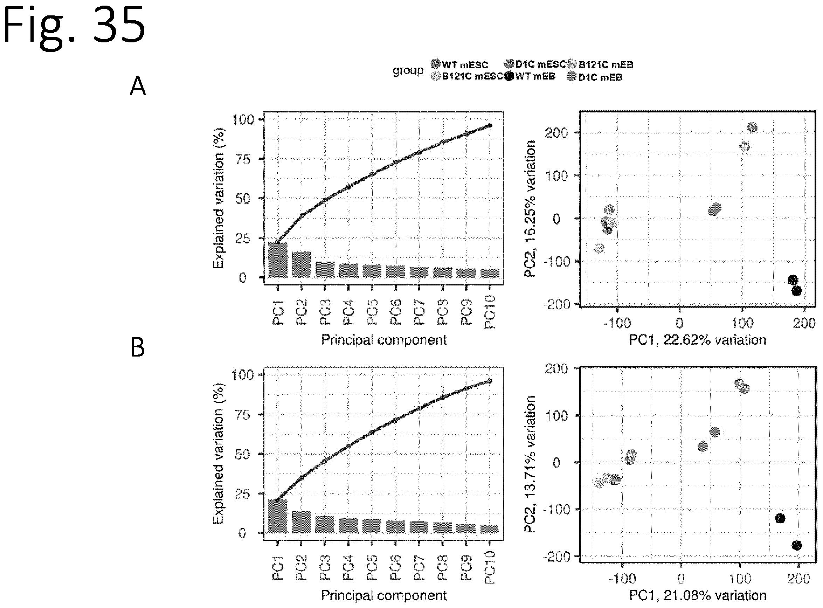

Figure **35:** Principal Component Analysis of RING1 B (A) and SUZ12 (B) using CUT&Tag in mESC and mEB of WT and MLL-AF9-expressing clones.

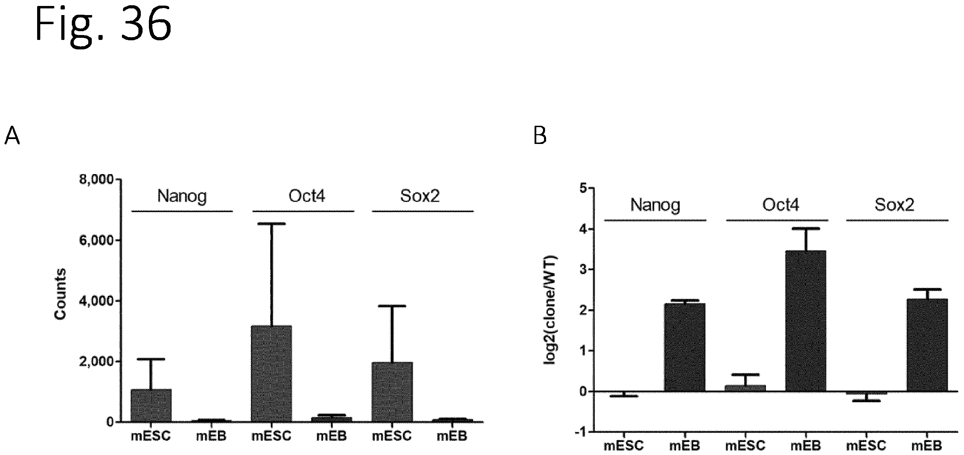

Figure **36:** mRNA expression of stemness markers Nanog, Oct4, and Sox2 during differentiation from mESCs (green) to mEBs (red) in WT and MLL-AF9 clones. (A) Decrease in sternness markers in mEBs in WT cells. Bars represent counts in mean $\pm$ SD of duplicates. (B) Maintained expression of Nanog, Oct4 and Sox2 in the clones expressing MLL-AF9 compared to WT. Bars represent counts in log2(MLL-AF9 clone/WT) mean $\pm$ SD of replicates from average of both MLL-AF9 clones.

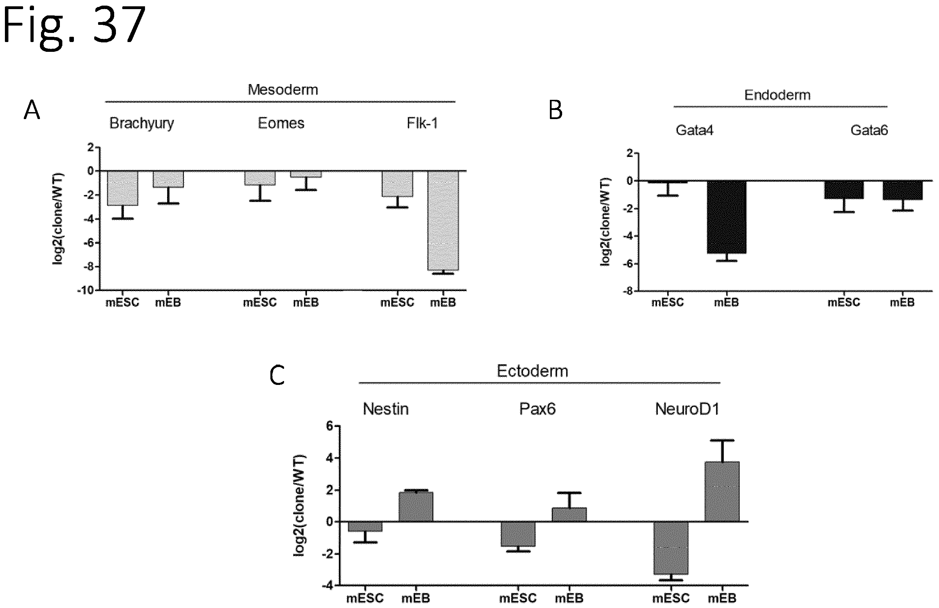

Figure **37:** mRNA expression of lineage commitment genes during differentiation from mESCs to mEBs in clones expressing MLL-AF9. Data represent the mean $\pm$ SD counts expressed in log2(MLL-AF9 clone/WT) of replicates from both clones. (A) Mesoderm lineage markers. (B) Endoderm lineage markers. (C) Ectoderm lineage markers.

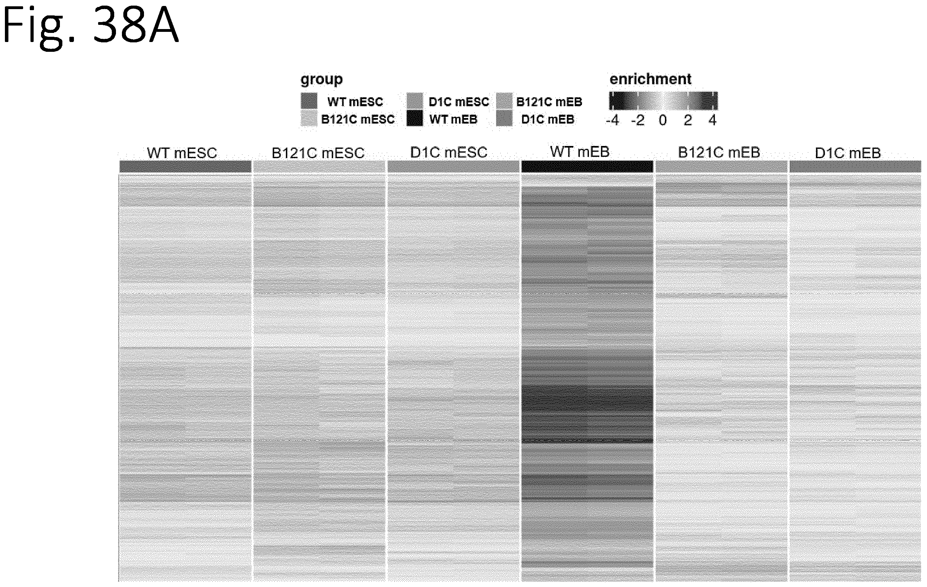

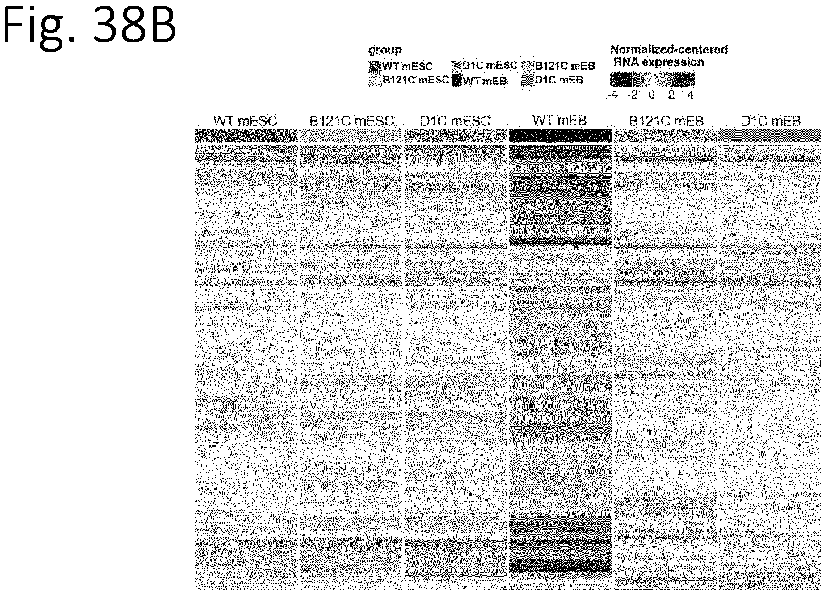

Figure **38:** Expressing MLL-AF9 slows down differentiation and activates different regions than in WT. (A) Chromatin accessibility using ATAC-seq. Heatmap of chromatin accessibility measured by ATAC-seq in WT and MLL-AF9 clones B121C and D1C in mESC (day 0) and mEB (day 5). Peaks were included that show a significant difference above log2FC>1.5(P<0.001) when comparing any of the MLL-AF9 clones in mEBs with WT mEBs, limited to top 2000 peaks by height. (B) Gene expression using mRNA-seq. Heatmap of mRNA features showing a significant difference above log2FC>1.5 (P<0.001) when comparing any of the MLL-AF9 clones in mEBs with WT mEBs. D0: mESC. D5: EBs. It should be noted that what is displayed are not necessarily the same genes in the same order, but a list of the top peaks in each ATAC-seq and mRNA-seq.

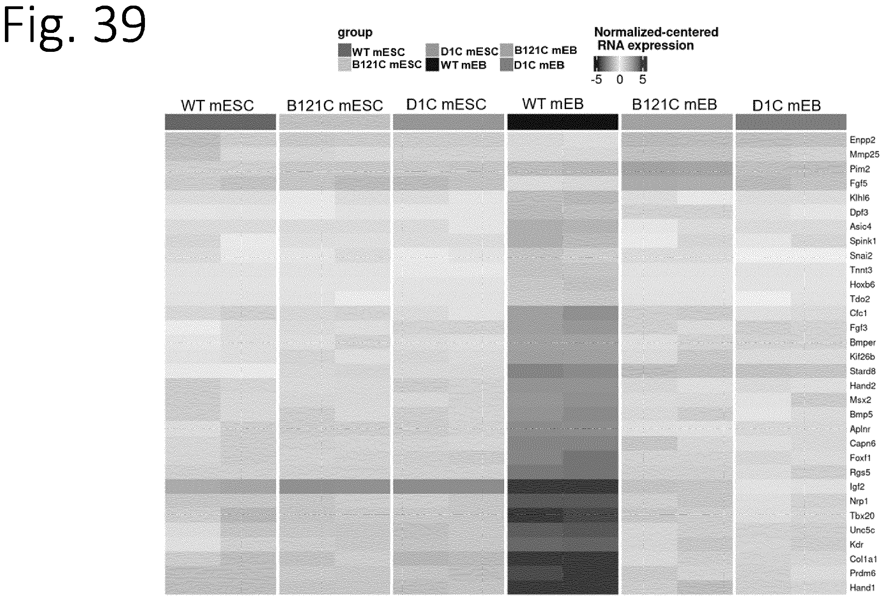

Figure **39:** Excerpt of figure 33B on mRNA expression in mESC and mEBs in WT and clones expressing MLL-AF9. The heatmap displays mRNA features showing a significant difference above log2FC>1.5 (P<0.001) when comparing any of the MLL-AF9 clones with WT in mEBs.

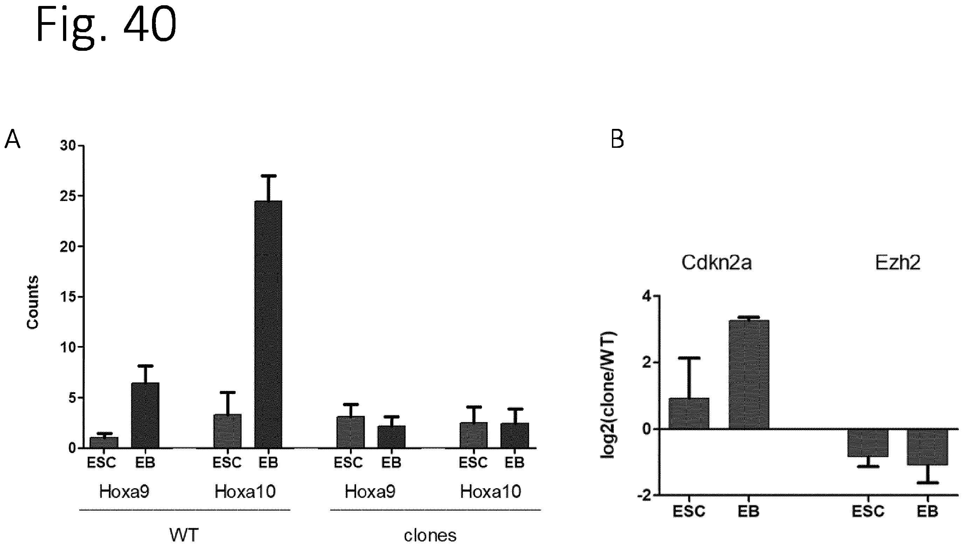

Figure **40:** MLL-AF9 expression does not affect expression of *Hoxa9* or *Hoxa10,* but of *Ezh2* and *Cdkn2a.* (A) Expression of *Hoxa9* and *Hoxa10* in WT and MLL-AF9 clones. Bars represent counts in mean $\pm$ SD of duplicates. (B) Expression of *Cdkn2a* and *Ezh2* in the MLL-AF9 clones in relation to WT cells. Bars represent log2fold changes in mean $\pm$ SD of duplicates.

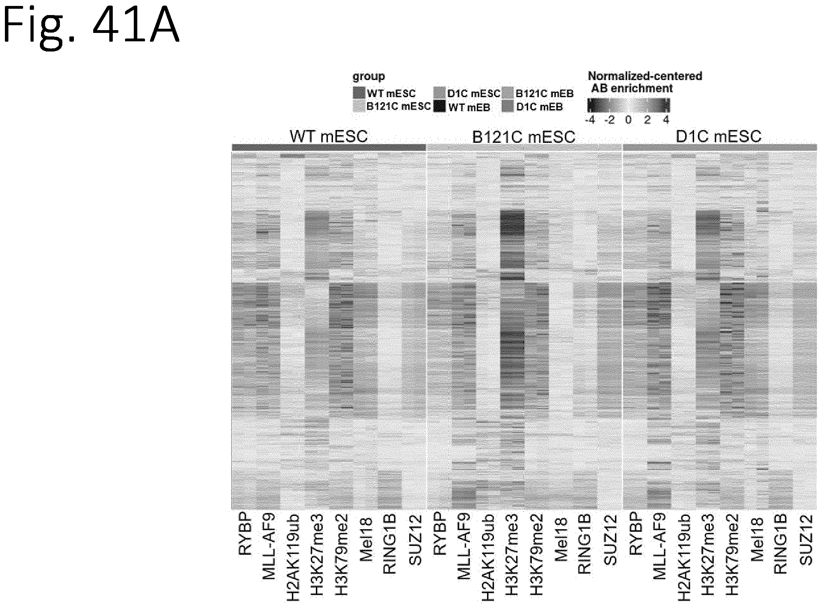

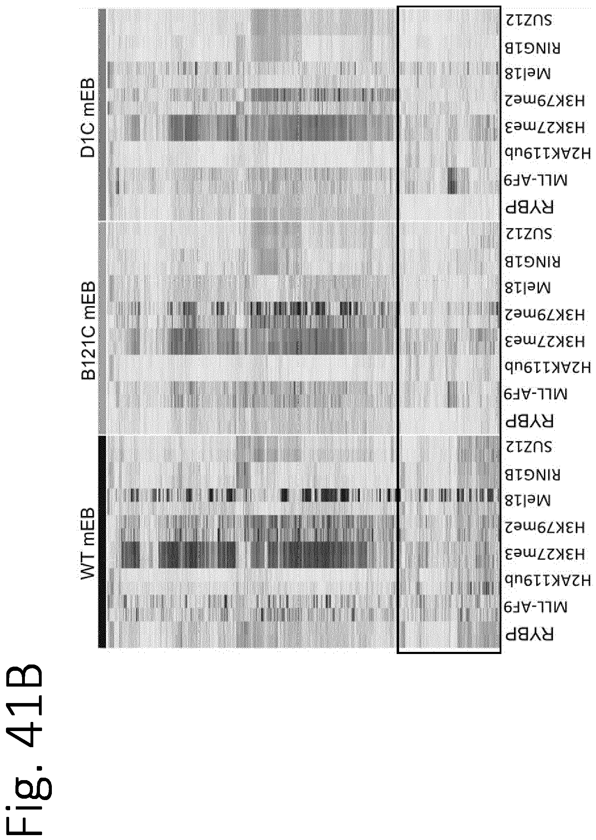

Figure **41:** Heatmaps of top CUT&Tag enrichments for RYBP, MLL-AF9 (FLAG), H2AK119ub, H3K27me3, H3K79me2, Mel18, RING1B, SUZ12. Signal of two replicates per antibody are shown separately. (A) WT and both MLL-AF9 clones at ESC stage. (B) WT and both MLL-AF9 clones at the EB stage. The green circled region indicates some interesting changes in MLL-AF9 clones.

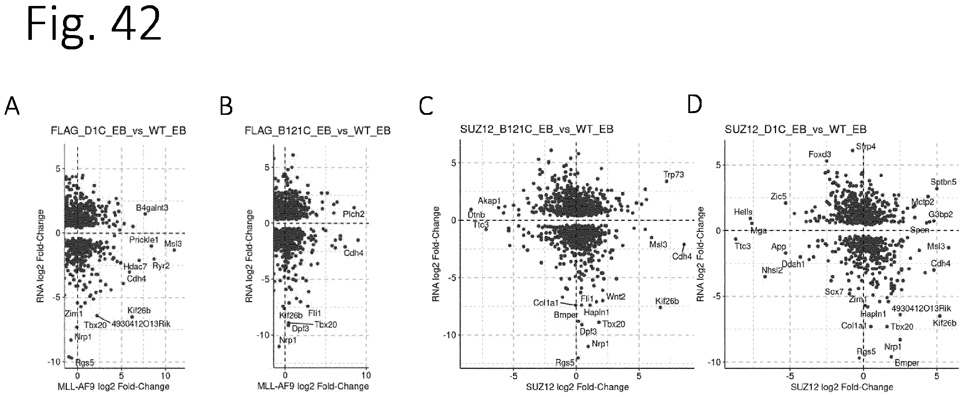

Figure **42:** Changes in gene expression at MLL-AF9- (A,B) and SUZ12- (C,D) bound sites in embryoid bodies of MLL-AF9 clones B121C (A,C) and D1C (B,D) compared to WT embryoid bodies. The MLL-AF9 plots (A, B) show only positive fold changes, as MLL-AF9 is not expressed in WT cells and thus cannot be lost in the clones compared to WT.

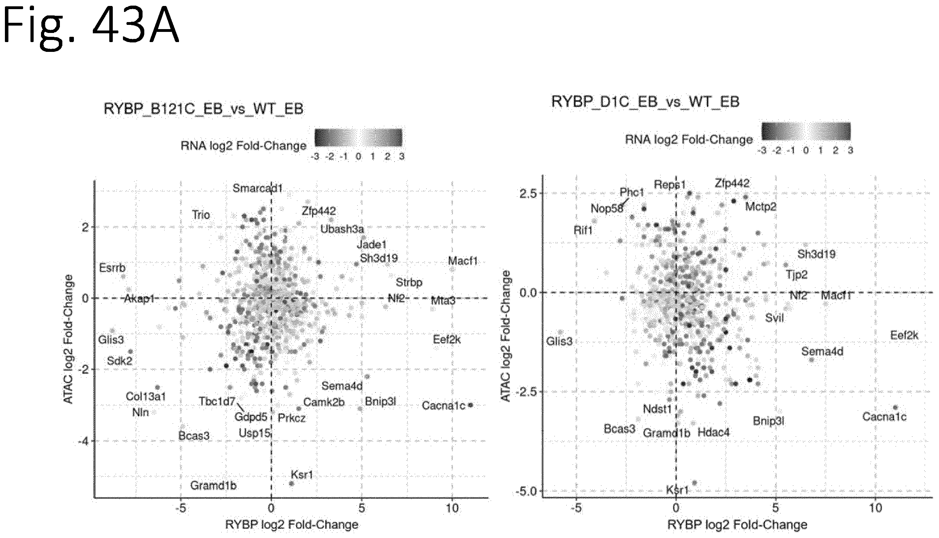

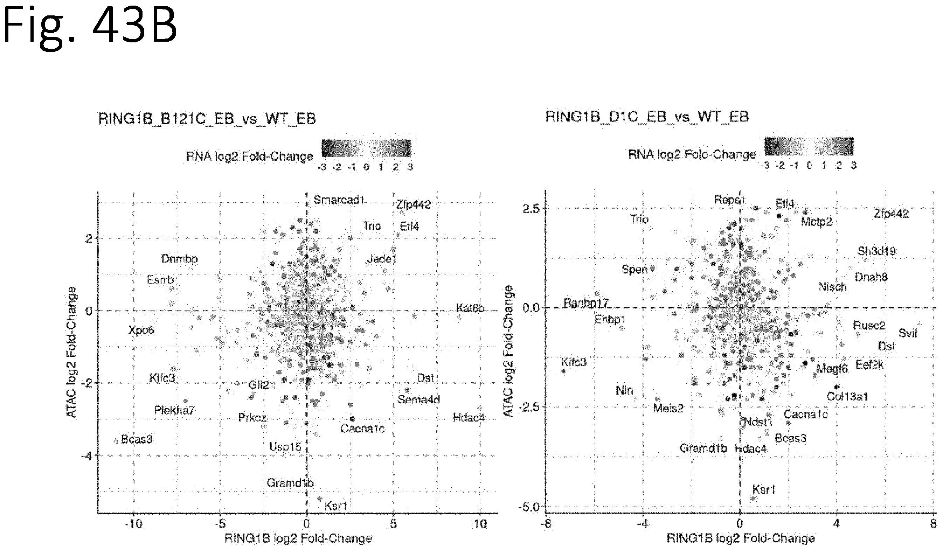

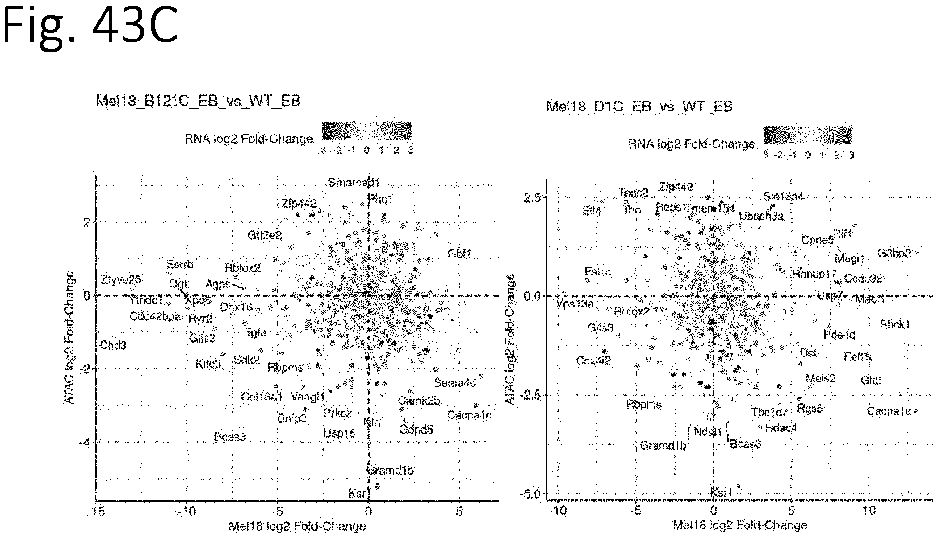

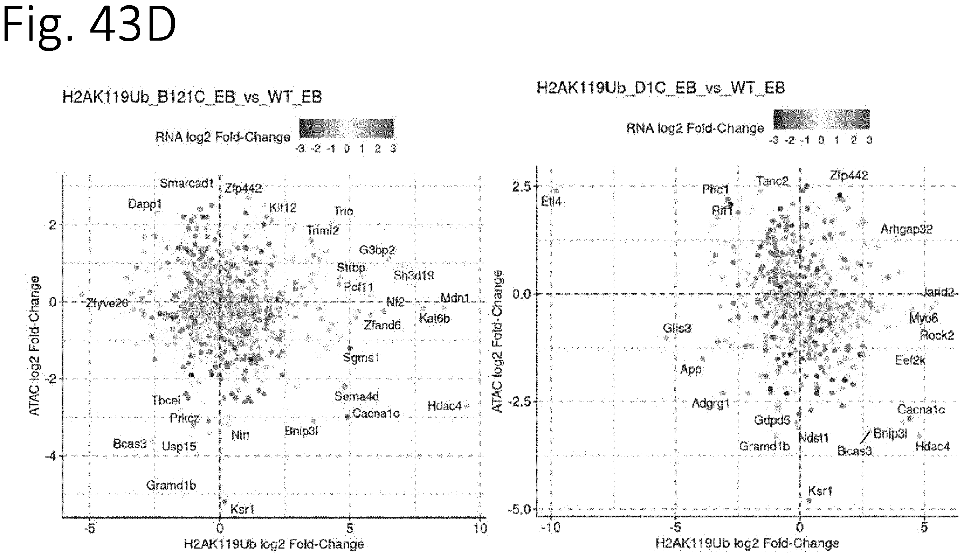

Figure **43:** Scatterplot showing changes in binding of PRC1 protein (A) RYBP, (B) RING1 B and (C) MEL18, and of (D) H2AK119ub in combination with changes in ATAC-seq peaks and mRNA expression in clones expressing MLL- AF9 in EBs. Left: MLL- AF9 clone B121C. Right: MLL- AF9 clone D1C.

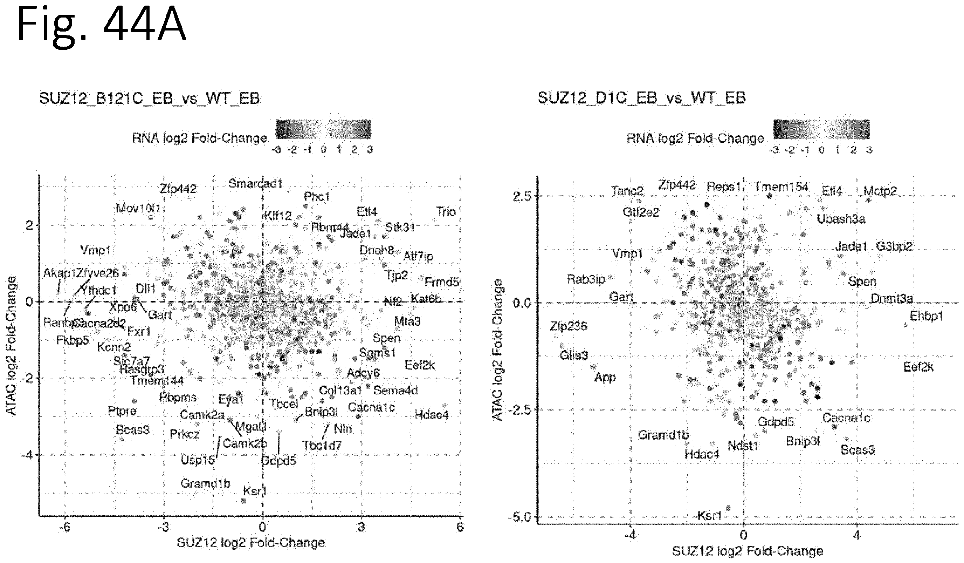

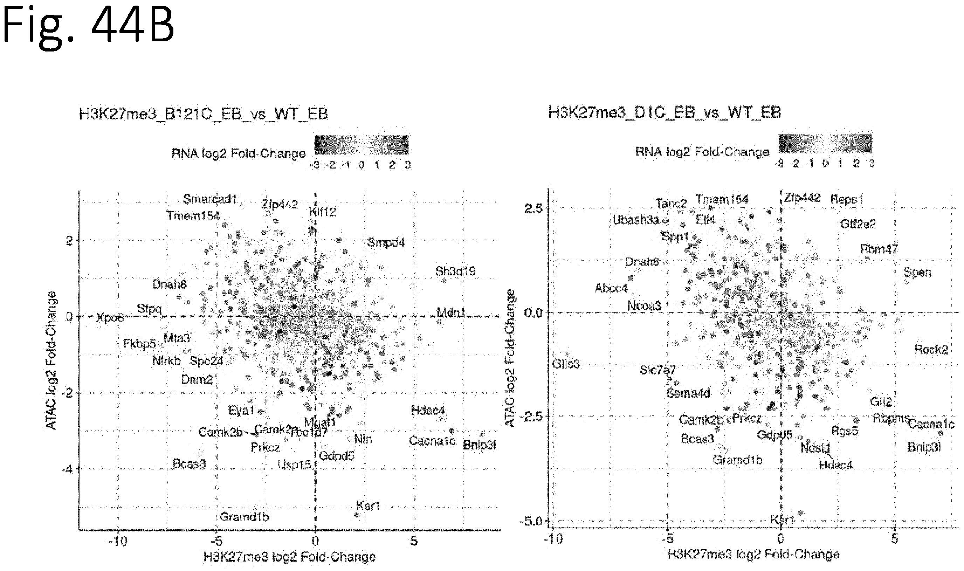

Figure **44:** Scatterplot showing changes in binding of (A) SUZ12 and (B) H3K27me3 in combination with changes in ATAC-seq peaks and mRNA expression in clones expressing MLL-AF9 in EBs. Left: MLL- AF9 clone B121C. Right: MLL- AF9 clone D1C.

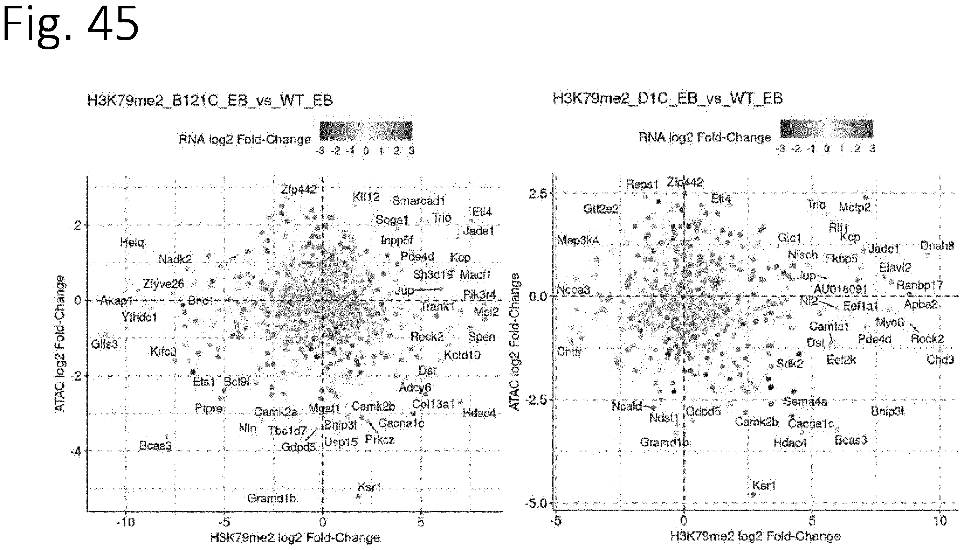

Figure **45:** Scatterplot showing H3K79me2 changes in combination with changes in ATAC- seq peaks and mRNA expression in MLL-AF9 clones in EBs. Left: MLL- AF9 clone B121C. Right: MLL- AF9 clone D1C.

## DETAILED DESCRIPTION OF THE INVENTION

While the following terms are believed to be well understood by one of ordinary skill in the art, the following definitions are set forth to facilitate explanation of the presently disclosed subject matter.

Unless defined otherwise, all technical and scientific terms used herein have the same meaning as commonly understood to one of ordinary skill in the art to which the presently disclosed subject matter belongs.

Further, the terms "a", "an", and "the" refer to "one or more" when used in this application, including the claims.

The term "and/or" when used in describing two or more items or conditions, refers to situations where all named items or conditions are present or applicable, or to situations wherein only one (or less than all) of the items or conditions is present or applicable.

The use of the term "or" in the claims is used to mean "and/or" unless explicitly indicated to refer to alternatives only or the alternatives are mutually exclusive, although the disclosure supports a definition that refers to only alternatives and "and/or." As used herein "another" can mean at least a second or more.

The term "comprising", which is synonymous with "including," "containing," or "characterized by" is inclusive or open-ended and does not exclude additional, unrecited elements or method steps. "Comprising" is a term of art used in claim language which means that the named elements are essential, but other elements can be added and still form a construct within the scope of the claim.

As used herein, the phrase "consisting of" excludes any element, step, or ingredient not specified in the claim. When the phrase "consists of" appears in a clause of the body of a claim, rather than immediately following the preamble, it limits only the element set forth in that clause; other elements are not excluded from the claim as a whole.

As used herein, the phrase "consisting essentially of' limits the scope of a claim to the specified materials or steps, plus those that do not materially affect the basic and novel characteristic(s) of the claimed subject matter.

With respect to the terms "comprising", "consisting of", and "consisting essentially of", where one of these three terms is used herein, the presently disclosed and claimed subject matter can include the use of either of the other two terms.

Unless otherwise indicated, all numbers expressing quantities of time, temperature, volume, diameter, percentage (%), and so forth used in the specification and claims are to be understood as being modified in all instances by the term "about". Accordingly, unless indicated to the contrary, the numerical parameters set forth in this specification and attached claims are approximations that can vary depending upon the desired properties sought to be obtained by the presently disclosed subject matter.

As used herein, the term "about", when referring to a value is meant to encompass variations of in one example $\pm20\%$ or $\pm10\%$, in another example $\pm5\%$, in another example $\pm1$ %, and in still another example $\pm0.1$ % from the specified amount, as such variations are appropriate to perform the disclosed methods.

The term "crowding agent" as used herein may refer to an agent that is used to mimic the physiological conditions of the cells. Nuclei and cells can be filled with about 200 to 400 mg/ml of different macromolecules (chromatin, associated proteins, active transcription machinery etc.) while the volume occupied by liquid is relatively small. A crowding effect may occur when high concentrations of macromolecules are present. Therefore, in the presence of a crowding agents, *in vitro* results obtained with methods that profile chromatin-associated proteins for their chromatin binding sites may tend to better reflect the *in vivo* situation. For example, crowding agents may aid the stabilisation of membranes and proteins. Crowding agents may also stabilise protein folding by displacing liquid because unfolded proteins would need more space and are thermodynamically less favourable when crowding agents are present. Further, at least at lower salt concentrations crowding may increase protein binding to DNA and may thus prevent chromatin proteins such as PRC2 proteins from detaching from DNA. Exemplary crowding agents include sucrose, hexylene glycol and glycerol.

"Permeabilization" as understood herein may allow antibodies or transposomes that are linked to specific binding agents to pass through the cellular membrane and enter the cell. Permeabilization of the cell membranes may be achieved by a chemical treatment (e.g. by adding detergents or solvents) or a physical treatment. The cell may be permeabilized with a permeabilization agent. The cell may be permeabilized by electroporation or biolistics. The permeabilization agent may be a lysolipid or a non-ionic detergent. A cell with a permeabilized cell membrane will generally retain the cell membrane such that the cell's structure remains substantially intact. A cell with a permeabilized membrane is not a "lysed" cell, for example as occurs in standard DNA purification techniques. In contrast, "disrupting" a cell membrane, as used herein, refers to reducing the integrity of a cell membrane such that the cell's structure does not remain intact (e.g., such as during cell lysis). As a test of effectiveness of the permeabilization, cells can first be treated with a permeabilization reagent and then stained with trypan blue (Merck Millipore). The cells-trypan blue mix can be transferred on a microscopy glass, covered with a glass slip, and looked at under a microscope. When the cells turn blue, the permeabilization is successful. Exemplary cell permeabilization reagents include organic solvents, such as methanol and acetone, and detergents, such as saponin (e.g. digitonin), Triton X-100 and Tween-20. In a specific embodiment, the cell permeabilization reagent is a non-saponin detergent such as Triton X-100 and Tween-20,

The term "Good's buffer" as used herein may refer to buffering agents for biochemical and biological research such as those selected and described by Norman Good and colleagues. Exemplary Good's buffers are HEPES, Tricine, MES, ADA, PIPEs, ACES, MPOSO, Cholamine chloride, MOPS, BES, TES, DIPSO, TASO, Acetamidoglycine, POPSO, Acetamidoglycine, POPSO, HEPPSO, HEPPS, Tris, Glycinamide, Glycylglycine, Bicine and TAPS. In conventional CUT&Tag methods, the Good's buffer used is HEPES. In some embodiments of the disclosure, a Good's buffer is used that can chelate $Mg^{2+}$ in solution, for example residuals from the cells, which may help avoiding premature activation of the transposase and unspecific cutting in accessible chromatin. A Good's buffer may further improve the tagmentation process through providing a wider pH range.

The term "transposase" as used herein may refer to an enzyme that catalyses the process of transposition, i.e. the movement of a transposon to a certain part of a genome typically by a cut-and-paste mechanism. Transposases are classified under EC No. 2.7.7. Genes encoding transposases are widespread in the genomes of most organisms and are the most abundant genes known. An exemplary transposase is Transposase (Tnp) Tn5.

The term "transposome" as used herein may refer to a transposase-transposon complex. A conventional way for transposon mutagenesis usually places the transposase on the plasmid. In some such systems, termed "transposomes", the transposase can form a functional complex with a transposon recognition site that is capable of catalysing a transposition reaction. The transposase or integrase may bind to the transposase recognition site and insert the transposase recognition site into a target nucleic acid. This process is also referred to as "tagmentation".

The term "transposon" as used herein may refer to a first DNA molecule comprising a first transposase recognition site and a second DNA molecule comprising a second transposase recognition site. Integration of the transposon (or the two parts of a broken transposon) yields a cleaved (or fragmented) DNA with the first and second DNA molecules integrated on either side of the fragmentation site. In this way, the chromatin DNA is both fragmented and tagged at the fragmentation site. In some examples, the transposase recognition sites have the same sequence, while in other examples, the transposase recognition sites have different sequences. With multiple insertions throughout the chromatin DNA, the DNA can be effectively fragmented into small fragments amenable to analysis by next generation sequencing methods. In some embodiments, the chromatin DNA is contacted with at least two different transposomes, and wherein the different transposomes comprise different DNA sequences. Thus, the tagged chromatin DNA can be tagged at the 5' and 3' end with different transposon sequences. The first and second DNA molecules of the transposon can further include a variety of tag sequences, which can be added covalently to the fragments in the process of the disclosed method.

As used herein, the term "tag" as used herein may refer to a nucleotide sequence that is attached to another nucleic acid to provide the nucleic acid with a functionality. Examples of tags include barcodes, primer sites, affinity tags, and reporter moieties or any combination thereof, such as those described above.

The term "activating the transposase" as used herein may refer to a process that results in excising and tagging the sequence of DNA bound to the chromatin-associated factor of interest wherein the transposase integrates the first and second DNA molecules into chromatin DNA. Activation may be achieved by addition of a divalent cation, e.g. $Mg^{2+}$.

The term "chromatin" as used herein may refer to the complex assembly of DNA and proteins. It may serve as the architectural foundation of eukaryotic cells and allows to orchestrate essential processes such as transcription, replication, and repair. Chromatin may also contain RNA. The fundamental unit of chromatin is the nucleosome which comprises about 146 base pairs of DNA wrapped about 1.7 times in a left-handed turn around a protein octamer of two each of the core histones H3, H4, H2A and H2B. Alternatively, the octamer may be comprised of one or more variant. H1 and H5 linker histones are generally located between nucleosomes. In addition to histones and nucleic acids, chromatin may also comprise proteins that associate with DNA such as transcription machinery (including RNA polymerases), DNA replication machinery, and transcription factors or repressors.

The term "histone" as used herein may refer to small, positively charged proteins around which DNA is wound to form nucleosomes. Histones are central to chromatin organization. About 150 bp of a DNA molecule is wrapped around a histone octamer consisting of two H2A/H2B dimers and two H3/H4 dimers like beads on a string. This nucleosomal arrangement facilitates both compaction and accessibility of the genetic material. The DNA part between nucleosomes is called linker DNA and it accessible for nucleases unlike the DNA around the histones. It varies in length among species with an average of 33 bp. The linker DNA is bound by histone H1 to stabilise the nucleosome and to further condensate the chromatin into a 30 nm fiber. By being organised as nucleosomes, the DNA molecule is compressed to a third of its length. Known human histones include five classes H1/H5, H2A, H2B, H3 and H4. The class H1 includes H1 F0, H1 FNT, H1 FOO, H1 FX, HIST1 H1A, HIST1 H1 B, HIST1 H1 C, HIST1 H1 D, HIST1 H1 E and HIST1 H1 T. Class H2A includes H2AFB1 , H2AFB2, H2AFB3, H2AFJ, H2AFV, H2AFX, H2AFY, H2AFY2, H2AFZ, HIST1 H2AA, HIST1 H2AB, HIST1 H2AC, HIST1 H2AD, HIST1 H2AE, HIST1 H2AG, HIST1 H2AI, HIST1 H2AJ, HIST1 H2AK, HIST1 H2AL, HIST1 H2AM, HIST2H2AA3 and HIST2H2AC. Class H2B includes H2BFM, H2BFS, H2BFWT, HIST1 H2BA, HIST1 H2BB, HIST1 H2BC, HIST1 H2BD, HIST1 H2BE, HIST1 H2BF, HIST1 H2BG, HIST1 H2BH, HIST1 H2BI, HIST1 H2BJ, HIST1 H2BK, HIST1 H2BL, HIST1 H2BM, HIST1 H2BN, HIST1 H2BO and HIST2H2BE. Class H3 includes HISTH3A, HISTH3B, HISTH3C, HISTH3D, HISTH3E, HISTH3F, HISTH3G, HISTH3H, HISTH3I, HISTH3J, HIST2H3C and HIST3H3. Class H4 includes HIST1 H4A, HIST1 H4B, HIST1 H4C, HIST1 H4D, HIST1 H4E, HIST1 H4F, HIST1 H4G, HIST1 H4H, HIST1 H4I, HIST1 H4J, HIST1 H4K, HIST1 H4L and HIST4H4. There are also variants of histones with special roles. Variant histones include H3.3, centromeric H3 variant (cenH3, called also CENPA), H3.1, H3.2, TS H3.4, H3.5, H3.Y, H2A.X, H2A.Z, H2A.Z.2, H2A.B, H2A.L, H2A.P, H2A.1, H2B.1, H2A.J, H2B.1, H2B.W, H2B.Z, H2B.E. H1 and H5 linker histones are generally located between nucleosomes. Histones may control accessibility and expression of the region of DNA (or specific locus) around which they are bound or regions of DNA with which they associate. For example, histones with repressive modifications to their N-terminal tails tend to promote tightly compacted chromatin that prevents transcriptional machinery from accessing the DNA. In contrast, histones with modifications to their N-terminal tails that increase access to the DNA tend to promote chromatin with high levels of gene expression.

The term "histone modification" as used herein may refer to a post-transcriptional modification of the N-terminal tails of the histone proteins. Histone modifications may play a crucial role in regulating chromatin structure and function. Exemplary histone modifications include methylation, acetylation, propionylation, butyrylation, crotonylation, 2-hydroxyisobutyrylation, malonylation, succinylation and ribosylation. Specific histone modifications include lysine methlyation, arginine methlyation, lysine acetylation, and serine/threonine/tyrosine phosphorylation. Histone modifications may be removed again.

The term "writer" as used herein may refer to enzymes responsible for catalyzing a histone modification. Exemplary writers include histone acetyltransferases (HATs) and histone methyltransferases (HMTs). Histone methyltransferases (HMTs) may add methyl groups to lysine and arginine residues. There are two main families: HMTs with a Su(var)3-9, Enhancer of Zeste and Trithorax (SET) domain only catalyse lysine residues, whereas HMTs without a SET domain add methyl groups to arginine residues. An exception of this rule is DOT1L which catalyses the methylation of lysine 79 of histone H3 despite the absence of a SET domain, causing the histone marks H3K79me1/2/3. Enhancer of Zeste Homologue 1 (EZH1) and its homologue EZH2 are the only histone methylases responsible for the mono-, di- and tri-methylation of lysine 27 of histone H3, associated with gene repression and chromatin compaction. EZH1 and EZH2 are part of the Polycomb Repressive Complex 2. The enzymes responsible for catalysing acetylation of lysine residues are called histone acetyltransferases (HATs). HAT enzymes include but are not limited to the GNAT superfamily including Gcn5, SAGA, SLIK, STAGA, ADA, A2, Gcn5L, p300/CREB-binding protein associated factor (PCAF), Elp3, HPA2, and HAT1), the MYST family including MOZ (Monocytic Leukemia Zinc Finger Protein), Ybf2/Sas3, Sas2, Tip60, Esa1, MOF, MORF, and HBO1, the p300/CBP family including adenoviral E1A associated protein of 300 kDa (p300) and the CREB-binding protein (CBP), and other HATs such as steroid receptor coactivator 1 (SRC 1), ATF-2, and TAFII250. Acetyl-Coenzyme A is a common source of the acetyl group transferred to a target histone lysine residue by HAT enzymes. Acetylation removes the positive charge of lysines. This way, the interaction of the histone proteins with the DNA is weakened since the backbone of DNA consists of negatively charged phosphate groups. It causes a more open chromatin, called euchromatin, and a higher accessibility for the transcription machinery. Generally, euchromatin and acetylated histone tails are associated with actively transcribed genes. Histone ubiquitin-transferases may catalyse monoubiquitinylation. Phosphate groups may be added to serine residues by histone kinases.

The term "erasers" as used herein may refer to enzymes responsible for removing a histone modification. Exemplary erasers include histone deacetylases (HDACs) and histone demethylases (HDMs). Histone monoubiquitinylation may be removed by deubiquitinating enzymes. Phosphate groups may be removed by phosphatases.

The term "reader" or "reader protein" as used herein may refer to a protein that is able to recognize a histone modification by binding specifically to it. Readers may recognise modified histone tails by certain domains. When a protein consists of a bromodomain, it may bind to acetylated lysines. Thereafter, it may recruit other proteins and cause other actions. Methylated histones may be bound by proteins with a chromodomain. Some chromodomains are specific for certain histone marks, such as the one of CBX proteins in canonical Polycomb Repressive Complex 1 that only recognises H3K27me3.

The term "transcription factor" as used herein may refer to a protein that regulates transcription. A transcription factor may be a protein that binds to specific DNA sequences, thereby controlling the rate of transcription of genetic information from DNA to messenger RNA. In particular, transcription factors may regulate the binding of RNA polymerase and the initiation of transcription. A transcription factor may bind upstream or downstream to either enhance or repress transcription of a gene by assisting or blocking RNA polymerase binding. The term transcription factor includes both inactive and activated transcription factors. Typically, a transcription factor may affect regulation of gene expression. Exemplary transcription factors include but are not limited to AAF, abl, ADA2, ADA-NF1, AF-1, AFP1, AhR, ATIN3, ALL-1, alpha-CBF, alpha-CP 1, alpha-CP2a, alpha-CP2b, alphaHo, alphaH2-alphaH3, Alx-4, aMEF-2, AML1, AMLIa, AMLIb, AMLIc, AMLIDeltaN, AML2, AML3, AML3a, AML3b, AMY-1L, A-Myb, ANF, AP-1, AP-2alphaA, AP-2alphaB, AP-2beta, AP-2gamma, AP-3 (1), AP-3 (2), AP-4, AP-5, APC, AR, AREB6, Arnt, Arnt (774 M form), ARP-1, ATBF1-A, ATBF1-B, ATF, ATF-1, ATF-2, ATF-3, ATF-3deltaZIP, ATF-a, ATF-adelta, ATPF1, Barhll, BarhI2, Barxl, Barx2, Bcl-3, BCL-6, BD73, beta-catenin, Binl, B-Myb, BP1, BP2, brahma, BRCA1, Brn-3a, Brn-3b, Brn-4, BTEB, BTEB2, B-TFIID, C/EBPalpha, C/EBPbeta, C/EBPdelta, CACCbinding factor, Cart-1, CBF (4), CBF (5), CBP, CCAAT-binding factor, CCMT-binding factor, CCF, CCG1, CCK-la, CCK-lb, CD28RC, cdk2, cdk9, Cdx-1, CDX2, Cdx-4, CFF, Chxl1O, CLIMI, CLIM2, CNBP, CoS, COUP, CPL, CPIA, CPIC, CP2, CPBP, CPE binding protein, CREB, CREB-2, CRE-BPI, CRE-BPa, CREMalpha, CRF, Crx, CSBP-1, CTCF, CTF, CTF-1, CTF-2, CTF-3, CTF-5, CTF-7, CUP, CUTLI, Cx, cyclin A, cyclin TI, cyclin T2, cyclin T2a, cyclin T2b, DAP, DAXI1, DB1, DBF4, DBP, DbpA, DbpAv, DbpB, DDB, DDB-1, DDB-2, DEF, deltaCREB, deltaMax, DF-1, DF-2, DF-3, Dlx-1, Dix-2, Dlx-3, Dlx4 (long isoform) Dlx-4 (short isoform, Dlx-5, Dlx-6, DP-1, DP-2, DSIF, DSIF-pl4, DSIF-pl60, DTF, DUX1, DUX2, DUX3, DUX4, E, EI 2, E2F, E2F+E4, E2F+pl07, E2F-1, E2F-2, E2F-3, E2F-4, E2F-5, E2F-6, E47, E4BP4, E4F, E4F1, EATF2, EAR2, EBP-80, EC2, EF1, EF-C, EGR1, EGR2, EGR3, EllaE-A, EllaE-B, EllaE-Calpha, EllaECbeta, EivF, Elf-1, Elk-1, Emx-1, Emx-2, Emx-2, En-1, En-2, ENH-bind. prot, ENKTF-1, EPASI, epsilonFI, ER, Erg-1, Erg-2, ERR1, ERR2, ETF, Ets-1, Ets-1 deltaVil, Ets-2, Evx-1, F2F, factor 2, Factor name, FBP, f-EBP, FKBP59, FKHL18, FKHRL1P2, Fli-1, Fos, FOXB1, FOXC1, FOXC2, FOXD1, FOXD2, FOXD3, FOXD4, FOXE1, FOXE3, FOXF1, FOXF2, FOXGla, FOXGlb, FOXGlc, FOXHI1, FOXI1, FOXJla, FOXJlb, FOXJ2 (long isoform), FOXJ2 (short isoform), FOXJ3, FOXKla, FOXKlb, FOXKllc, FOXL1, FOXMlla, FOXMIIb, FOXMlc, FOXN1, FOXN2, FOXN3, FOXOIa, FOXOlb, FOX02, FOX03a, FOX03b, FOX04, FOXP1, FOXP3, Fra-1, Fra-2, FTF, FTS, G factor, G6 factor, GABP, GABP-alpha, GABP-betal, GABP-beta2, GADD 153, GAF, gammaCMT, gammaCACI, gammaCAC2, GATA-1, GATA-2, GATA-3, GATA-4, GATA-5, GATA-6, Gbx-1, Gbx-2, GCF, GCMa, GCNS, GF1, GLI, GLI3, GR alpha, GR beta, GRF-1, Gsc, Gscl, GT-IC, GTITA, GT-IIBalpha, GT-IIBbeta, HITF1, HITF2, H2RIIBP, H4TF-1, H4TF-2, HAND1, HAND72, HB9, HDACI, HDAC2, HDAC3, hDaxx, heat-induced factor, HEB, HEBI-p67, HEBI-p94, HEF-1 B, HEF-1T, HEF-4C, HENI1, HEN2, Hesxl, Hex, HIF-1, HIF-lalpha, HIF-lbeta, HiNF-A, HiNF-B, HINF-C, HINF-D, HiNF-D3, HiNF-E, HiNF-P, HIP1, HIV-EP2, Hlf, HLTF, HLTF (Metl23), HLX, HMBP, HMG I, HMG I(Y), HMG Y, HMGI-C, HNF-IA, HNF- IB, HNF-IC, HNF-3, HNF-3alpha, HNF-3beta, HNF-3gamma, HNF4, HNF-4alpha, HNF4alphal, HNF-4alpha2, HNF-4alpha3, HNF- 4alphad4, HNF4gamma, HNF-6alpha, hnRNP K, HOX11, HOXAI, HOXAIO, HOPXAi PL2, HOXA11, HOXA13, HOXA2, HOXA3, HOXA4, HOXAS, HOXA6, HOXA7, HOXA9A, HOXA9B, HOXB-1, HOXB13, HOXB2, HOXB3, HOXB4, HOXBS, HOXB6, HOXAS, HOXB7, HOXBS, HOXB9, HOXC10, HOXC11, HOXC12, HOXC13, HOXC4, HOXCS, HOXC6, HOXC8, HOXC9, HOXD10, HOXD11, HOXD12, HOXD13, HOXD3, HOXD4, HOXD8, HOXD9, Hp55, Hp65, HPX42B, HrpF, HSF, HSF1 (long), HSF1 (short), HSF2, hsp56, Hsp90, IBP-1, ICER-II, ICERligamma, ICSBP, Idl, Idl H', 1d2, 1d3, Id3/Heir-1, IF1, IgPE-1, IgPE-2, IgPE-3, IkappaB, IkappaBalpha, IkappaB-beta, IkappaBR, II-1 RF, IL-6 RE-BP, 11-6 RF, INSAF, IPF1, IRF-1, IRF-2, B, IRX2a, Irx-3, Irx-4, ISGF-1, ISGF-3, ISGF3alpha, ISGF-3gamma, 1st- 1, ITF, ITF-1, ITF-2, JRF, Jun, JunB, JunD, kappay factor, KBP-1, KER1, KER-1, Koxl, KRF-1, Ku autoantigen, KUP, LBP-1, LBP-la, LBXI, LCR-FI, LEF-1, LEF-IB, LF-AI, LHX1, LHX2, LHX3a, LHX3b, LHXS, LHX®6.1a, LHX6.1b, LIT-1, Lmol, Lmo2, LMX1A, LMXI1B, L-Myl (long form), L-Myl (short form), L-My2, LSF, LXRalpha, LyF-1, Lyl-I, M factor, Madl, MASH-1, Maxi, Max2, MAZ, MAZ1, MB67, MBF1, MBF2, MBF3, MBP-1 (1), MBP-1 (2), MBP-2, MDBP, MEF-2, MEF-2B, MEF-2C (433 AA form), MEF-2C (465 AA form), MEF-2C (473 M form), MEF-2C/delta32 (441 AA form), MEF-2D00, MEF-2D0B, MEF-2DA0O, MEF-2DAO, MEF-2DAB, MEF-2DA'B, Meis-1, Meis-2a, Meis-2b, Meis-2¢, Meis-2d, Meis-2e, Meis3, Meoxl, Meoxla, Meox2, MHox (K-2), Mi, MIF-1, Miz-1, MM-1, MOP3, MR, Msx-1, Msx-2, MTB-Zf, MTF-1, mtTFl, Mxil, Myb, Myc, Myc 1, Myf-3, Myf-4, Myf-5, Myf-6, MyoD, MZF-1, NCI, NC2, NCX, NELF, NER1, Net, NF III-a, NF NF-1, NF-1A, NF-1B, NF-1X, NF-4FA, NF-4FB, NF-4FC, NF-A, NF-AB, NFAT-1, NF-AT3, NF-Atc, NF-Atp, NF-Atx, NfetaA, NF-CLEOa, NF-CLEOb, NFdeltaE3A, NFdeltaE3B, NFdeltaE3C, NFdeltaE4A, NFdeltaE4B, NFdeltaE4C, Nfe, NF-E, NF-E2, NF-E2 p45, NF-E3, NFE-6, NF-Gma, NF-GMb, NF-IL-2A, NF-IL-2B, NF-jun, NF-kappaB, NF-kappaB(-like), NF-kappaBI, NF-kappaB 1, precursor, NF-kappaB2, NFkappaB2(p49), NF-kappaB2 precursor, NF-kappaEl, NF-kappaE2, NF-kappaE3, NF-MHCIIA, NFMHCIIB, NF-muEl, NF-muE2, NF-muE3, NF-S, NF-X, NF-X1, NF-X2, NF-X3, NF-Xc, NF-YA, NF-Zc, NF-Zz, NHP-1, NHP-2, NHP3, NHP4, NKX2-5, NKX2B, NKX2C, NKX2G, NKX3A, NKX3A vl, NKX3A v2, NKX3A v3, NKX3A v4, NKX3B, NKX6A, Nmi, N-Myc, N-Oct-2alpha, NOct-2beta, N-Oct-3, N-Oct-4, N-Oct-5a, N-Oct-5b, NP-TCII, NR2E3, NR4A2, Nrfl, Nrf-1, Nrf2, NRF-2betal, NRF-2gammal, NRL, NRSF form 1, NRSF form 2, NTF, 02, OCA-B, Oct-1, Oct-2, Oct-2.1, Oct-2B, Oct-2C, Oct-4A, OctdB, Oct-5, Oct-6, Octa-factor, octamer-binding factor, oct-B2, oct-B3, Otxl, Otx2, OZF, pl07, pl30, p28 modulator, p300, p38erg, p43, p49erg,-p33, p53, pSierg, p65delta, p67, Pax-1, Pax-2, Pax-3, Pax-3A, Pax-3B, Pax-4, Pax-3, Pax-6, Pax-6/Pd-5a, Pax-7, Pax-8§, Pax-8a, Pax-8b, Pax-8c, Pax-8d, Pax-8e, Pax-8f, Pax-9, Pbx-la, Pbx-lb, Pbx-2, Pbx-3a, Pbx-3b, PC2, PC4, PCS, PEA3, PEBP2alpha, PEBP2beta, Pit-1, PITX1, PITX2, PITX3, PKNOXI1, PLZF, PO-B, Pontin52, PPARalpha, PPARbeta, PPARgammal, PPARgamma2, PPUR, PR, PR A, pRb, PRD1-BF1, PRDI-BFc, Prop-1, PSE1, P-TEFb, PTF, PTFalpha, PTFbeta, PTFdelta, PTFgamma, Pu box binding factor, Pu box binding factor (B JA-B), PU.1 , PuF, Pur factor, RI , R2, RAR-alphal, RAR-beta, RAR-beta2, RAR-gamma, RAR-gammal, RBP60, RBP-Jkappa, Rel, RelA, RelB, RFX, RFX], RFX2, RFX3, RFXS, RF-Y, RORalphal, RORalpha2, RORalpha3, RORbeta, RORgamma, Rox, RPFI, RPGalpha, RREB-1, RSRFC4, RSRFC9, RVF, RXR-alpha, RXR-beta, SAP-la, SAPlb, SF-1, SHOX2a, SHOX2b, SHOXa, SHOXb, SHP, SIII-pl 10, SIII-pl5, SHI-pl8, SIM', Six-1, Six-2, Six-3, Six-4, Six-5, Six-6, SMAD-1, SMAD-2, SMAD-3, SMAD-4, SMAD-5, SOX-11, SOX- 12, Sox-4, Sox-5, SOX-9, Spl, Sp2, Sp3, Sp4, Sph factor, Spi-B, SPIN, SRCAP, SREBP-la, SREBP-1b, SREBPle, SREBP-2, SRE-ZBP, SRF, SRY, SRP, Staf-50, STATIalpha, STATIbeta, STAT2, STAT3, STAT4, STAT6, T3R, T3R-alphal, T3R-alpha2, T3R-beta, TAF(I)110, TAF(I)48, TAF(I)63, TAF(I)100, TAFI)125, TAFII)135, TAF(II)170, TAF(II)18, TAFI)20, TAF(I)250, TAF(ID)250Delta, TAF(II)28, TAFI)30, TAF(ID)31, TAF(ID55, TAF(IT)70-alpha, TAF(IT)70-beta, TAF(I)70-gamma, TAF- I, TAF-II, TAF-L, Tal-1, Tal-Ibeta, Tal-2, TAR factor, TBP, TBX1A, TBX1B, TBX2, TBX4, TBXS (long isoform), TBXS (short isoform), TCF, TCF-1, TCF-1A, TCF-1B, TCF-1C, TCF-1D, TCF-1E, TCF-1F, TCF-1G, TCF-2alpha, TCF-3, TCF-4, TCF-4(K), TCF-4B, TCF-4E, TCFbetal, TEF-1, TEF-2, tel, TFE3, TFEB, TFIIA, TFIIA-alpha/beta precursor, TFITAalpha/beta precursor, TFIIA-gamma, TFIIB, TFIID, TFIE, TFIIE-alpha, TFIIE-beta, TFIIF, TFIIFalpha, TFIIF-beta, TFIIH, TFIIH\*, TFIIH-CAK, TFIIH-cyclin H, TFIIH-ERCC2/CAK, TFIIHMAT, TFIIH-MO015, TFIIH-p34, TFIIH-p44, TFIIH-p62, TFIIH-p80, TFITH-p90, TFII-1, Tf-LFI, Tf-LF2, TGIF, TGIF2, TGT3, THRAI, TIF2, TLE1, TLX3, TMF, TR2, TR2-11, TR2-9, TR3, TR4, TRAP, TREB-1, TREB-2, TREB-3, TREFI, TREF2, TRF (2), TTF-1, TXRE BP, TxREF, UBF, UBP-1, UEF-1, UEF-2, UEF-3, UEF-4, USF1, USF2, USF2b, Vav, Vax-2, VDR, vHNF-1A, vHNF-IB, vHNF-IC, VITF, WSTF, WT1, WT11I, WT1 I-KTS, WT1 I-del2, WTI-del2, X2BP, XBP-1, XW-V, XX, YAF2, YB-1, YEBP, YY, ZEB, ZF1, ZF2, ZFX, ZHX1, ZIC2, ZID, ZNF 174, amongst others.

The term "chromatin-associated factor" as used herein may refer to any molecule, for instance a protein, that is able to bind one or more chromatin binding sites. Exemplary chromatin-associated factors include histones and non-histone proteins. Exemplary non-histone proteins include transcription factors, writers, readers and erasers such as those mentioned above.

The term "Chromatin immunoprecipation (ChIP)" as used herein may refer to a method involving crosslinking of chromatin that is used to determine whether a particular protein binds to or is localized to a specific DNA sequence. ChIP has been used to study DNA-protein interactions for decades. In a conventional ChIP workflow, harvested cells are crosslinked with formaldehyde to ensure covalent reversible proteins crosslinking to DNA. Cells are lysed and the chromatin is sheared by sonication. These steps are known as chromatin preparation. Antibodies against a protein or histone modification of interest bind to their epitope on the chromatin. The antibodies also bind to the Protein A-coated magnetic beads. Therefore, manually processed samples can be washed easily when the beads and the chromatin bound to them are retained by a magnet. This also allows to remove the non-target chromatin part without centrifugation. Immunoprecipitated DNA is typically eluted from the beads using SDS-containing buffer, the crosslinking is typically released by heating at 65°C in the presence of SDS and sodium chloride. The recovered DNA can be purified and can be used for the enrichment check by qPCR, library preparation and sequencing.

The term "Cleavage Under Targets and Tagmentation" or "CUT&Tag", as used herein, may be used to describe methods for chromatin profiling without the need for chromatin preparation. In conventional protocols, cells are bound to magnetic concanavalin A-coated beads during the process up to DNA extraction and purification. An antibody against the desired chromatin mark enters the permeabilized cells and marks the site in the genome. A subsequently added fusion protein consisting of protein A and the transposase Tn5 is bound to the antibody. After binding of the protein A part to the antibody and activation of Tn5, the latter cuts the DNA and incorporates sequencing adapters at the site. These can be sequenced after extraction and PCR amplification. CUT&Tag can be used to study transcription factors and chromatin alterations in various species, including humans and mice. Permeabilization in conventional CUT&Tag protocols can be performed using digitonin. In some embodiments, CUT&Tag can be performed using a non-saponin detergent, for instance Triton X-100 or Tween-20.

The term "Chromatin immunocleavage" or "ChIC" as used herein may refer to a chromatin profiling technique. It may use a fusion protein of micrococcal nuclease and protein A (pAMNase) to fragment the chromatin at target sites. After a permeabilisation of the cells, an antibody enters the cells, binds to its epitope on the chromatin mark of interest, and thereby marks the site in the genome. The fusion protein pA-MNase can be tethered to the antibody and bind it. After activation, the MNase part cuts the DNA and DNA sequences are released into solution. After the ChIC workflow, a subsequent library preparation may follow to transform these DNA fragments into sequencing libraries ready to be analysed by NGS. MNase preferably binds at linker regions; therefore, peaks appear ca. 100 to 200 bp from known binding sites. In comparison, the resolution in ChIP may be in range of 1,000 bp.

The term "Cleavage Under Targets and Release Using Nuclease" or "CUT&RUN" as used herein may refer to another chromatin profiling technique. Here, cells are bound to magnetic beads coated with concanavalin A throughout the process until DNA extraction and purification, allowing wash steps without centrifugation and the sample loss associated with centrifugation. In addition, to reduce the appearance of background breaks in the DNA, incubation conditions typically need to be adjusted during the MNase reaction. This may require precise, temperature-sensitive work and depend on the abundance and availability of the protein of interest. Subsequent library preparation is typically performed prior to analyzing samples using sequencing technologies, which may increase time, cost, labor and potential sample loss.

The term "binding site" as used herein may refer to a region on a protein, DNA, or RNA to which other molecules specifically bind. For instance, the binding site a chromatin-associated factor of interest binds to may be referred to as chromatin binding site. Methods for determining the chromatin binding sites of a chromatin-associated factor of interest known in the art include ChIP, ChIC, CUT&RUN, and conventional CUT&Tag.

Within a nucleus, there is a spectrum of DNA accessibility states that range from hyper-accessible, which is often referred to as open chromatin, to more moderate states of accessibility known as permissive chromatin, and to more inaccessible or repressive states denoted as closed chromatin. Open and permissive states are often transcriptionally active chromatin and are often interchangeably used with the term 'euchromatin', whereas closed states are commonly referred to as 'heterochromatin' or 'inactive' chromatin.

The term "crosslinking" as used herein may refer to a method of chemically joining two or more molecules by a covalent bond. Crosslinking methods may allow for the stabilization of molecular structures and the capture of molecular interactions within a cell so that they can be preserved and studied. DNA-protein crosslinking can be caused by a variety of chemical and physical agents. Crosslinking agents or reagents appropriate for use with the disclosed methods include but are not limited to formaldehyde, paraformaldehyde, methylene blue with or without exposure to white light, sodium fluorescein, acridine orange, cisplatin, dimethylarsinic acid, potassium chromate, ultraviolet light, and lasers.

The term "strong crosslinking conditions" as used herein may refer to crosslinking conditions that may result in masking antibody epitopes, making chromatin less accessible for antibodies and/or the transposome that is linked to a specific binding agent. Strong crosslinking conditions may include the use of 1% formaldehyde. Incubation times for strong crosslinking conditions may be around 8 minutes for histone modifications and around 15 minutes for non-histone proteins.

The term "light crosslinking conditions" or "mild crosslinking conditions" as used herein may refer to crosslinking conditions that may lower the risk of masking antibody epitopes and thus making chromatin less accessible for antibodies and/or the transposome that is linked to a specific binding agent. Such mild crosslinking conditions may include the use of less than 0.5% formaldehyde or 0.4% formaldehyde or less or 0.3% formaldehyde or less or 0.2% formaldehyde or less, e.g. 0.1% formaldehyde or less.

The term "formaldehyde" ("FA") as used herein is a chemical having the formula $CH_2O$. It can be used as a chemical crosslinking reagent to crosslink DNA to protein or DNA to DNA within a chromatin complex to maintain chromatin structure.

The term "PBS" as used herein may refer to Phosphate-Buffered Saline-PBS is a buffer solution which is typically used in biological research to simulate physiological conditions.

The term "specific binding agent" as used herein may refer to an agent that binds substantially or preferentially only to a defined target such as a protein, enzyme, polysaccharide, oligonucleotide, DNA, RNA, recombinant vector or a small molecule. A nucleic acid-specific binding agent binds substantially only to the defined nucleic acid, such as DNA, or to a specific region within the nucleic acid. In some embodiments, a specific binding agent is a probe or primer that specifically binds to a target nucleic acid of interest. In some embodiments, a specific binding agent is a transcription factor that specifically binds to a target nucleic acid of interest, such as chromatin DNA. A protein-specific binding agent binds substantially only the defined protein, or to a specific region within the protein. For example, a "specific binding agent" includes antibodies and other agents that bind substantially to a specified polypeptide. Antibodies can be monoclonal or polyclonal antibodies that are specific for the polypeptide, as well as immunologically effective portions ("fragments") thereof. The determination that a particular agent binds substantially only to a specific polypeptide may readily be made by using or adapting routine procedures. One suitable *in vitro* assay makes use of the Western blotting procedure (as described in many standard texts). A specific binding agent may include all or parts of protein A (pA) or protein G (pG) or derivatives thereof which have affinity for antibodies. Protein A is a 42 kDa surface protein originally found in the cell wall of the bacterium *Staphylococcus aureus.* It is encoded by the spa gene and its regulation is controlled by DNA topology, cellular osmolarity, and a two-component system called ArIS-ArIR. It is commonly used in biochemical research because of its ability to bind immunoglobulins. Like Protein A but with differing binding specificities, Protein G is an immunoglobulin-binding protein expressed in group C and G *Streptococcal* bacteria. Protein G is a 65-kDa (G148 protein G) and a 58 kDa (C40 protein G) cell surface protein commonly used for purifying antibodies through its binding to the Fab and Fc region.

The term "antibody" refers to a polyclonal, monoclonal, recombinant, or synthetic immunoglobulin molecule that specifically binds a target antigen. The term includes intact immunoglobulin molecules, fragments or polymers of those immunoglobulin molecules, chimeric antibodies containing sequences from more than one species, class, or subclass of immunoglobulin, and human or humanized versions of immunoglobulin molecules or fragments thereof containing a least the idiotype of an immunoglobulin that specifically binds the target antigen. Accordingly, the agents binding to chromatin, in particular the antibody or chemical substance, used in the methods of the invention may specifically bind to histones, modified histones and/or other factors, in particular polypeptides such as enzymes, interacting with such histones and/or modified histones.

The term "adapter" as used herein may refer to a short double-stranded DNA oligonucleotide whose sequence is known.

The term "sequencing library" as used herein may refer to a nucleic acid representation, wherein each nucleic acid is identifiable by, e.g., the use of an individual sequence tag. Accordingly, obtaining a sequencing library typically requires a process capable of ensuring that specific adaptor sequences are added to the ends of the nucleic acid fragments to be analyzed. Most of the next generation sequencing applications require the preparation of a sequencing library comprising nucleic acids with specific adapters at 5' and 3' ends. For example, the Illumina sequencing workflow utilizes partially complementary adaptor oligonucleotides that are used for priming the PCR amplification and introducing the specific nucleotide sequences required for cluster generation by bridge PCR and facilitating the sequencing-by-synthesis reactions. Accordingly, the resulting sequencing library is suitable for use in standard sequencing applications, e.g. next generation sequencing.

As used herein, the term "normalization" may refer to a process that results in removal of at least some biases in the data such as unspecific chromatin binding sites of the chromatin-associated factor of interest, while specific binding sites persist.

As used herein, the term "replicate" may be used for samples of a cell where the biological material is the same. For example, the steps used to determine at least one chromatin binding site of a chromatin-associated factor of interest in a cell are repeated with at least one, at least two replicates, at least three replicates or at least four replicates. Alternatively or in addition, the method for determining at least one chromatin binding site of a chromatin-associated factor of interest in a cell as described herein is performed with a first replicate and a normalization control is generated with a second replicate. As used herein, the term "first replicate" may be used for a first sample of a cell and the term "second replicate" (or "third replicate" or "fourth replicate") may be used for a second sample (or third sample or fourth sample, respectively) of the same biological material. In other words, if the steps used to determine at least one chromatin binding site of a chromatin-associated factor of interest in a cell are repeated with two replicates (or three replicates or four replicates), the method is conducted twice (or three or four times, respectively).

As used herein, the term "false positive" may denote a result of the methods for determining at least one chromatin binding site of a chromatin-associated factor of interest in a cell as described herein which incorrectly indicates a chromatin binding site of the chromatin-associated factor of interest.

As used herein, the term "input normalization" may refer to a method in which the chromatin binding sites are determined in a cell which has not been contacted with a first antibody or second antibody in a method of the invention, and the determined chromatin binding sites are used for the purpose of normalization in a method of the invention. Alternatively, the term "input normalization" may refer to a method in which the chromatin binding sites are determined in DNA extracted from a cell which has not been contacted with a first antibody or second antibody in a method of the invention, and the determined chromatin binding sites are used for the purpose of normalization in a method of the invention. For example, an input sample can be subjected to method steps (c) to (f) or method steps (d) to (f) of a method as described herein in order to allow for a normalisation of the at least one chromatin binding site of the chromatin-associated factor of interest in the cell determined in step f).

In an exemplary embodiment, normalization may be carried out by performing in a first replicate method steps (a) to (f) of a method as described herein and the following modified method steps in a second replicate:

- in step (a), instead of permeabilizing of the cell, purifying DNA from the cell,
- omitting step (b) of the method as described herein, i.e. omitting the step of contacting the permeabilized cell with a first antibody that specially binds the chromatin-associated factor of interest and with a second antibody that specifically binds to the first antibody,
- performing step (c) of the method as described herein with the purified DNA, i.e. contacting the purified DNA with the transposome that is linked to a specific binding agent that specifically binds the first and/or second antibody wherein the transposome comprises a transposase and a first and second DNA molecule;
- performing step (d) of the method as described herein, i.e. activating the transposase;
- if a DNA segment is excised and tagged with the first and second DNA molecule by activating the transposase, determining the sequence of the excised and tagged DNA in the second replicate;

wherein the sequence of the excised and tagged DNA segment determined in the second replicate is used as a normalization control in the step of determining the at least one chromatin binding site of the chromatin-associated factor of interest in the cell determined in step (f) in the first replicate.

In another exemplary embodiment, normalization may be carried out by performing in a first replicate method steps (a) to (f) of a method as described herein and the following modified method steps in a second replicate:

- purifying DNA from the cell,
- contacting the purified DNA with the transposome that is linked to a specific binding agent that specifically binds the first and/or second antibody wherein the transposome comprises a transposase and a first and second DNA molecule;
- activating the transposase;
- if a DNA segment is excised and tagged with the first and second DNA molecule by activating the transposase, determining the sequence of the excised and tagged DNA in the second replicate;

wherein the sequence of the excised and tagged DNA segment determined in the second replicate is used as a normalization control in the step of determining the at least one chromatin binding site of the chromatin-associated factor of interest in the cell determined in step (f) in the first replicate.

As used herein, the term "input sample" may refer to sequence information which can be used as a normalization control in the step of determining the at least one chromatin binding site of the chromatin-associated factor of interest in the cell determined in step f) of the method as described herein.

An exemplary method for generating a normalization control suitable for use in the above described method for determining at least one chromatin binding site of the chromatin-associated factor of interest in the cell comprises the following steps:

- purifying DNA from the cell;
- contacting the purified DNA with a transposome that is linked to a specific binding agent suitable for binding a first and/or second antibody wherein the transposome comprises a transposase and a first and second DNA molecule;
- activating the transposase;
- if a DNA segment is excised and tagged with the first and second DNA molecule by activating the transposase, determining the sequence of the excised and tagged DNA.

As used herein, the term "IgG normalization" may refer to a method in which the chromatin binding sites are determined in a cell which has been subjected to a first antibody that does not target a chromatin-associated factor of interest but instead constitutes an IgG antibody that is not supposed to target any specific molecule in the cell in a method of the invention, and the determined chromatin binding sites are used for the purpose of normalization in a method of the invention.

Further exemplary embodiments are described in the following.

In some embodiments, the steps of contacting the permeabilized cell with the transposome and activating the transposase are performed in the presence of a crowding agent. In some embodiments, the crowding agent is selected from a group comprising sucrose, hexylene glycol and glycerol. In some embodiments, the crowding agent is sucrose. Without being bound to a particular theory the presence of a crowding agent in the steps of contacting a permeabilised cell with a transposome and activating the transposase as described herein may mimic the "crowding" of cytoplasm and nucleus with macromolecules. The use of a crowding agent may thus mimic a situation closer to the actual *in vivo* situation. In particular, a crowding agent may stabilize DNA-protein interactions.

In some embodiments, the steps of contacting the permeabilized cell with the transposome and activating the transposase are performed in the presence of a Good's buffer. In some embodiments, the Good's buffer is a buffer that can chelate $Mg^{2+}$. In some embodiments, the Good's buffer is a buffer other than HEPES. In some embodiments, the Good's buffer is selected from Tricine, MES, ADA, PIPEs, ACES, MPOSO, Cholamine chloride, MOPS, BES, TES, DIPSO, TASO, Acetamidoglycine, POPSO, Acetamidoglycine, POPSO, HEPPSO, HEPPS, Tris, Glycinamide, Glycylglycine, Bicine and TAPS. In some embodiments, the Good's buffer is Tricine. Without being bound to a particular theory, a Good's buffer as described herein may help avoid premature activation of the transposase and unspecific cutting in accessible chromatin. The presence of Good's buffer may be beneficial for the tagmentation process through a wider pH range.

In some embodiments, the steps of contacting the permeabilized cell with the transposome and activating the transposase are performed in the presence of a crowding agent and a Good's buffer.

In some embodiments, the steps of contacting the permeabilized cell with the transposome and activating the transposase are performed in the presence of sucrose and tricine.

In some embodiments, a cell comprises a plurality of cells. In some embodiments, a plurality of cells is a mixed population of cells. A mixed population of cells as used herein comprises cells from different cell types.

A cell used in a method of the invention may be obtained from a biological sample obtained directly from a subject. Non-limiting examples of biological samples are samples of: blood, saliva, lymph, cerebrospinal fluid, vitreous humor, aqueous humor, mucous, tissue, etc.

In some embodiments, a cell is a primary immune cell, such as but not limited to a T-cell. In some embodiments, a method of the invention is performed on invertebrate cells, including but not limited to Drosophila cells. In other embodiments, a method of the invention is performed on vertebrate cells. In some embodiments, a method of the invention is carried out on mammalian cells, including but not limited to cells from cell lines, primary immune cells (e.g., T-cells), stem cells, diseased cells, healthy cells, etc. In some embodiments, a method of the invention is performed on mixed human cells and cells of another organism, examples of which are non-human primate cells, mouse cells, etc. In some embodiments, a method of the invention is performed on human cells. Examples of mammalian cells that may be used in methods of the invention are cells obtained directly from a subject, cells obtained from a mammalian cell line, cultured mammalian cells, transgenic mammalian cells, etc.

Cells used in certain methods of the invention may be obtained from a living animal, e.g., a mammal, or may be obtained from a collection of isolated cells. An isolated cell may be a primary cell, such as those recently isolated from an animal (e.g., cells that have undergone none or only a few population doublings and/or passages following isolation), or may be cells of a cell line that is capable of prolonged proliferation in culture (e.g., for longer than 3 months) or indefinite proliferation in culture (immortalized cells).

In some embodiments, a cell is a somatic cell. Somatic cells may be obtained from an individual, e.g., a human, and cultured according to standard cell culture protocols known to those of ordinary skill in the art.

Cells may be obtained from surgical specimens, tissue or cell biopsies, etc, Cells may be obtained from any organ or tissue of interest, including but not limited to: skin, lung, cartilage, brain, CNS, PNS, breast, blood, blood vessel (e.g., artery or vein), fat, pancreas, liver, muscle, gastrointestinal tract, heart, bladder, kidney, urethra, and prostate gland.

In some embodiments, a cell used in conjunction with the invention is a healthy normal cell, which is not known to have a disease, disorder, or abnormal condition. In some embodiments, a cell used in conjunction with methods of the invention is an abnormal cell, for example, a cell obtained from a subject diagnosed as having a disorder, disease, or condition, including, but not limited to a degenerative cell, a neurological disease-bearing cell, a cell model of a disease or condition, an injured cell, etc. In some embodiments, a cell is an abnormal cell obtained from cell culture, a cell line known to include a disorder, disease, or condition. In some embodiments, a cell is a control cell. In some aspects of the invention a cell can be a model cell for a disease or condition.

Examples of a cell that may be used in an embodiment of a method of the invention are one or more of: eukaryotic cells, vertebrate cells, which in some embodiments of the invention may be mammalian cells. A non-limiting example of cells that may be used in methods of the invention are: vertebrate cells, invertebrate cells, and non-human primate cells. Additional, examples of cells that may be used in an embodiment of a method of the invention are one or more of: rodent cells, dog cells, cat cells, avian cells, fish cells, cells obtained from a wild animal, cells obtained from a domesticated animal, and other suitable cell of interest. In some embodiments, a cell is a human cell. In some embodiments, a cell is a stein cell, an embryonic stem cell, or embryonic stem cell-like cell. In some embodiments, a cell is a naturally occurring cell and, in some embodiments, a cell is an engineered cell.

Cells useful in embodiments of methods of the invention may be maintained in cell culture following their isolation. Cells may be genetically modified or not genetically modified in various embodiments. Cells may be obtained from normal or diseased tissue.

In some embodiments, cells are obtained from a donor, and their state or type is modified *ex vivo* using a method of the invention. In some embodiments, a cell may be a free cell in culture, a free cell obtained from a subject, a cell obtained in a solid biopsy from a subject, organ, or solid culture, etc.

In some embodiments, the number of cells is ≤ 500,000. In some embodiments, the number of cells is ≤ 100,000. In some embodiments, the number of cells is ≤ 50,000. In some embodiments, the number of cells is ≤ 10,000. In some embodiments, the number of cells is ≤ 5,000. In some embodiments, the number of cells is ≤ 1,000.

In some embodiments, the cells and/or nuclei may be subjected to crosslinking. In other embodiments, the cells and/or nuclei are not subjected to crosslinking. In some embodiments, the cells and/or nuclei are subjected to crosslinking directly after harvest. In some embodiments, crosslinking is followed by cryopreservation. In some embodiments, crosslinking is performed prior to permeabilization. In some embodiments, mild crosslinking is performed prior to the permeabilization step. In some embodiments, crosslinking is followed by one or more washing steps and/or a quenching step prior to permeabilization. In some embodiments, crosslinking using mild cross-linking conditions is performed directly after harvest, optionally followed by cryopreservation and followed by permeabilization. In some embodiments, mild crosslinking conditions are 0.1% formaldehyde for ≤ 15 min, ≤ 10 min or ≤ 5 min. In some embodiments, the quenching agent is glycine. In some embodiments, quenching is performed for ≤ 10 min or ≤ 5 min. In some embodiments, mild crosslinking conditions are 0.1% formaldehyde for 5 min and are followed by quenching with glycine for additional 5 minutes. In some embodiments, cells and/or nuclei subjected to crosslinking and quenching may be subjected to cryopreservation prior to further processing.

In some embodiments, the chromatin-associated factor of interest is a protein. In some embodiments, the chromatin-associated factor of interest is a histone protein. In some embodiments, the chromatin-associated factor of interest is a non-histone protein. In some embodiments, the chromatin-associated factor of interest is a transcription factor. In some embodiments, the chromatin-associated factor of interest is a histone reader protein. In some embodiments, the chromatin-associated factor of interest is a writer protein. In some embodiments, the chromatin-associated factor of interest is an eraser protein.

In a method of the invention, the first antibody specially binds the chromatin-associated factor of interest. In some embodiments, the first antibody binds to proteins. In some embodiments, the first antibody binds to histone proteins. In some embodiments, the first antibody may specifically bind to histones, modified histones and/or other factors, in particular polypeptides such as enzymes, interacting with such histones and/or modified histones.

In some embodiments, the first antibody binds to modified histones. In some embodiments, the first antibody binds to H3K4me1/2/3, H2BK5me1, H3K27me1/2/3, H3K9me1/2/3, H4K20me1 , H3K79me1 , H3K36me3, H2AK5ac, H2AK9ac, H2BK5ac, H2BK12ac, H2BK20ac, H2BK120ac, H3K4ac, H3K9ac, H3K14ac, H3K18ac, H3K23ac, H3K27ac, H3K36ac, H4K5ac, H4K8ac, H4K12ac, H4K16ac, H4K91 ac, H2Aub or H2Bub.

In some embodiments, the first antibody binds to non-histone proteins. In some embodiments, the first antibody binds to transcription factors. In some embodiments, the first antibody binds to AAF, abl, ADA2, ADA-NF1 , AF-1 , AFP1 , AhR, AIIN3, ALL-1 , alpha-CBF, alpha-CP 1 , alpha-CP2a, alpha-CP2b, alphaHo, alphaH2-alphaH3, Alx-4, aMEF-2, AML1 , AMLIa, AMLIb, AMLIc, AMLIDeltaN, AML2, AML3, AML3a, AML3b, AMY- 1 L, A-Myb, ANF, AP-1 , AP-2alphaA, AP-2alphaB, AP-2beta, AP-2gamma, AP-3 (1), AP-3 (2), AP-4, AP-5, APC, AR, AREB6, Arnt, Arnt (774 M form), ARP-1 , ATBF1 -A, ATBF1 -B, ATF, ATF-1 , ATF-2, ATF-3, ATF-3deltaZIP, ATF-a, ATF- adelta, ATPF1 , Barhll, Barhl2, Barxl, Barx2, Bcl-3, BCL-6, BD73, beta-catenin, Binl, B-Myb, BP1, BP2, brahma, BRCA1, Brn-3a, Brn-3b, Brn-4, BTEB, BTEB2, B-TFIID, C/EBPalpha, C/EBPbeta, C/EBPdelta, CACCbinding factor, Cart-1, CBF (4), CBF (5), CBP, CCAAT-binding factor, CCMT-binding factor, CCF, CCG1, CCK-la, CCK-lb, CD28RC, cdk2, cdk9, Cdx-1, CDX2, Cdx-4, CFF, ChxlO, CLIMI, CLIM2, CNBP, CoS, COUP, CPI, CPIA, CPIC, CP2, CPBP, CPE binding protein, CREB, CREB-2, CRE-BPI, CRE-BPa, CREMalpha, CRF, Crx, CSBP-1, CTCF, CTF, CTF-1, CTF-2, CTF-3, CTF-5, CTF-7, CUP, CUTL1, Cx, cyclin A, cyclin TI, cyclin T2, cyclin T2a, cyclin T2b, DAP, DAX1, DB1, DBF4, DBP, DbpA, DbpAv, DbpB, DDB, DDB-1, DDB-2, DEF, deltaCREB, deltaMax, DF-1, DF-2, DF-3, Dlx-1, Dlx-2, Dlx-3, Dlx4 (long isoform), Dlx-4 (short isoform, Dlx-5, Dlx-6, DP-1, DP-2, DSIF, DSIF-pl4, DSIF-pl60, DTF, DUX1, DUX2, DUX3, DUX4, E, EI 2, E2F, E2F+E4, E2F+pl07, E2F-1, E2F-2, E2F-3, E2F-4, E2F-5, E2F-6, E47, E4BP4, E4F, E4F1, E4TF2, EAR2, EBP-80, EC2, EF1, EF-C, EGR1, EGR2, EGR3, EllaE-A, EIIaE-B, EllaE-Calpha, EllaE-Cbeta, EivF, Elf-1, Elk-1, Emx-1, Emx-2, Emx-2, En-1, En-2, ENH-bind. prot, ENKTF-1, EPASI, epsilonFI, ER, Erg-1, Erg-2, ERR1, ERR2, ETF, Ets-1, Ets-1 deltaVil, Ets-2, Evx-1, F2F, factor 2, Factor name, FBP, f-EBP, FKBP59, FKHL18, FKHRL1P2, Fli-1, Fos, FOXB1, FOXC1, FOXC2, FOXD1, FOXD2, FOXD3, FOXD4, FOXE1, FOXE3, FOXF1, FOXF2, FOXGla, FOXGlb, FOXGlc, FOXH1, FOXI1, FOXJla, FOXJlb, FOXJ2 (long isoform), FOXJ2 (short isoform), FOXJ3, FOXKla, FOXKlb, FOXKlc, FOXL1, FOXMla, FOXMlb, FOXMlc, FOXN1, FOXN2, FOXN3, FOXOIa, FOXOIb, FOX02, FOX03a, FOX03b, FOX04, FOXP1, FOXP3, Fra-1, Fra-2, FTF, FTS, G factor, G6 factor, GABP, GABP-alpha, GABP- betal, GABP-beta2, GADD 153, GAF, gammaCMT, gammaCACI, gammaCAC2, GATA-1, GATA-2, GATA-3, GATA-4, GATA-5, GATA-6, Gbx-1, Gbx-2, GCF, GCMa, GCNS, GF1, GLI, GLI3, GR alpha, GR beta, GRF-1, Gsc, Gscl, GT-IC, GT-IIA, GT-IIBalpha, GT-IIBbeta, H1TF1, H1TF2, H2RIIBP, H4TF-1, H4TF-2, HAND1, HAND2, HB9, HDAC1, HDAC2, HDAC3, hDaxx, heat-induced factor, HEB, HEBI-p67, HEBI-p94, HEF-1 B, HEF-1T, HEF-4C, HEN1, HEN2, Hesxl, Hex, HIF-1, HIF-lalpha, HIF-lbeta, HiNF-A, HiNF-B, HINF-C, HINF-D, HiNF- D3, HiNF-E, HiNF-P, HIP1, HIV-EP2, Hlf, HLTF, HLTF (MetI23), HLX, HMBP, HMG I, HMG I(Y), HMG Y, HMGI-C, HNF-IA, HNF- IB, HNF-IC, HNF-3, HNF- 3alpha, HNF-3beta, HNF-3gamma, HNF4, HNF-4alpha, HNF4alphal, HNF- 4alpha2, HNF-4alpha3, HNF-4alpha4, HNF4gamma, HNF-6alpha, hnRNP K, HOX11 , HOXAI, HOXAIO, HOXAIO PL2, HOXAI I, HOXA13, HOXA2, HOXA3, HOXA4, HOXA5, HOXA6, HOXA7, HOXA9A, HOXA9B, HOXB-1 , HOXB13, HOXB2, HOXB3, HOXB4, HOXBS, HOXB6, HOXA5, HOXB7, HOXB8, HOXB9, HOXC10, HOXC1 1, HOXC12, HOXC13, HOXC4, HOXC5, HOXC6, HOXC8, HOXC9, HOXD10, HOXD1 1 , HOXD12, HOXD13, HOXD3, HOXD4, HOXD8, HOXD9, Hp55, Hp65, HPX42B, HrpF, HSF, HSF1 (long), HSF1 (short), HSF2, hsp56, Hsp90, IBP-1 , ICER-II, ICERligamma, ICSBP, Idl, Idl H', Id2, Id3, Id3/Heir-1 , IF1 , IgPE-1 , IgPE-2, IgPE-3, IkappaB, IkappaB-alpha, IkappaB-beta, IkappaBR, II-I RF, IL-6 RE-BP, 1 1 -6 RF, INSAF, IPF1 , IRF-1 , IRF- 2, B, IRX2a, Irx-3, Irx-4, ISGF-1 , ISGF-3, ISGF3alpha, ISGF-3gamma, 1 st- 1 , ITF, ITF-1 , ITF-2, JRF, Jun, JunB, JunD, kappay factor, KBP-1 , KER1 , KER-1 , Koxl, KRF-1 , Ku autoantigen, KUP, LBP-1 , LBP-la, LBXI, LCR-FI, LEF-1 , LEF- IB, LF-A1 , LHX1, LHX2, LHX3a, LHX3b, LHXS, LHX6.1 a, LHX6.1 b, LIT-1 , Lmol, Lmo2, LMX1A, LMX1 B, L-Myl (long form), L-Myl (short form), L-My2, LSF, LXRalpha, LyF-1 , Lyl-I, M factor, Madl, MASH-1 , Maxi, Max2, MAZ, MAZ1 , MB67, MBF1 , MBF2, MBF3, MBP-1 (1), MBP-1 (2), MBP-2, MDBP, MEF-2, MEF-2B, MEF-2C (433 AA form), MEF-2C (465 AA form), MEF-2C (473 M form), MEF-2C/delta32 (441 AA form), MEF-2D00, MEF-2D0B, MEF-2DA0, MEF-2DAO, MEF-2DAB, MEF-2DA'B, Meis-1 , Meis-2a, Meis-2b, Meis-2c, Meis-2d, Meis-2e, Meis3, Meoxl, Meoxla, Meox2, MHox (K-2), Mi, MIF-1 , Miz-1 , MM-1 , MOP3, MR, Msx-1 , Msx-2, MTB-Zf, MTF-1 , mtTFI, Mxil, Myb, Myc, Myc 1 , Myf-3, Myf-4, Myf-5, Myf-6, MyoD, MZF-1 , NCI, NC2, NCX, NELF, NER1 , Net, NF III-a, NF NF NF-1 , NF-1A, NF-1 B, NF-1X, NF-4FA, NF-4FB, NF- 4FC, NF-A, NF-AB, NFAT-1 , NF-AT3, NF-Atc, NF-Atp, NF-Atx, Nf etaA, NF- CLEOa, NF-CLEOb, NFdeltaE3A, NFdeltaE3B, NFdeltaE3C, NFdeltaE4A, NFdeltaE4B, NFdeltaE4C, Nfe, NF-E, NF-E2, NF-E2 p45, NF-E3, NFE-6, NF- Gma, NF-GMb, NF-IL-2A, NF-IL-2B, NF-jun, NF-kappaB, NF-kappaB(-like), NF- kappaBI, NF-kappaB 1 , precursor, NF-kappaB2, NF-kappaB2 (p49), NF-kappaB2 precursor, NF-kappaEI, NF-kappaE2, NF-kappaE3, NF-MHCIIA, NF-MHCIIB, NF-muEl, NF-muE2, NF-muE3, NF-S, NF-X, NF-X1 , NF-X2, NF-X3, NF-Xc, NF- YA, NF-Zc, NF-Zz, NHP-1 , NHP-2, NHP3, NHP4, NKX2-5, NKX2B, NKX2C, NKX2G, NKX3A, NKX3A vl, NKX3A v2, NKX3A v3, NKX3A v4, NKX3B, NKX6A, Nmi, N-Myc, N-Oct-2alpha, N-Oct-2beta, N-Oct-3, N-Oct-4, N-Oct-5a, N-Oct-5b, NP-TCII, NR2E3, NR4A2, Nrfl, Nrf-1 , Nrf2, NRF-2betal, NRF- 2gammal, NRL, NRSF form 1 , NRSF form 2, NTF, 02, OCA-B, Oct-1 , Oct-2, Oct- 2.1 , Oct-2B, Oct-2C, Oct-4A, Oct4B, Oct-5, Oct-6, Octa-factor, octamer-binding factor, oct-B2, oct-B3, Otxl, Otx2, OZF, pl07, pl30, p28 modulator, p300, p38erg, p45, p49erg,-p53, p55, p55erg, p65delta, p67, Pax-1 , Pax-2, Pax-3, Pax-3A, Pax-3B, Pax-4, Pax-5, Pax-6, Pax-6/Pd-5a, Pax-7, Pax-8, Pax-8a, Pax-8b, Pax-8c, Pax-8d, Pax-8e, Pax-8f, Pax-9, Pbx-la, Pbx-lb, Pbx-2, Pbx-3a, Pbx-3b, PC2, PC4, PC5, PEA3, PEBP2alpha, PEBP2beta, Pit-1 , PITX1 , PITX2, PITX3, PKNOX1 , PLZF, PO-B, Pontin52, PPARalpha, PPARbeta, PPARgammal, PPARgamma2, PPUR, PR, PR A, pRb, PRD1 -BF1 , PRDI-BFc, Prop-1 , PSE1 , P-TEFb, PTF, PTFalpha, PTFbeta, PTFdelta, PTFgamma, Pu box binding factor, Pu box binding factor (B JA- B), PU.1 , PuF, Pur factor, RI , R2, RAR-alphal , RAR-beta, RAR-beta2, RAR- gamma, RAR-gammal, RBP60, RBP-Jkappa, Rel, RelA, RelB, RFX, RFXI, RFX2, RFX3, RFXS, RF-Y, RORalphal, RORalpha2, RORalpha3, RORbeta, RORgamma, Rox, RPF1 , RPGalpha, RREB-1 , RSRFC4, RSRFC9, RVF, RXR-alpha, RXR-beta, SAP-la, SAPlb, SF-1 , SHOX2a, SHOX2b, SHOXa, SHOXb, SHP, SIll-pl IO, SIN- pl5, SIII-pI8, SIM', Six-1 , Six-2, Six-3, Six-4, Six-5, Six-6, SMAD-1 , SMAD-2, SMAD-3, SMAD-4, SMAD-5, SOX-1 1 , SOX- 12, Sox-4, Sox-5, SOX-9, Spl, Sp2, Sp3, Sp4, Sph factor, Spi-B, SPIN, SRCAP, SREBP-la, SREBP-lb, SREBP-Ic, SREBP-2, SRE-ZBP, SRF, SRY, SRPI, Staf-50, STATIalpha, STATIbeta, STAT2, STAT3, STAT4, STAT6, T3R, T3R-alphal, T3R-alpha2, T3R-beta, TAF(I)1 10, TAF(I)48, TAF(I)63, TAF(II)100, TAF(II)125, TAF(II)135, TAF(II)170, TAF(II)18, TAF(II)20, TAF(II)250, TAF(II)250Delta, TAF(II)28, TAF(II)30, TAF(II)31 , TAF(II)55, TAF(II)70-alpha, TAF(II)70-beta, TAF(II)70-gamma, TAF- I, TAF-II, TAF-L, Tal-1, Tal-lbeta, Tal-2, TAR factor, TBP, TBX1A, TBX1 B, TBX2, TBX4, TBXS (long isoform), TBXS (short isoform), TCF, TCF-1, TCF-1A, TCF-1 B, TCF-1 C, TCF-1 D, TCF-1 E, TCF-1 F, TCF-1 G, TCF-2alpha, TCF-3, TCF- 4, TCF-4(K), TCF-4B, TCF-4E, TCFbetal, TEF-1, TEF-2, tel, TFE3, TFEB, TFIIA, TFIIA-alpha/beta precursor, TFIIA-alpha/beta precursor, TFIIA-gamma, TFIIB, TFIID, TFIIE, TFIIE-alpha, TFIIE-beta, TFIIF, TFIIF-alpha, TFIIF-beta, TFIIH, TFIIH*, TFIIH-CAK, TFIIH-cyclin H, TFIIH-ERCC2/CAK, TFIIH-MAT1, TFIIH-M015, TFIIH-p34, TFIIH-p44, TFIIH-p62, TFIIH-p80, TFIIH-p90, TFII-I, Tf-LFI, Tf-LF2, TGIF, TGIF2, TGT3, THRAI, TIF2, TLE1, TLX3, TMF, TR2, TR2-11, TR2-9, TR3, TR4, TRAP, TREB-1, TREB-2, TREB-3, TREFI, TREF2, TRF (2), TTF-1, TXRE BP, TxREF, UBF, UBP-1, UEF-1, UEF-2, UEF-3, UEF-4, USF1, USF2, USF2b, Vav, Vax-2, VDR, vHNF-IA, vHNF-IB, vHNF-IC, VITF, WSTF, WT1, WT1 I, WT1 I-KTS, WT1 I-del2, WT1 -KTS, WTI-del2, X2BP, XBP-1, XW-V, XX, YAF2, YB-1, YEBP, YY1, ZEB, ZF1, ZF2, ZFX, ZHX1, ZIC2, ZID or ZNF 174. In some embodiments, the first antibody binds to a transcription factor known to be associated with diseases, e.g. cancer. In this regard, the methods of the invention may be used to study the interaction between DNA and transcription factors in a diseased cell and/or cells derived from diseased tissue.

In some embodiments, the cells are immobilized on a solid surface, for example a bead or the wall of a microtiter plate. Methods of coupling cells to such solid surfaces are known in the art, for example in the context of high throughput techniques. In some embodiments, the bead is a magnetic bead. In some embodiments, the bead is a magnetic Concanavalin A (ConA) bead. In some embodiments, prior to the permeabilization step, the cells are bound to beads, optionally magnetic beads.

In some embodiments, one or more method steps are performed in single 1.5 ml tubes. In some embodiments, one or more method steps are performed in tube strips having a volume of 0.5 ml or less such as 0.2 ml. In some embodiments, the use of 0.2 ml tube strips instead of single 1.5 ml tubes allows the use of a multichannel pipette for wash and other resuspension steps, and/or the addition of reagents, provided that the volume is high enough for the use of a multichannel pipette. Without being bound to a particular theory, the use of 0.2 ml tube strips and/ or the lowering of the total volume may decrease the exposure time of the samples to the buffer, reduce the detaching rate and increase the recovery.

In some embodiments, one or more method steps are performed using a total buffer volume of $\leq 1000\ \mu l$. In some embodiments, one or more method steps are preformed using a total buffer volume of $\leq 800\ \mu l$. In some embodiments, one or more method steps are preformed using a total buffer volume of $\leq 500\ \mu l$. In some embodiments, one or more method steps are preformed using a total buffer volume of $\leq 200\ \mu l$. In some embodiments, one or more method steps are preformed using a total buffer volume of $\leq 100\ \mu l$. In some embodiments, one or more method steps are preformed using a total buffer volume of $\leq 50\ \mu l$.

In a method of the invention, a transposome is linked to a specific binding agent that specifically binds the first and/or second antibody wherein the transposome comprises a transposase and a first and second DNA molecule. In some embodiments, the transposase is Tn5. In some embodiments, a modified transposase is used, which has a higher activity than the naturally occurring Tn5 transposase. In some embodiments, the transposase is loaded with a first and second DNA molecule, e.g. oligonucleotides, which are inserted into the target nucleic acid, in particular the target DNA. The transposome comprises a transposase and a first and second DNA molecule. The first DNA molecule may comprise a first transposase recognition site and the second DNA molecule may comprise a second transposase recognition site. In some embodiments, a hyperactive Tn5 transposase and a Tn5- type transposase recognition or MuA transposase and a Mu transposase recognition site comprising RI and R2 end sequences are used. More examples of transposition systems that can be used in the methods of the present invention include Staphylococcus aureus Tn552 (Colegio et al, J. Bacteriol, 183: 2384-8, 2001; Kirby C et al, Mol. Microbiol, 43: 173-86, 2002), Tyl (Devine & Boeke, Nucleic Acids Res., 22: 3765-72, 1994 and International Publication WO 95/23875), Transposon Tn7 (Craig, N L, Science. 271: 1512, 1996; Craig, N L, Review in: Curr Top Microbiol Immunol, 204:27-48, 1996), Tn/O and IS 10 (Kleckner N, et al, Curr Top Microbiol Immunol, 204:49-82, 1996), Mariner transposase (Lampe D J, et al, EMBO J., 15: 5470-9, 1996), Tel (Plasterk R H, Curr. Topics Microbiol. Immunol, 204: 125-43, 1996), P Element (Gloor, G B, Methods Mol. Biol, 260: 97- 1 14, 2004), Tn3 (Ichikawa & Ohtsubo, J Biol. Chem. 265: 18829-32, 1990), bacterial insertion sequences (Ohtsubo & Sekine, Curr. Top. Microbiol. Immunol. 204: 1 -26, 1996), retroviruses (Brown, et al, Proc Natl Acad Sci USA, 86:2525-9, 1989), and retrotransposon of yeast (Boeke & Corces, Annu Rev Microbiol. 43 :403-34, 1989). More examples include IS5, TnIO, Tn903, IS91 1, and engineered versions of transposase family enzymes (Zhang et al, (2009) PLoS Genet. 5:el000689. Epub 2009 Oct 16; Wilson C. et al (2007) J. Microbiol. Methods 71:332-5).

In some embodiments, the amount of transposase that is linked to a specific binding agent is $\leq 1$: 50 of the reaction volume. In some embodiments, the amount of transposase that is linked to a specific binding agent is $\leq 1$:100 of the reaction volume. In some embodiments, the amount of transposase that is linked to a specific binding agent is $\leq 1$:150 of the reaction volume. In some embodiments, the amount of transposase that is linked to a specific binding agent is $\leq 1$:200 of the reaction volume. In some embodiments, the amount of transposase that is linked to a specific binding agent is $\leq 1$:250 of the reaction volume. Without being bound to any theory, the optimal amount of transposase that is linked to a specific binding agent may vary batch-by-batch. Methods to determine the optimal amount of transposase that is linked to a specific binding agent are well known in the art and the optimal amount of transposase can be determined by routine optimization. These include, for example, subjecting different dilutions of the amount of transposase that is linked to a specific binding agent to conventional CUT&Tag or a method disclosed herein, followed by the evaluation of the library concentration and the data obtained after sequencing, e.g. by visual inspection and bioinformatic analysis.

Cell membranes can be permeabilized or disrupted in any way known in the art. The methods of permeabilizing or disrupting the cell membrane do not disrupt the structure of the genomic DNA of the cell such that nucleosomal or chromatin structure is destroyed. The cell membrane may be contacted with a reagent that permeabilizes the cell membrane. Lysolipids are an exemplary class of reagents that permeabilize cell membranes. Exemplary lysolipids include lysophosphatidylcholine (also known in the art as lysolecithin) or monopalmitoylphosphatidylcholine. A variety of lysolipids are also described in, e.g., WO 2003/052095. The precise concentration of the agent will depend on the agent used as well as the cell to be permeabilized. As an example, 0.25, 0.5%, 0.75 or 1 % (or a concentration between 0.25% and 1%) of lysolecithin (w/v) may be used. Alternatively, electroporation or biolistic methods can be used to permeabilize a cell membrane such that a DNA cleaving agent is introduced into the cell and can thus contact the genomic DNA. A wide variety of electroporation methods are well known and can be adapted for delivery of DNA modifying agents as described herein. Exemplary electroporation methods include those described in WO 2000/062855. Biolistic methods include those described in US Patent 5,179,022. Non-ionic detergents are an exemplary class of reagents that disrupt cell membranes. Exemplary non-ionic detergents include NP40, Tween 20 and Triton X-100. The precise concentration of the reagent will depend on the non-ionic detergent used and/or the cell to be permeabilized. Commonly used permeabilization reagents include digitonin or related saponin compounds. In some embodiments, cells are permeabilized using digitonin at a concentration of 0.1%. Alternatively, Triton X-100 may be used to permeabilize cells. In some embodiments, cells are permeabilized using Triton X-100 at a concentration of 0.1% to 0.4%. In some embodiments, cells are permeabilized using 0.1% Triton X-100.

DNA can be purified from the cell by various techniques known in the art (see e.g. Gupta N. DNA Extraction and Polymerase Chain Reaction. J Cytol. 2019 Apr-Jun;36(2):116-117; Preetha J Shetty, The Evolution of DNA Extraction Methods. 2020 - 8(1). AJBSR.MS.ID.001234. DOI:10.34297/AJBSR.2020.08.001234.). Conventional purification methods may include the following steps: leukocyte isolation or red cell lysis, nuclear lysis, deproteinization, RNAse-A treatment, and DNA precipitation. Several commercial products are available for rapid purification of genomic DNA from blood spots, whole blood, and other sources. Exemplary methods for DNA purification from cells are also described below in the context of extracting excised and tagged DNA segments.

Following the step of activating the transposase, thereby excising a DNA segment comprising at least one chromatin binding site of a chromatin-associated factor of interest and tagging the DNA with the first and second DNA molecule, the tagmentation reaction may be stopped by various techniques known in the art. Tagmentation may be quenched using a buffer containing EDTA, and DNA fragments may be released into solution by heated digestion in a SDS buffer. SDS may subsequently neutralized using a nonionic detergent (i.e. Triton-X 100). Stopping the tagmentation reaction may involve adding EDTA, SDS and/or proteinase K and may be following protein digestion at higher temperatures, e.g. 55°C. In some embodiments, tagmentation is stopped by adding 0.5 M EDTA, 10% SDS and proteinase K followed by protein digestion at 55°C.

Prior to the step of determining the sequence of the excised and tagged DNA segment, the excised and tagged DNA segment may be extracted. This may be achieved by various techniques known in the art. For example, the excised and tagged DNA can be purified using column purification, phenol-chloroform extraction followed by ethanol precipitation, Solid Phase Reversible Immobilisation and Chelex® 100 and other techniques known in the art. Column purification relies on binding of nucleic acids, in particular DNA, (adsorption) to the solid phase (silica or other) depending on the pH and the salt content of the used buffer. After centrifugation of the sample, denaturated proteins remain in the organic phase while the aqueous phase containing nucleic acid, in particular DNA, is mixed with chloroform removing phenol residues from solution. To isolate DNA from the aqueous phase, phenol-chloroform extraction is followed by ethanol or isopropanol precipitation. Since DNA is insoluble in these alcohols, it will aggregate, giving a pellet upon centrifugation. Precipitation of DNA is improved by increasing ionic strength, usually by adding sodium acetate. Chelex® 100 is a chelating material distributed by Bio-Rad, which is used to purify other compounds via ion exchange. It can also be used to purify DNA. SPRI (Solid Phase Reversible Immobilisation) beads are paramagnetic (magnetic only in a magnetic field). Each bead is made of polystyrene surrounded by a layer of magnetite, which is coated with carboxyl molecules. It is these that reversibly bind DNA in the presence of polyethylene glycol (PEG) and salt (commonly 20% PEG, 2.5M NaCl). PEG causes the negatively-charged DNA to bind with the carboxyl groups on the bead surface. As the immobilization is dependent on the concentration of PEG and salt in the reaction, the volumetric ratio of beads to DNA is critical. DNA purification is often supported by removal of RNA and protein by the addition of RNase and Proteinase to the solution.

In some embodiments of the invention, extraction of the excised and tagged DNA segment may be performed using phenol-chloroform extraction with subsequent ethanol precipitation. In some embodiments, extraction of the excised and tagged DNA segment may be performed using the MicroChiP DiaPure columns (#C03040001, Diagenode).

Moreover, prior to the step of determining the sequence of the excised and tagged DNA segment, and optionally following extraction of the excised and tagged DNA segment, a sequencing library may be obtained. A sequencing library may comprise adaptor sequences. The adaptor sequences may vary depending on the sequencing method used subsequent to preparing the sequencing library. For example, where Illumina sequencing is used, i5 and i7 ends may be attached to the nucleic acid fragments. In some embodiments, the first and second DNA molecule comprise the adaptor sequences and are added by the transposase to the one or more chromatin-binding sites. This may eliminate a time-consuming step in the workflow and lower the amounts of starting material required. In other embodiments, methods of the invention for preparing a sequencing library may further comprise a step for integrating said adaptor sequences, which may be part of the amplification step.

In some embodiments, obtaining a sequencing library comprises an amplification step. Amplification of the sequencing libraries may be achieved by various techniques known in the art. The best-known technique for nucleic acid, in particular DNA, amplification is polymerase chain reaction (PCR), in which a sample is contacted with a pair of oligonucleotide primers under conditions that allow for the hybridization of the primers to a nucleic acid template in the sample. The primers are extended under suitable conditions, dissociated from the template, re-annealed, extended, and dissociated to amplify the number of copies of the nucleic acid. This cycle can be repeated. In some embodiments, the amplification step comprises insertion of next-generation sequencing indices and/or adaptors. The primers may comprise sequences hybridisable to the sequence comprised in the oligonucleotides comprised in the transposomes. In addition, primers may comprise sequences necessary for sequencing. In some embodiments, specific primers are used that are compatible with the subsequently used sequencing method. In this regard, Illumina sequencing, as one method of sequencing, is compatible with primers introducing flowcell ends, which can hybridize to the flowcell needed in cluster amplification. In this regard, primers may introduce i5 and i7 ends for Illumina sequencing. Furthermore, primers may introduce barcodes for multiplexing. In particular, barcodes comprised in the primer sequences may be used as unique molecular identifiers to discriminate between PCR duplicates and/or as defined barcodes to combine multiple experiments in one sequencing run.

When the methods of the invention comprise an amplification step, it is important to achieve a sufficient amount of eluted library without overamplifying the libraries. In some embodiments, sequencing libraries are amplified for at least 18, at least 17, at least 16, at least 15, least 14, at least 13, at least 12, at least 11, at least 10, at least 9 or at least 8 cycles. The cycle number may depend on the starting cell number and context. Without being bound by any particular theory, a cycle number greater than 18 may reduce the information that can be obtained from sequencing. If the library concentration is not considered sufficient for the sequencing step after amplification with 18 or more cycles, consideration may be given to repeating the experiment, e.g. with a higher cell number or other adjustments to the protocol. In some embodiments, amplification is directly followed by a purification step, e.g. using AMPure beads directly after the amplification step.

The present method may integrate adapters into DNA in the vicinity of the transposome that is linked to a specific binding agent. The exact sites of integration may be affected by the accessibility of surrounding DNA. For this reason, fragments that share exact starting and ending positions can indeed be common, and such 'duplicates' may not be due to duplication during PCR. Hence, in one embodiment, fragments that share exact starting and ending positions are used for further analysis according to the present method. Alternatively, for example in experiments with very small amounts of material or where PCR duplication is suspected, fragments that share exact starting and ending positions are disregarded in the analysis according to the present method. For example, Picard command-line tools may be used to check the duplication rate.

The product of amplification may be characterized by techniques such as electrophoresis, restriction endonuclease cleavage patterns, oligonucleotide hybridization or ligation, and/or nucleic acid sequencing. In some embodiments, the purification may be followed by a determination of the size distribution and/or concentration of libraries by methods known in the art, e.g. capillary electrophoresis using a TapeStation (Agilent Technolgies) or Fragment Analyzer (Agilent Technologies) or equivalent.

In some embodiments, following an optional determination of the concentration of the sequencing libraries, libraries for which the concentration is considered too low may be re-amplified. Without being bound by any particular theory, assessing library concentration only after a purification step in which the PCR polymerase and unique dual-indexing primers (UDI) are removed and samples are left in an elution buffer may not be optimal for potential re-amplification PCR reactions. In addition, samples that have been re-amplified under these conditions may be less comparable with other samples. While re-amplification is technically possible if the required reagents containing the polymerase are added again, there is a risk of over-amplification. While the concentration achieved during re-amplification may be technically sufficient for the sequencing step, over-amplification can result in certain DNA fragments, e.g. short libraries, being preferentially amplified, distorting the actual representation of the DNA pool and leading to low library complexity and a high duplication rate. This can affect further analysis and interpretation of the results, especially if it is difficult to fully quantify the PCR duplicates without Unique Molecule Identifiers (UMIs). Therefore, in some embodiments, amplification may be first performed using a lower number of cycles, for instance 9 cycles for histone modifications, e.g. H3K4me3, and 12 cycles for non-histone proteins such as PRC2 proteins. Following initial amplification, the concentration of the obtained libraries may be quantified, e.g. using KAPA Library Quantification Kits (Roche) according to the manufacturer's instructions. In this regard, between 0.5 and 1 $\mu$l per initially amplified library may be removed and quantified. For this purpose, DNA standards of a known concentration may be used to create a calibration curve to calculate the concentration of the sequencing libraries. A concentration of 5 nM at this step may be regarded as minimum to proceed. If the concentration is lower, additional PCR cycles may be added. This way, an appropriate number of PCR cycle may be chosen for each sample separately, ensuring the balance between a sufficient enrichment and minimizing the risks of PCR-induced artifacts and biases.

Prior to the step of determining the sequence of the excised and tagged DNA segment, the quality of the sequencing library can also be evaluated by testing for enrichment at target loci. This may be done using qPCR on the ready-to-sequence libraries. Such a quality test can give an indication of the success of the methods disclosed here, while only sequencing can give a complete insight into the quality and biology of the data obtained with the disclosed methods. The enrichment test may be used to decide whether to proceed with sequencing the libraries obtained or to repeat the experiment.

In some embodiments, sequencing libraries generated from different samples may be mixed in an equimolar sequencing pool prior to sequencing.

The method of the invention involves determining the sequence of the excised and tagged DNA segment. There are various sequencing methods known in the art. Generally, sequencing can be performed using pyrosequencing on a solid support (such as 454 sequencing, Roche), sequencing-by-synthesis with reversible terminations (such as with the ILLUMINA® Genome Analyzer), or nanopore technology (e.g. Oxford Nanopore Technologies MinION™).

In some embodiments, the excised and tagged DNA fragments are analyzed, for example by determining the nucleotide sequence. In some examples, the nucleotide sequence is determined using sequencing or hybridization techniques with or without amplification. In some embodiments, sequencing may be performed using single-end sequencing. In other embodiments, sequencing may be performed using paired-end sequencing.

Based on the determined sequence of the excised and tagged DNA segment, in the method of invention the at least one chromatin binding site of the chromatin-associated factor of interest in the cell is determined. This step may also be referred to as "mapping" of the at least one chromatin binding site of the chromatin-associated factor of interest. For example, data may be analyzed using sequence comparison software that aligns sequenced nucleic acids to genomic sequences. Genomic sequences are generally known and obtainable from freely accessible data sources. A match of a sequenced nucleic acid, which is found in the sample to be analyzed, and a genomic sequence may be used as indicator that said sequenced nucleic acid is bound by the chromatin-associated factor of interest, for example the histone or transcription factor, which is recognized by the first antibody in the methods of the invention. In this regard, the method of the invention may involve quality control of sequencing reads, for instance using FastQC (Andrews, 2010) algorithm.

In some embodiments, determining the at least one binding site involves trimming of the adaptors. Algorithms for adaptor trimming are well known in the art, for instance cutadapt or trimmomatic. In some embodiments, the trimmed reads are aligned to a reference genome. The reference genome may be obtained by sequencing technologies or from open sources, such as the UCSC genome browser (Rosenbloom et al., 2015). Alignment methods are well known in the art, comprising, for instance, BWA software v.0.7.5a (Li and Durbin, 2009), Bowtie1 and/or Bowtie2. In some embodiments, the data may be filtered for regions blacklisted by the ENCODE project (Hoffman et al., 2013) and/or multimapping reads may be removed, for instance using samtools (Li et al., 2009). Alignment coordinates may be converted to BED format, for instance using BEDTools v.2.17 (Quinlan and Hall, 2010). Based on matching the sequenced nucleic acids to genomic sequences, statistical computational methods may be used to determine regions of significant binding to distinguish them from unspecific "background signal". Peak calling may be performed, for instance, using MACS, epic2, SEACR, GoPeaks, and/or CUT&RUNTools2.0.

Following the step of determining the at least one chromatin binding site of the chromatin-associated factor of interest in the cell, a normalization step may be performed. In some embodiments, normalization may be based on binding sites obtained using an input sample. In some embodiments, normalization may be based on binding sites obtained using an IgG sample. Without being bound by theory, IgG and input samples may capture the background signal and potential unspecific off-targets peaks produced by unspecific tagmentation by the transposase. Therefore, by using either IgG or input to normalize chromatin binding sites obtained in the inventive methods only true peaks will be considered for further processing.

In some embodiments, an input sample is set aside prior to the permeabilization step and subjected to method steps of activating the transposase, thereby excising a DNA segment comprising at least one chromatin binding site of a chromatin-associated factor of interest and tagging the DNA with the first and second DNA molecule; determining the sequence of the excised and tagged DNA segment; and based on the determined sequence of the excised and tagged DNA segment determining the at least one chromatin binding site of the chromatin-associated factor of interest in the cell. In this embodiment, the input sample is used for normalization in the step of determining the at least one chromatin binding site of the chromatin-associated factor of interest in the cell based on the determined sequence of the excised and tagged DNA.

In some embodiments, for a first portion of the cell population an antibody targeting the chromatin-associated factor of interest is used as first antibody and for a second portion of the cell population IgG is used as first antibody and the sequence of the excised DNA determined for the portion of the cell population using IgG as first antibody is used for normalization in the step of determining the sequence of the excised DNA for the portion of cell population using the antibody targeting the chromatin-associated factor of interest as first antibody.

Normalization may be further assessed in a pipeline that quantifies peaks resulting from a Tn5 bias. Pipelines known in the art comprise the NOCOA pipeline as developed by Susami et al. 2022 (Susami, K., Ikeda, S., Hoshino, Y., Honda, S., Minami, N., 2022. Genome-wide profiling of histone H3K4me3 and H3K27me3 modifications in individual blastocysts by CUT&Tag without a solid support (NON-TiE-UP CUT&Tag). Sci. Rep. 12, 11727). Here, the number of peaks not overlapping with reference ChIP-seq peaks (NOC), but overlapping with ATAC-seq peaks (NOCOA) are determined. Then, a false positive rate is calculated by dividing this number by the total number of peaks. With this technique, a false positive rate between 12% and 25% for conventional CUT&Tag protocols on H3K27me3 have been achieved.

In some aspects of the present invention, a method for quality control by determining the percentage of false positives which may result from off-target transposase activity is provided which comprises

(i) conducting the above described steps from permeabilizing the cell to determining the at least one chromatin binding site of the chromatin-associated factor of interest in the cell for the chromatin-associated factor of interest at least twice,

(ii) identifying the chromatin binding sites that overlap for the at least one chromatin binding site determined in each step of determining the at least one chromatin binding site of the chromatin-associated factor of interest in the cell;

(iii) identifying a subset of the chromatin binding sites identified in step (ii) that does not overlap with a reference set of chromatin binding sites determined for the chromatin-associated factor of interest identified using ChIP-seq,

(iv) identifying a subset of the subset of chromatin binding sites identified in step (iii) that overlaps with a reference set of chromatin sites identified using ATAC-seq; and

(v) determining the number of chromatin binding sites identified in step (iv) and dividing said number with the number of chromatin binding sites identified in step (ii) in order to obtain the percentage of false positives.

In one embodiment of this method, step (i) is conducted with at least two replicates. Alternatively, step (i) is conducted at least twice with the same replicate.

In one embodiment of the above-described method for quality control by determining the percentage of false positives, the chromatin binding site of the chromatin-associated factor of interest is present in closed chromatin. In another embodiment, the first or primary antibody specifically binds to a chromatin-associated factor of interest in closed chromatin. For example, the first or primary antibody is an antibody against H3K27me3 or H3K9me3.

The identified at least one chromatin binding site may be used to further infer their biological role by correlating it to other datasets including gene-expression, genome annotation, gene ontology or other systems biology datasets.

**EXAMPLES**

**Introduction:**

Cleavage Under Targets and Tagmentation (CUT&Tag) was first mentioned in 2019 as a novel method for chromatin profiling without chromatin preparation. The cells are bound to magnetic concanavalin A-coated beads during the process until DNA extraction and purification. An antibody against the desired chromatin mark enters the permeabilized cells and thus marks the site in the genome. A subsequently added fusion protein consisting of protein A and the transposase Tn5 is bound to the antibody. After binding of the protein A part to the antibody and activation of Tn5, the latter cuts the DNA and incorporates sequencing adapters at the site. These can be sequenced after extraction and PCR amplification. Conventional CUT&Tag protocols have proven to be a successful for the investigation of histone proteins and certain transcription factors in several species, including humans and mice. However, protein abundancy, binding properties to chromatin, quality of available antibodies, cell models and cell- and tissue-specific expression patterns vary largely between chromatin-associated factors of interest. Therefore, it is desirable to develop a robust protocol that enables high detection sensitivity even when the target protein is low in abundance and/or has weak binding properties to chromatin, limited number of cells available and/or other unfavourable initial conditions are present.

Example 1: Conventional CUT&Tag protocols are not able to detect several non-histone proteins

This example aims to investigate whether conventional CUT&Tag protocols allow the robust detection of different non-histone proteins.

**Materials and methods:**

Cell culture. All cells were grown in an incubator at 37°C, 5% $CO_2$ and 95% humidity.

K-562 cell culture. K-562 cells were cultured in Iscove's Modified Dulbecco's Medium (IMDM, #12440046) medium supplemented with 10% heat inactivated bovine serum (FBS, ThermoFisher, #26170-043) and 1% antibiotic-antimycotic reagent (ThermoFisher, #15240096). The cells were passaged three times per week in a T-75 falcon.

HPC7 cell culture. HPC7 cells were cultured in IMDM medium supplemented with 10% serum (ES), 0.1% stem cell factor (SCF), 1% 2-Mercaptoethanol, 1% L-Glutamine & Glutamax and 1% Penicillin-Streptomycin.

Chromatin Immunoprecipitation (ChiP): HeLa cells were harvested using trypsin-EDTA and crosslinked with formaldehyde according to the iDeal ChIP-seq Kit for Histones (Diagenode). Chromatin was sheared in a Bioruptor (Diagenode) for 11 cycles (30s ON/30s OFF). Sheared chromatin stored at -80°C until use. A shearing assessment was performed according to the manufacturer's instructions. Fragment size was assessed using Bioanalyzer (Agilent Technologies). There was a ChIP-qPCR conducted as a quality control for each new chromatin batch. To start a ChIP experiment, the IP mixes were prepared according to the iDeal ChIP-seq kit for histones (Diagenode) and using antibodies against H3K4me3 and H3K9me3 (both Diagenode). Sheared chromatin was added corresponding to the desired cell number. Enrichment qPCR with primer pairs of positive and negative control regions using KAPA SYBR@ FAST qPCR master mix 2x (Roche) were run on LightCycler96 (Roche) and normalised using input.

Library preparation after CHIP using MicroPlex. The library preparation of ChIP samples was conducted using the MicroPlex Library Preparation Kit v2 (Diagenode). An equimolar pool of libraries was generated and sequenced on NovaSeq instrument (Illumina).

Bioinformatics analysis of ChIP-seq data. Samples were sequenced on NovaSeq instrument (Illumina) using paired-end 2x50 bp mode. A standard ChIP-seq pipeline for paired-end reads was used, i.e., trimming with cutadapt, alignment with bwa mem and peak calling with Sicer.

Conventional Cut & Tag. Cut & Tag was performed as described in conventional protocols. In brief, cells were harvested, washed in wash buffer (20 mM HEPES pH 7.5; 150 mM NaCl; 0.5 mM Spermidine; 1x Protease inhibitor cocktail), and bound to activated, magnetic Concanavalin A (ConA) beads (Polysciences, #86057) for 8 min (10 µl per sample). Beads were resuspended in 50 µl antibody buffer per sample (20 mM HEPES pH 7.5; 150 mM NaCl; 0.5 mM Spermidine; 1x Protease inhibitor cocktail; 0.05% digitonin; 2 mM EDTA; 0.1% BSA) and 1 µg of an antibody against the protein or histone modification of interest was added and incubated overnight at 4°C. The next day, the buffer was removed and 1 µg of a secondary antibody (anti-rabbit or anti-mouse), already mixed 1:100 in 100 µl of antibody buffer, was added and incubated for 45 min at RT. Unbound antibodies were washed away in 1 ml Dig-wash buffer (wash buffer containing 0.05% digitonin) per wash step. pA-Tn5 was added in 1:250 Dig-300 buffer (20 mM HEPES pH 7.5; 300 mM NaCl; 0.5 mM Spermidine; 1x Protease inhibitor cocktail; 0.05% digitonin) and incubated at RT for 1h. Unbound pA-Tn5 was washed away using 1 ml of the same buffer per wash step. Beads were resuspended in 300 µl tagmentation buffer (Dig-300 buffer containing 10 mM $MgCl_2$) and the tagmentation step took place at 37°C for 1h. It was stopped by adding EDTA, SDS and proteinase K, and the following protein digestion took place at 55°C according to the manufacturer's instructions. A phenol-chloroform extraction with subsequent ethanol precipitation was performed and DNA was dissolved in 10 mM Tris-HCl pH8 1 mM EDTA. This was used as input in a PCR with NEBNext High-Fidelity 2x PCR master mix (New England Biolabs, #M0541) and barcoded i5 and i7 sequencing primers (Buenrostro et al., 2015). The following PCR programme was applied (Table 1).

*Table 1 PCR program used in conventional CUT&Tag.*

| Step | Temperature | Duration |
|---|---|---|
| Gap filling | 72°C | 5 min |
| Initial denaturation | 98°C | 30 sec |
| A minimum of 12-14 cycles of: | 98°C<br>63°C | 10 sec<br>10 sec |
| Final elongation | 72°C | 1 min |
| Hold | 8°C | |

An intermediate quantification of the samples was performed using the KAPA Library Quantification kit (see below). After the PCR and a potential re-amplification, samples were purified using AMPure XP beads (Beckman Coulter, #A63880) and resuspended in 10 mM Tris-HCl pH8. Samples were run on Fragment Analyzer using the NGS kit (Agilent Technologies, #DNF-473-0500), pooled in an equimolar ratio and sequenced. All steps until the PCR took place in 1.5 ml tubes and all wash steps were performed with 1 ml of the respective buffer.

intermediate library quantification in CUT&Tag. To evaluate the success of a CUT&Tag experiment, a library quantification using the kit KAPA Library Quantification Kit (Roche, #07960140001) was performed. An aliquot of 0.5 - 1.0 μl was taken from the CUT&Tag samples and mixed in a 1:1000 dilution with library dilution buffer (10 mM Tris-HCl, pH 8.0 - 8.5, 0.05% Tween® 20). These dilutions were vortexed at full speed for 10 seconds. A qPCR was conducted using the sample dilutions and DNA standards of known concentrations (20 pM to 0.0002 pM) and the samples' concentration was calculated according to the manufacturer's instructions. The KAPA Library Quantification Kit ) contains primers matching to sequencing adapters (included in the kit). Therefore, the qPCR amplifies fragments which contain the Illumina sequencing primers. If necessary, the samples could be re-amplified according to the manufacturer's instructions to reach a sufficient concentration high to fulfil the sequencing requirements, and then purified with AMPure XP beads as described above.

Sequencing of CUT&Tag samples: CUT&Tag samples were sequenced in 2x50 bp on an Illumina NovaSeq 6000 flow cell, running NovaSeq Control Software 1.7.5, RTA v3.4.4 and bcl2fastq 2.20 v2.20.0.422.

Bioinformatics analysis of CUT&Tag: Quality control of sequencing reads was performed using FastQC. Trimming of the adaptors was performed with cutadapt (Martin, M., 2011. Cutadapt removes adapter sequences from high-throughput sequencing reads. EMBnet.journal 17, 10. https://doi.org/10.14806/ej.17.1.200). Trimmed reads were aligned to the reference genome obtained from the UCSC genome browser using BWA software v.0.7.5a. Samples were filtered for regions blacklisted by the ENCODE project. Multimapping reads were removed using samtools while PCR duplicates were kept. Alignment coordinates were converted to BED format using BEDTools v.2.17. Peak calling was performed using MACS2 without the broad option, with a q-value of 1e-05. Differential binding analysis was performed with the R/Bioconductor package: DiffBind. Heatmaps around TSS were generated using computeMatrix functions from deepTools using the bigwig files. For the generating of screenshots of genome landscapes, the USCS genome browser and the IGV server have been used.

*Table 2: Antibodies and enzymes used in CUT& Tag and ChIP*

| **Antibody** name | **Supplier and catalogue number** | **Host** | **Experiment type** |
|---|---|---|---|
| H3K4me3 | Diagenode, #C15410003 | Rabbit | CUT&Tag, ChIP |
| H3K9me3 | Diagenode, #C15410193 | Rabbit | CUT&Tag, ChIP |
| H3K27me3 | Diagenode, #C15410195 | Rabbit | CUT&Tag, ChIP |
| PHF19 | Cell Signaling Technology, #77271 | Rabbit | CUT&Tag |
| CTCF | Diagenode, #C15410210 | Rabbit | CUT&Tag, ChIP |
| NRF1 | Diagenode, #C15200013 | Mouse | CUT&Tag, ChIP |
| HDAC2 | Diagenode, #C15200201 | mouse | CUT&Tag, ChIP |
| SUZ12 | Abcam, #ab12073 | Rabbit | CUT&Tag, ChIP |
| JARID2 | Novus Biologicals, #NB100-2214 | Rabbit | CUT&Tag |
| IgG | Diagenode, #C1541 0206 | Rabbit | CUT&Tag |
| pA-Tn5 | Provided by Steven Henikoff, Fred Hutchinson Cancer Center | N/A | CUT&Tag |
| Proteinase K | ThermoFisher, #AM2546 | N/A | CUT&Tag |

**A) Conventional CUT&Tag on Polycomb-group proteins**

**Results:**

The conventional CUT & Tag protocol was performed in 50,000 wild-type (WT) and Phf19 knockdown (kd) haematopoietic progenitor (HPC7) cells using primary antibodies against SUZ12, JARID2 and PHF19 as well as IgG as control. Low library concentrations were observed except for SUZ12 (Figure 1). To gain as much information as possible, samples were sequenced despite the low library concentrations. The sequencing profiles showed almost void profiles (Figure 2) and a high percentage of unmapped reads of 40-90% per sample. These unmapped reads mapped to the *E. coli* genome instead, corresponding to a contamination originating from the pA-Tn5 protein. Besides the *E. coli* peaks, there were only few reads in the samples and no antibody-specific peaks. Interestingly, even the SUZ12 samples that showed a higher library concentration did not lead to the expected results, but had more reads mapping to the *E. coli* reference genome than other samples.

Conclusion:

In conclusion, conventional CUT&Tag protocols are not suitable for investigating Polycomb-group proteins when 50,000 cells are used.

B) Conventional CUT&Tag on histone proteins and other non-histone proteins Results:

The conventional CUT&Tag protocol was also performed for the histone modifications H3K4me3, H3K27me3 and H3K9me3 using 50,000 K-562 cells and for the non-histone proteins CTCF, NRF1 and HDAC2 using 50,000 or 300,000 K-562 cells. As a control, ChIP was performed using the True MicroChIP-seq kit (Diagenode, #C01010132) with the same antibodies using 50,000 cells for histone modifications and the Deal ChIP-seq Kit for Transcription Factors (Diagenode, #C01010055) with 4 million cells for non-histone proteins, respectively. Two replicates per condition were used for all samples.

For ChIP, the subsequent library quantification was performed using the MicroPlex Library Preparation kit (Diagenode, #C05010012). This was not required for CUT&Tag samples as the workflow already integrates sequencing adapters to the resulting target DNA fragments.

*Table 3: Sequencing statistics with the values representing the average of the duplicates. Samples marked with an asterisk consist of only one replicate. FRiP: Fraction of peaks in reads.*

| Experiment type | Number of cells | Antibody | Total reads | Uniquely mapped ratio [%] | Peaks | Average peak width [bp] | Average peak score | FRiP [%] |
|---|---|---|---|---|---|---|---|---|
| **ChIP** | 50,000 | H3K4me3 | 56.7 M | 76.03 | 22,726 | 4,529 | 761 | 0.19 |
| **ChIP** | 50,000 | H3K27me3 | 49.1 M | 89.66 | 4,333 | 192,687 | 889 | 0.48 |
| **ChIP** | 50,000 | H3K9me3 | 56.7 M | 78.62 | 4,688 | 194,851 | 595 | 0.44 |
| **CUT&Tag** | 50,000 | H3K4me3 | 26.5 M | 48.71 | 16,174 | 2,827 | 4,044 | 0.94 |
| **CUT&Tag** | 50,000 | H3K27me3 | 23.0 M | 58.71 | 6,360 | 100,233 | 2,690 | 0.85 |
| **CUT&Tag** | 50,000 | H3K9me3 | 31.4 M | 35.86 | 10,202 | 104,575 | 1,125 | 0.70 |
| **ChIP** | 4 million | CTCF | 61.5 M | 75.52 | 102,979 | 254 | 137 | 0.21 |
| **ChIP** | 4 million | NRF1 | 67.7 M | 80.40 | 8,030 | 250 | 84 | 0.01 |
| **ChIP** | 4 million | HDAC2 | 56.0 M | 83.17 | 11,015 | 289 | 11 | 0.01 |
| **CUT&Tag** | 300,000 | CTCF | 33.7 M | 27.92 | 146,172 | 361 | 82 | 0.69 |
| **CUT&Tag** | 300,000 | NRF1* | 30.4 M | 10.90 | 182,840 | 313 | 63 | 0.69 |
| **CUT&Tag** | 300,000 | HDAC2 | 74.7 M | 3.19 | 151,104 | 262 | 229 | 0.73 |
| **CUT&Tag** | 50,000 | CTCF | 81.8 M | 7.18 | 362,677 | 356 | 70 | 0.73 |
| **CUT&Tag** | 50,000 | NRF1* | 7.6 M | 5.40 | 129,130 | 54 | 27 | 0.69 |
| **CUT&Tag** | 50,000 | HDAC2* | 1.8 M | 4.94 | 23,304 | 41 | 46 | 0.65 |

As expected, the experiments for histone modifications showed a higher library concentration than for CTCF, NRF1 and HDAC2 and a decrease in concentration with cell number (Figure 3). In addition, CUT&Tag samples for non-histone proteins had to be amplified much more than CUT&Tag samples for histone modifications to achieve a sufficient concentration for sequencing (17 vs. 13 PCR cycles).

In conventional CUT&Tag, there is no input sample. Therefore, the ChIP data were not input-normalised to allow a better comparison of the two methods. For the CUT&Tag samples of non-histone proteins, the sequencing statistics showed a decrease in sequencing quality parameters such as peak score and FRiP as well as unrealistically narrow peaks, e.g. peaks of 54 bp in some NRF1 CUT&Tag samples (Table 3).

A low peak score indicates a low confidence in peak calling. It is worth noting that even with ChIP using 4 million cells, the peak scores and FRiP were low for NRF1 and HDAC2 but acceptable for CTCF. This highlights the need for a reliable method to profile non-histone proteins. Nevertheless, the CUT&Tag samples for the histone modifications H3K4me3, H3K27me3 and H3K9me3 had a similar quality to their ChIP-seq counterparts, but with a lower proportion of uniquely mapped reads and a higher proportion of reads in peaks (FRiP). The latter underlines the low background signal in CUT&Tag, which can also be seen in the genome tracks (Figure 4). It should be noted that the comparison of CUT&Tag with ChIP for some parameters, such as the number of peaks, is limited due to the differences in the data, the shape of the peaks and the high signal-to-noise ratio in CUT&Tag (Figure 4).

Only a small proportion of shared peaks was observed between CUT&Tag replicates as well as between CUT&Tag samples and ChIP samples examining non-histone proteins. In contrast, CUT&Tag samples examining histone modifications shared the majority of the peaks among replicates (Table 4). As there are roughly twice as many peaks detected in H3K9me3 CUT&Tag samples than in their ChIP-seq controls, it is logical that their overlap with ChIP is limited to around 50%, which was still achieved. Conversely, however, most H3K9me3 ChIP-seq peaks overlapped with CUT&Tag peaks. H3K4me3 and H3K27me3 also had large overlaps with ChIP-seq peaks. According to the ENCODE criteria, true replicates have at least 75 % of their peaks in common, which was achieved for most samples.

**Table 4: Common peaks among CUT&Tag replicates and their respective ChIP-seq controls. All experiments were performed using 50,000 K-562 cells. CT: CUT&Tag.**

| | CT H3K4me3 A | CT H3K4me3 B | ChIP H3K4me3 A | ChIP H3K4me3 B |
|---|---|---|---|---|
| CT H3K4me3 A | / | 90.40% | 93.75% | 93.75% |
| CT H3K4me3 B | 94.09% | / | 94.92% | 92.10% |
| ChIP H3K4me3 A | 60.52% | 59.01% | / | 73.48% |
| ChIP H3K4me3 B | 70.08% | 68.84% | 86.18% | / |

| | CT H3K27me3 A | CT H3K27me3 B | ChIP H3K27me3 A | ChIP H3K27me3 B |
|---|---|---|---|---|
| CT H3K27me3 A | / | 95.49% | 83.51% | 83.51% |
| CT H3K27me3 B | 93.75% | / | 83.32% | 75.80% |
| ChIP H3K27me3 A | 88.67% | 89.28% | / | 87.31% |
| ChIP H3K27me3 B | 88.15% | 88.91% | 95.63% | / |

| | CT H3K9me3 A | CT H3K9me3 B | ChIP H3K9me3 A | ChIP H3K9me3 B |
|---|---|---|---|---|
| CT H3K9me3 A | / | 91.06% | 52.39% | 52.39% |
| CT H3K9me3 B | 87.44% | / | 51.05% | 51.24% |
| ChIP H3K9me3 A | 89.02% | 90.30% | / | 86.67% |
| ChIP H3K9me3 B | 88.47% | 89.34% | 82.01% | / |

Figure 4A showed a good overlap among CTCF CUT&Tag samples and with ChIP controls. However, the overlaps of HDAC2 and NRF1 between replicates and the ChIP controls are limited. Some samples, as the HDAC2 CUT&Tag and the NRF1 CUT&Tag sample both using 50,000 cells, hardly detected any signal (Figure 4B-C). For the latter, no peaks could be called when using 50,000 cells. However, when increasing the cell number to 300,000, the conventional CUT&Tag protocol could detect peaks better as shown in the overlaps with ChIP.

**Conclusion:**

To conclude, the sequencing statistics, overlap of peaks among replicates and with ChIP controls, and genome tracks confirm that conventional CUT&Tag protocols are able to detect histone modifications. For these samples, the conventional CUT&Tag protocol shows a good reproducibility according to ENCODE criteria.

In contrast, the conventional CUT&Tag protocol had difficulties detecting non-histone proteins CTCF, NRF1 and HDAC2 even with high cell numbers such as 300,000 K-562 cells. Even the peaks of the more easily detectable protein CTCF overlap only about 40% between the replicates or with ChIP-seq controls. The genome tracks showed some shared peaks with ChIP, especially in the case of CTCF, but other peaks were missing, indicating a lack of sensitivity. Together with the experiment on PRC2 proteins using 50,000 HPC7 cells shown in Example 1A, this emphasizes the need for improved methods to study non-histone proteins in low cell numbers such as 50,000 cells.

Example 2: Permeabilization with Triton-X-100 increases safety and lowers variability

**Introduction:**

In conventional CUT&Tag, digitonin is used to permeabilize the cells by interacting with cholesterol in the membrane, thereby allowing the antibodies, pA-Tn5 and other reagents to enter the cell membrane and nucleus.

However, the use of digitonin has several disadvantages. Digitonin is toxic, especially when dissolved in DMSO, which penetrates the skin, and therefore requires handling under a chemical safety hood. In addition, the digitonin stock solutions are not stable for long, so a new stock solution often needs to be prepared. Furthermore, digitonin is extracted from the *Digitalis purpurea* plant, resulting to batch-to-batch variations. The use of different batches of digitonin in turn affects the reproducibility of the CUT&Tag samples.

It is thus desirable to use less toxic, more stable permeabilization reagents that show less batch-to-batch variability. One potential alternative is Triton X-100 which has been used to permeabilize cells in other methods, such as CUT&RUN.

The aim of this example is to investigate whether the use of Triton X-100 as a permeabilization reagent in CUT&Tag provides comparable cell permeabilization and sequencing results to digitonin.

**Materials and methods:**

The conventional CUT&Tag protocol, as described in Example 1, was performed for the histone protein H3K4me3 using 50,000 K-562 cells. The cells were permeabilized with 0.1% Triton X-100 or digitonin (control) and sequenced as described in Example 1.

**Results:**

*Table 5: Sequencing statistics of experiments using the conventional CUT&Tag protocol with either Triton X-100 (TX) or digitonin (Dig; control) as permeabilization reagent. The experiments were carried out in duplicates with 50,000 K-562 cells per experiment. FRiP: Fraction of peaks in reads.*

| Sample | Total reads | Uniquely mapped ratio [%] | Peaks | Average peak width | Average peak score | FRIP [%] |
|---|---|---|---|---|---|---|
| TX H3K4me3 A | 30.5 M | 93.72 | 21,164 | 3,766 | 5,753 | 0.86 |
| TX H3K4me3 B | 46.0 M | 93.31 | 23,801 | 3,102 | 5,499 | 0.90 |
| Dig H3K4me3 A | 32.3 M | 42.91 | 16,438 | 2,875 | 4,424 | 0.94 |
| Dig H3K4me3 B | 20.7 M | 54.52 | 15,910 | 2,779 | 3,664 | 0.95 |

All CUT&Tag samples showed similar sequencing statistics and a high FRiP (Table 5) as well as a high proportion of shared peaks between replicates and compared between permeabilization conditions of 89%. Therefore, both permeabilization strategies led to consistent sequencing results with similar signal. In addition, the genomic tracks showed good overlap when compared to H3K4me3 ChIP-seq data generated with 50,000 K-562 cells (Figure 5).

**Conclusions:**

In summary, the use of Triton X-100 as a cell permeabilization reagent in CUT&Tag resulted in good overlap between replicates and compared to data obtained with digitonin. Triton X-100 therefore represents a safer and less variable alternative to digitonin for the purposes of CUT&Tag experiments.

Example 3: Improved methods for CUT&Tag

**Introduction:**

In conventional CUT&Tag protocols, high salt concentrations of 300 mM NaCl are considered necessary for the complete removal of unbound pA-Tn5 during the washing steps. This is considered to be important since upon addition of the tagmentation buffer containing $MgCl_2$, unbound pA-Tn5 is activated and may cut accessible chromatin sites in addition to antibody-specific target sites.

However, at the same time, high salt concentrations may result in the detachment of more proteins that are weaker and/or more dynamically bound to chromatin, as for instance non-histone proteins, which may prevent their robust detection using CUT&Tag.

This example aims to investigate, whether the modification of conventional CUT&Tag protocols enables robust detection of non-histone proteins even under high salt concentrations.

A crowding agent was added. Without being bound to a particular theory, crowding agents may mimic the "crowding" of cytoplasm and nucleus with macromolecules, thus mimicking a situation closer to the actual situation *in vivo* by stabilizing DNA-protein interactions.

In addition, a different buffer base was used. Without being bound to a particular theory, using a different buffer base may achieve a more appropriate pH range during the tagmentation step and/or chelate $Mg^{2+}$ in the solution, e.g. residual $Mg^{2+}$ from the cells, which may help to avoid premature activation of pA-Tn5 and non-specific cutting of accessible chromatin.

Furthermore, light crosslinking was performed immediately after the harvest. Without being bound to a particular theory, light crosslinking, e.g. using 0.1% formaldehyde for 5 min, may further stabilize protein DNA-interactions without resulting in the masking of epitopes and thus making the target protein less accessible for antibodies and pA-Tn5.

Moreover, 0.2 ml PCR tube strips were used for each individual sample instead of 1.5 ml tubes. This may advantageously reduce the amount of beads clumping on the tube wall or cap and may facilitate resuspension, thereby increasing the recovery. The volumes of buffers were adjusted accordingly, such that the volume of the wash buffers and of the tagmentation buffer was reduced to 200 $\mu$l as compared to 0.8 to 1.0 ml used in conventional protocols. These changes enable the use of multichannel pipettes for the washing steps, which has the advantage of reducing the hands-on time during the washing steps, thereby increasing throughput. In addition, the use of 0.2 ml PCR tubes may improve data quality and recovery as the exposure time of samples to buffers is reduced. Without being bound by theory, decreased exposure times may prevent the change of chromatin structures and detachment of proteins from DNA.

Furthermore, $MgCl_2$ was added prior to the activation of the transposase in the tagmentation buffer.

In addition, the amount of pA-Tn5 was increased from a 1:250 to a 1:100 dilution and the amount of primary antibody was increased from 1 $\mu$g to 2 $\mu$g per reaction for 5,000 cells and more and to 3 $\mu$g per reaction for 50,000 cells and more, respectively. Dilution of pA-Tn5 and antibody amounts were adjusted based on routine measures known to the skilled person. Typically, the amount of pA-Tn5 and antibodies used in CUT&Tag is evaluated for each batch of pA-Tn5 and/or antibody and for each target chromatin factor of interest. Methods to determine an optimized dilution of pA-Tn5 and amount of primary and secondary antibodies are well known in the art. These include, for example, the titration of pA-Tn5 and antibody amounts, respectively, and, after subjecting the samples to CUT&Tag, evaluating the library concentration and the data obtained after sequencing, e.g. by visual inspection and bioinformatic analysis.

**Materials and methods:**

Cell culture. All cells were grown in an incubator at 37°C, 5% $CO_2$ and 95% humidity.

K-562 cell culture. K-562 cells were cultured in Iscove's Modified Dulbecco's Medium (IMDM, #12440046) medium supplemented with 10% heat inactivated bovine serum (FBS, ThermoFisher, #26170-043) and 1% antibiotic-antimycotic reagent (ThermoFisher, #15240096). The cells were passaged three times per week in a T-75 falcon.

CUT&Tag: In general, all wash steps and buffer removals after the cells were bound to the magnetic ConA beads took place on a magnet after a quick spin. All incubations took place on a rotator except the tagmentation step which took place on a thermocycler for 0.2 ml tubes. A multichannel pipette can be used for most wash steps and some reagents additions. Samples were processed in duplicates or triplicates.

For frozen cell pellets: Cells were thawed on ice for 2 min, resuspended in cold Buffer D (20 mM Tricin KOH pH 7.8, 0.1% Triton X-100, 25 mM KCI, sucrose 250 mM, protease inhibitors cocktail) and bound to washed ConA beads. From here, the steps remain the same as for fresh cells.

For freshly harvested cells: Cells were crosslinked in 0.1% formaldehyde (Merck Millipore, #F8775) for 5 min and quenched with glycine for additional 5 min. Cells were washed twice in Dulbecco's phosphate-buffered saline (DPBS, ThermoFisher, #14040133). From this point, all steps took place on ice or 4°C. Cells were resuspended in 50 $\mu$l of cold Antibody buffer D (20 mM Tricine KOH pH 7.8, 0.1% Triton X-100, 25 mM KCI, sucrose 250 mM, 5 mM $MgCl_2$, 1x protease inhibitors cocktail) per sample. If desired, an aliquot of CUT&Tag input can be put aside at this step. The remaining cells were bound to ConA beads for 20 min (10 $\mu$l per sample). Beads were resuspended in 50 $\mu$l of Antibody buffer D (20 mM Tricine KOH pH 7.8, 0.1% Triton X-100, 25 mM KCI, 250 mM sucrose, 150 mM NaCl, 2 mM EDTA, 0.1% BSA, 5 mM $MgCl_2$, 1X protease inhibitor cocktail, 0.5 mM spermidine) per sample, distributed into 0.2 ml tube strips and 2 $\mu$g of the primary antibody for 5,000 cells and more and 3 $\mu$g of the primary antibody for 50,000 cells and more, respectively, was added unless stated otherwise. This was incubated overnight at 4°C. The next day, the buffer was removed. Beads were again resuspended in 100 $\mu$l of Antibody buffer D (20 mM Tricine KOH pH 7.8, 0.1 % Triton X-100, 25 mM KCI, 250 mM sucrose, 150 mM NaCl, 2mM EDTA, 0.1% BSA, 5 mM $MgCl_2$, 1X protease inhibitor cocktails, 0.5 mM spermidine). 3 $\mu$g of the secondary antibody was added and incubated for 45 min. 100 $\mu$l of a 1:100 pA-Tn5 dilution (Diagenode, #C01070001) in Buffer D300 (20 mM Tricine KOH pH 7.8, 0.1% Triton X-100, 25 mM KCI, 250 mM sucrose, 300 mM NaCl, 5 mM $MgCl_2$, 1X protease inhibitors cocktail, 0.5 mM spermidine) was added and incubated at 4°C for 1h. Unbound pA-Tn5 was washed away in 200 $\mu$l of the same buffer.

Beads were resuspended in 200 $\mu$l of Tagmentation Buffer D300 (20 mM Tricine KOH pH 7.8, 0.1% Triton X-100, 25 mM KCI, 250 mM sucrose, 300 mM NaCl, 15 mM $MgCl_2$, 1X protease inhibitor cocktail, 0.5 mM spermidine) and incubated at 37°C for 1h. Input samples were included at this step. Tagmentation was stopped by adding 0.5 M EDTA, 10% SDS and proteinase K, and the following protein digestion took place at 55°C according to the manufacturer's instructions. A phenol-chloroform extraction with subsequent ethanol precipitation was performed and DNA was dissolved in 10 mM Tris-HCl pH8 1 mM EDTA. The resulting DNA solution was used in a PCR with NEBNext High-Fidelity 2x PCR master mix (New England Biolabs, #M0541) and barcoded i5 and i7 sequencing primers. Samples were subjected to PCR (Table 6).

*Table 6: PCR protocol for the CUT&Tag protocols disclosed herein.*

| Step | Temperat ure | Duration |
|---|---|---|
| Gap filling | 72°C | 5 min |
| Initial denaturation | 98°C | 30 sec |
| 8 - 18 cycles of: | | |
| | 98°C | 10 sec |
| | 63°C | 10 sec |
| Final elongation | 72°C | 1 min |
| Hold | 4°C | |

An intermediate quantification of the samples was performed using the KAPA Library Quantification kit (see below). After the PCR and a potential re-amplification, samples were purified using AMPure XP beads (Beckman Coulter, #A63880) and resuspended in 10 mM Tris-HCl pH8. An enrichment qPCR was performed for most samples to test enrichment of known loci (see below). Samples were run on Fragment Analyzer using the NGS kit (Agilent Technologies, #DNF-473-0500), pooled in an equimolar ratio and sequenced.

All steps after the mentioned distribution into 0.2 ml tube strips took place in these tubes until a transfer in new 0.2 ml tubes for the PCR step. All wash steps were performed with 0.2 ml of the respective buffer.

Intermediate library quantification in CUT&Tag. To evaluate the success of a CUT&Tag experiment, a library quantification using the kit KAPA Library Quantification Kit (Roche, #07960140001) was performed. An aliquot of 0.5 - 1.0 μl was taken from the CUT&Tag samples and mixed in a 1:1000 dilution with library dilution buffer (10 mM Tris-HCl, pH 8.0 -8.5, 0.05% Tween® 20). These dilutions were vortexed at full speed for 10 seconds. A qPCR was conducted using the sample dilutions and DNA standards of known concentrations (20 pM to 0.0002 pM) and the samples' concentration was calculated according to the manufacturer's instructions. The KAPA Library Quantification Kit) contains primers matching to sequencing adapters (included in the kit). Therefore, the qPCR amplifies fragments which contain the Illumina sequencing primers. If necessary, the samples could be re-amplified according to the manufacturer's instructions to reach a sufficient concentration high to fulfil the sequencing requirements, and then purified with AMPure XP beads as described above.

*Table 7: Antibodies and enzymes used in CUT&Tag and ChIP*

| Antibody name | Supplier and catalogue number | Host | Experiment type |
|---|---|---|---|
| H3K4me3 | Diagenode, #C15410003 | Rabbit | CUT&Tag, ChIP |
| H3K27me3 | Diagenode, #C15410195 | Rabbit | CUT&Tag, ChIP |
| CTCF | Diagenode, #C15410210 | Rabbit | CUT&Tag, ChIP |
| HDAC2 | Diagenode, #C15200201 | mouse | CUT&Tag, ChIP |
| EZH2 | Diagenode, #C15410039 | Rabbit | CUT&Tag |
| IgG | Diagenode, #C15410206 | Rabbit | CUT&Tag |
| pA-Tn5 | Diagenode, #C01070001 | N/A | CUT&Tag |
| Proteinase K | ThermoFisher, #AM2546 | N/A | CUT&Tag |

Sequencing of CUT&Tag samples. CUT&Tag samples were sequenced in 2x50 bp on an Illumina NovaSeq 6000 flow cell, running NovaSeq Control Software 1.7.5, RTA v3.4.4 and bcl2fastq 2.20 v2.20.0.422.

Bioinformatics analysis of CUT&Tag: Quality control of sequencing reads was performed using FastQC. Trimming of the adaptors was performed with cutadapt. Trimmed reads were aligned to the reference genome obtained from the UCSC genome browser using BWA software v.0.7.5a (Rosenbloom, K.R., Armstrong, J., Barber, G.P., Casper, J., Clawson, H., Diekhans, M., Dreszer, T.R., Fujita, P.A., Guruvadoo, L., Haeussler, M., Harte, R.A., Heitner, S., Hickey, G., Hinrichs, A.S., Hubley, R., Karolchik, D., Learned, K., Lee, B.T., Li, C.H., Miga, K.H., Nguyen, N., Paten, B., Raney, B.J., Smit, A.F.A., Speir, M.L., Zweig, A.S., Haussler, D., Kuhn, R.M., Kent, W.J., 2015. The UCSC Ge- nome Browser database: 2015 update. Nucleic Acids Res. 43, D670-D681. https://doi.org/10.1093/nar/gku1177). Samples were filtered for regions blacklisted by the ENCODE project (Landt, S.G., Marinov, G.K., Kundaje, A., Kheradpour, P., Pauli, F., Batzoglou, S., Bernstein, B.E., Bickel, P., Brown, J.B., Cayting, P., Chen, Y., DeSalvo, G., Epstein, C., Fisher-Aylor, K.I., Euskirchen, G.,

Gerstein, M., Gertz, J., Hartemink, A.J., Hoffman, M.M., Iyer, V.R., Jung, Y.L., Karmakar, S., Kellis, M., Kharchenko, P.V., Li, Q., Liu, T., Liu, X.S., Ma, L., Milosavljevic, A., Myers, R.M., Park, P.J., Pazin, M.J., Perry, M.D., Raha, D., Reddy, T.E., Rozowsky, J., Shoresh, N., Sidow, A., Slattery, M., Stamatoyannopoulos, J.A., Tolstorukov, M.Y., White, K.P., Xi, S., Farnham, P.J., Lieb, J.D., Wold, B.J., Snyder, M., 2012. ChIP-seq guidelines and practices of the ENCODE and modENCODE consortia. Genome Res. 22, 1813-1831. https://doi.org/10.1101/gr.136184.111). Multimapping reads were removed using samtools while PCR duplicates were kept. Alignment coordinates were converted to BED format using BEDTools v.2.17. Peak calling was performed using MACS2 without the broad option, with a q-value of 1e-05. Differential binding analysis was performed with the R/Bioconductor package: DiffBind. Heatmaps around TSS were generated using computeMatrix functions from deepTools using the bigwig files. For the generating of screenshots of genome landscapes, the USCS genome browser and the IGV server have been used.

**Results.**

50,000 K-562 cells were subjected to either the conventional CUT&Tag protocol described in Example 1 or the CUT&Tag protocol described in this example with or without crosslinking. Antibodies targeting CTCF or H3K4me3 were used as well as IgG (control).

Using the CUT&Tag protocol described herein, the library concentration for CTCF could be vastly increased using 50,000 K-562 cells, even when no crosslinking was used (Figure 6). As expected, the histone protein modification H3K4me3 could be detected in all protocol versions.

In a further experiment, the CUT&Tag protocol disclosed herein was also applied to non-histone proteins CTCF, EZH2, HDAC2. Antibodies against the histone protein modification H3K27me3 and IgG were used as controls. Either 100,000, 50,000, 10,000 or 5,000 K-562 cells were washed twice in Buffer D to extract the nuclei and then cross-linked according to the protocol described above.

Relatively high library concentrations were achieved for all samples, especially for CTCF using 100,000 K-562 cells, while using the same number of PCR cycles (14) compared to previous experiments (Figure 7). Nevertheless, the concentrations using 10,000 and 5,000 K-562 cells were still comparatively low, indicating that these cell numbers were still below the threshold for robust detection.

Samples assessing CTCF, EZH2 and HDAC2 using 1000,000 and 50,000 cells, respectively, were further subjected to sequencing.

*Table 8 Sequencing statistics of experiments using CUT&Tag as disclosed herein. Experiments were performed using 100,000 (100K) or 50,000 (50K) K-562 cells. FRiP: Fraction of reads in peaks.*

| Antibody | Cell number and | Total reads | Uniquely mapped ratio | Peaks | Average peak width | Average peak score | FRiP [%] |
|---|---|---|---|---|---|---|---|
| CTCF | 100K A | 14.7 M | 88.89 | 38,88 | 433.86 | 13.91 | 0.14 |
|  | 100K B | 167.1 | 88.49 | 122,7 | 399.21 | 20.44 | 0.29 |
|  | 50K A | 38.9 M | 89.68 | 62,55 | 379.87 | 16.77 | 0.21 |
|  | 50K B | 30.4 M | 88.76 | 56,67 | 350.38 | 15.97 | 0.21 |
| EZH2 | 100K A | 66.6 M | 85.94 | 46,28 | 352.13 | 16.13 | 0.09 |
|  | 100K B | 24.7 M | 87.80 | 22,50 | 431.61 | 14.24 | 0.05 |
|  | 50K A | 13.2 M | 87.89 | 0 | 369.36 | 11.17 | 0.03 |
|  | 50K B | 13.4 M | 85.75 | 18,69 | 386.07 | 13.03 | 0.07 |
| HDAC2 | 100K A | 53.9 M | 90.79 | 93,10 | 313.27 | 18.80 | 0.34 |
|  | 100K B | 137.6 | 89.93 | 122,7 | 372.06 | 18.24 | 0.34 |
|  | 50K A | M | 89.86 | 89 | 343.05 | 18.77 | 0.30 |
|  | 50K B | 78.8 M | 90.27 | 70,45 | 340.38 | 16.78 | 0.22 |

The number of reads obtained was quite heterogeneous between samples (Table 8). The fraction of reads in peaks (FRiP) was 21% for CTCF, 5-7% for EZH2 and 26-34% for HDAC2.

The CUT&Tag peaks were further compared with ChIP-seq peaks from ENCODE. When comparing CUT&Tag with ChIP-seq data it has to be taken into consideration that the peaks in the ENCODE ChIP-seq data set are much broader than for CUT&Tag. The latter has more and smaller peaks whereas the same enrichment was called as one broad peak in ENCODE. Therefore, a comparison of number of peaks or common peaks is limited.

A high number of reads also increases the number of peaks, as the background is artificially enriched and called as a peak. This was the case for CTCF CUT&Tag replicate B in 100,000 cells: due to the high number of reads for this replicate, some of the background signal was sufficiently enriched to be called as peak. This contributed to the higher FRiP of 28% and a much higher coverage in genome tracks, and many small enrichments were called as peaks (Figure 8). This also showed that a too high sequencing depth may bias the results. Also, the scale of the heatmap was increased to 150, while the replicates using 50,000 cells showed a signal of about 25 on the scale (Figure 9). Few of the false positive peaks in background were expected to overlap with true peaks of the other samples.

The CUT&Tag replicates showed a high correlation of around 0.9 based on peaks occupancy (Figure 8), except one CUT&Tag replicate using 100,000 cells, which also showed a much lower signal in genomic tracks and other aspects of the results. In contrast, the overlap between all CUT&Tag and the corresponding ENCODE ChIP reference data was only 0.2. Interestingly, the correlation between the two ENCODE EZH2 replicates was only about 0.3, which is another indication that ENCODE data is not always a good reference. Similar to the CTCF samples, the EZH2 samples also showed a high correlation between 0.7 and 0.9. The correlation between CUT&Tag and ENCODE data was basically non-existent. The same trend was observed for the HDAC2 CUT&Tag data. The correlation was around 0.95 between the HDAC2 CUT&Tag replicates, but only 0.4 when compared to the ENCODE data. This shows that the CUT&Tag protocol disclosed herein resulted a high reproducibility between CUT&Tag replicates.

To further assess whether the detected peaks were located in the target genes, genome tracks at known target loci were investigated (Figure 10).

Moreover, the functions computeMatrix and plotHeatmap from deepTools were used to create heatmaps around TSS from bigwig files. The signals of the CUT&Tag CTCF, EZH2 and HDAC2 samples 3 kb upstream and downstream of the TSS were found to be heterogeneous, which is related to the heterogeneous number of reads (Figure 9). Despite their heterogeneity, most of the CUT&Tag samples showed a higher signal than the ENCODE ChIP-seq samples. This can be observed in particular for the ENCODE EZH2 ChIP-seq data, for which the high background leads to peak profiles that are more orange than red.

All EZH2 CUT&Tag samples showed noise instead of the expected broad peak at the *HOXA* locus as seen in the ENCODE data. Only one replicate with 100,000 cells (100K_A) appeared to resemble the ENCODE signal. This confirmed the high signal of this replicate observed in the heatmaps (Figure 9). At the *PHF19* locus, some peaks present in the ENCODE data were also seen in the EZH2 CUT&Tag data, while others were absent. The HDAC2 CUT&Tag data showed the same signal as the ENCODE reference data, albeit with higher intensity and lower background level. A CUT&Tag replicate generated with 100,000 cells had a slightly lower intensity than the others, but still showed the same form of enrichment.

**Conclusion:**

In summary, the CUT&Tag protocol disclosed herein was shown to detect the non-histone protein CTCF in 50,000 K-562 cells, which was not possible using conventional protocols. The use of a crowding agent was a major driving force of the improved detection. In addition, the use of another buffer basis may have generated a more suitable pH range during the tagmentation step. The use of light crosslinking immediately after the harvest may have further contributed to the improved detection. Moreover, lowering of the buffer volumes to 200 μl per step and per sample allowed more efficient washing and reactions. For EZH2 and HDAC2, the data showed a higher success than conventional protocols in HPC7 cells, or even in 300,000 of K-562 cells.

Example 4:

**Introduction**

A challenge when examining CUT&Tag data assessing low abundance proteins with weaker interaction with DNA compared to histone modifications, such as PRC2 proteins, is to distinguish between a low but true positive signal and a true negative background signal. As seen in the previous example, the comparison of CUT&Tag data with ChIP-seq data is limited due to the high background and lower sensitivity of ChIP-seq, which may lead to false negative peaks.

This example aims to confirm the results obtained in K-562 cell using a WT/KO comparison approach in mouse embryonic stem cells (mESCs). A KO in the SUZ12 gene is not only expected to decrease the signal obtained with antibodies against SUZ12 itself, but also when using antibodies against the other PRC2 core proteins EZH2 and EED as well as H3K27me3, since PRC2 is its deposition complex. Thus, by using *Suz12$^{-/-}$* (SUZ12 KO) mESCs a WT/KO comparison may be achieved for all these proteins. The data obtained with Suz12$^{-/-}$ cells may allow a more in-depth analysis of the WT data. For example, the lists of detected genes in WT and KO cell lines can be compared to estimate overlaps and the nature of the signal, which may be used to further improve the protocol disclosed herein.

**Methods:**

The CUT&Tag protocol disclosed in Example 3 was performed using 50,000 WT and Suz12$^{-/-}$ mESCs, generated using a CRISPR/Cas9 approach, respectively. As a KO-independent control, CTCF was assessed using CUT&Tag in both WT and Suz12$^{-/-}$ mESCs. The cells were crosslinked immediately after harvest and cryopreserved. In contrast to the previous Example, the cell nuclei were not extracted, but the cells were permeabilized with Buffer D and bound to ConA beads. This may lead to a higher recovery, as several centrifugation steps are omitted compared to nucleus extraction. CUT&Tag experiments were performed with antibodies against H3K27me3, CTCF, EZH2, SUZ12 or IgG using 50,000 WT and Suz12$^{-/-}$ mESCs, respectively. The H3K27me3 samples were processed in duplicate, the PRC2 samples in triplicate and the CTCF and IgG samples as single samples.

**Results:**

The library concentrations of all samples were sufficiently high for sequencing and were in a similar range in both WT and Suz12$^{-/-}$ cells and compared to IgG samples (Figure 11A). This was surprising, as a lower library yield was expected for the CUT&Tag in Suz12$^{-/-}$ cells, with the exception of CTCF, when the library concentration is normalized to the same number of PCR cycles. Nevertheless, the PRC2 samples in mESC-WT cells showed considerable enrichment at the target loci in contrast to the data generated using Suz12$^{-/-}$ cells and IgG (Figure 11B). Both the library concentration and the relative enrichment were merely used as indicators to decide whether the concentration of the samples was sufficient for sequencing.

In addition, CUT&Tag was analyzed for CTCF in both WT and Suz12$^{-/-}$ cells, as this should result in the same signal in both cell lines. Despite the difference in relative enrichment at C-MYC1 observed in qPCR (Figure 11B), the CUT&Tag data on CTCF showed a high overlap of gene sets between cells (Figure 12A) and in the genomic landscape (Figure 12C), as expected. In addition, the expected matches with low p-values were observed for both cell lines in the ChEA 2022 database using Enrichr (Figure 12B). The low p-value obtained in the Enrichr analysis indicates a very specific and correct signal. This further suggested a high reproducibility and specificity of the CUT&Tag protocol disclosed herein.

Moreover, the same functional analysis using Enrichr was performed using CUT&Tag data on SUZ12, EZH2 and H3K27me3 in both WT and Suz12$^{-/-}$ cells, resulting in lists of detected genes. In WT mESCs, the CUT&Tag samples showed a large overlap of genes between replicates (Figure 13A). Therefore, only one replicate was shown in some of the later figures. In addition, as expected, a strong overlap was observed between the EZH2 and SUZ12 gene sets and a moderately high overlap between H3K27me3 and the PRC2 proteins (Figure 13D). Using H3K27me3 CUT&Tag in WT cells, about 3,000 genes were detected, which is within the expected range. Nevertheless, surprisingly, many genes were detected for CUT&Tag on H3K27me3, EZH2 and SUZ12 even in Suz12$^{-/-}$ cells, some of which were also detected in WT cells (Figure 13C).

The genomic tracks showed the expected pattern at the HOXA cluster in WT mESC for H3K27me3, EZH2 and SUZ12 CUT&Tag samples (Figure 14B). In addition, as expected, a lack of enrichment at the HOXA cluster was observed for EZH2 and SUZ12 CUT&Tag samples in Suz12$^{-/-}$ cells. However, *de novo* peaks were observed in both H3K27me3 repeats in Suz12$^{-/-}$ cells. This was surprising as no signal was expected in the absence of SUZ12, especially at sites that did not overlap with the WT signal.

To find reasons for the surprisingly high number of genes detected in Suz12$^{-/-}$ cells, the signal intensity at the locations of the peaks of the three subgroups was examined (Figure 14A). If the signal from the KO peaks was low, it could be argued that such peaks represent background and that adjusting the peak caller would solve the problem. However, the signal of the KO sample at the locations of the KO-only peaks was higher than that of the WT sample at the same locations, which represented background, and comparable to the level of the WT-only peaks, i.e. true peaks (Figure 14B). Therefore, the peaks in the KO samples turned out to be actual enrichments, which was unexpected. The genome distribution shows further differences. The KO-only peaks were found less frequently in promoters, the classical target sites of SUZ12, but more frequently in genes (26.5 %) and intergenic regions (28.5 %), compared to the WT-only peaks with 18.6 % and 17.8 %, respectively (Figure 14C).

Next, the subsets of WT-only peaks, common peaks, and KO-only peaks were assigned to genes, and three sets of genes were thus identified. The group of common genes was quite large (Figure 15A). Functional analysis of the gene sets using Enrichr showed that the WT-only gene set had features of true PRC2 targets, as it matched the Enrichr ENCODE database for histone modifications (Figure 15B). In contrast, the approximately 3,800 genes overlapping between WT and KO samples and the approximately 1,800 genes detected only in KO cells showed features of expressed genes, such as matches with H3K4me3 and H3K79me2/3 gene sets, which is unusual for PRC2 and H3K27me3.

The peaks in actively expressed regions may be due to a Tn5 bias in the protein A-Tn5 transposase used in CUT&Tag. Solitary Tn5 tends to cut in accessible chromatin and generates libraries with attached sequencing adaptors. This is exploited in ATAC-seq to obtain information about accessible chromatin. In CUT&Tag, on the other hand, pA-Tn5 (comprising a still inactive Tn5) is bound to antibodies against the desired chromatin protein via the protein A part. After removal of unbound pA-Tn5 by washing steps, it is expected that only antibody-bound pA-Tn5 remains, which is then activated by MgCl$_2$ in the tagmentation buffer, resulting in cuts and the generation of libraries only at these sites.

The count of reads at the TSS of the three gene sets of the WT and KO samples was analyzed (Figure 15C). At the TSS of WT-only genes, the KO samples were found to have the expected reduced signal compared to the WT samples. However, in common and KO-only genes, the signal was found to be comparable between WT and KO samples.

This was confirmed by the ChIP reference data to SUZ12. WT-only genes showed higher signal at the TSS of genes detected in SUZ12 ChIP, while common genes between WT and KO cells showed no SUZ12 signal (Figure 15D). Moreover, when compared with ATAC-seq data, common genes showed higher ATAC-seq peaks in WT mESC, corresponding to accessible chromatin, while WT-only genes showed the lowest ATAC-seq peak intensity, corresponding to expected compact chromatin (Figure 15D). RNA-seq data showed that genes in KO-only samples and common genes between WT and KO had higher expression levels than genes in WT-only samples.

**Conclusion:**

In conclusion, analysis with a KO cell line enabled a deeper understanding of the data and an assessment of the data quality. Without the use of a KO cell line, the detection of expected target genes may overshadow the additional detection of unexpected peaks, e.g. the detection of PRC2 peaks in open chromatin due to the bias of Tn5 to cut in open chromatin. Here, evidence of a strong Tn5 bias was found analyzing common peaks between WT and KO cells and KO-only peaks as these corresponded to active chromatin, and countermeasures could be taken. Despite the peaks in open chromatin, the protocol described in Example 3 showed promising results with high reproducibility between replicates using 50,000 mESCs. In addition to some adjustments in the experimental design, normalization of the signal could be beneficial to fully evaluate the success of the protocol and remove the peaks in open chromatin.

Example 5: Improved methods for CUT&Tag

**Introduction:**

The protocol disclosed in Example 3 was able to detect the peaks and associated genes expected for each chromatin-associated target factor and cell model. However, a bias towards open chromatin was also evident, most likely due to off-target activity of Tn5, leading to unexpected peaks at expressed genes in addition to peaks at PRC2 targets. To solve this issue, the protocol was further modified.

The amount of pA-Tn5 was lowered from a 1:100 to a 1:250 dilution. Without being bound to particular theory, this may reduce Tn5 off-target activity.

Furthermore, the amount of antibody was reduced from 3 $\mu$g to 1 $\mu$g per reaction. Without being bound to particular theory, this may reduce Tn5 off-target activity.

Also, MgCl$_2$ was not added until the tagmentation step, as described in the conventional protocol. Without being bound to a particular theory, this may reduce Tn5 off-target activity and thus reduce the detection of false-positive peaks in the data set while not affecting the detection of the true positive binding sites of the chromatin-associated factor of interest as observed in Example 3.

Moreover, peaks were normalized either using an input sample-based normalization strategy as described herein or an IgG-based normalization strategy. Without being bound to particular theory, peak normalization of CUT&Tag data may leave only true positive peaks for further processing.

The ratio of false positive peaks resulting from a potential Tn5 bias was assessed using a bioinformatics pipeline disclosed herein.

This example aims to demonstrate that further modifications to the protocol disclosed in Example 3 further improved the avoidance of false-positive peaks due to a Tn5 bias. Moreover, this example aims to demonstrate that the protocol disclosed herein can be successfully applied using cell numbers of 5,000 cells even when studying non-histone proteins.

**Materials and methods:**

Culture of mouse embryonic stem cells (mESCs). ES-E14TG2a were cultured in Glasgow's Minimal Essential Medium (GMEM, Gibco, 11710035), supplemented with 20% FBS (ES cell qualified, FisherScientific, #15946942), 1% Glutamax (FisherScientific, #11574466), 1% non-essential amino acids (FisherScientific #12084947), 1 mM sodium pyruvate (FisherScientific, #11530396), 1 mM 2-Mercaptoethanol (ThermoFisher, #21985023), 100 U Penicillin-Streptomycin (ThermoFisher, #15140122), 1,000 U/ml mouse Leukaemia Inhibitory Factor (mLIF, Merck Millipore, #ESG1106), on flasks coated with 0.1% gelatine.

CUT&Tag: In general, all wash steps and buffer removals after the cells were bound to the magnetic ConA beads took place on a magnet after a quick spin. All incubations took place on a rotator except the tagmentation step which took place on a thermocycler for 0.2 ml tubes. A multichannel pipette can be used for most wash steps and some reagents additions. Samples were processed in duplicates or triplicates.

For frozen cell pellets: Cells were thawed on ice for 2 min, resuspended in Buffer D (20 mM Tricin KOH pH 7.8, 0.1% Triton X-100, 25 mM KCI, sucrose 250 mM, protease inhibitors cocktail) and bound to washed ConA beads. From here, the steps remain the same as for fresh cells.

For freshly harvested cells: Cells were crosslinked in 0.1% formaldehyde (Merck Millipore, #F8775) for 5 min and quenched with glycine for additional 5 min. Cells were washed twice in Dulbecco's phosphate-buffered saline (DPBS, ThermoFisher, #14040133). From this point, all steps took place on ice or 4°C. Cells were resuspended in 50 $\mu$l of cold Buffer D (20 mM Tricine KOH pH 7.8, 0.1% Triton X-100, 25 mM KCI, sucrose 250 mM, 5 mM MgCl$_2$, 1X protease inhibitors cocktail) per sample. If desired, an aliquot of CUT&Tag input can be put aside at this step. The remaining cells were bound to ConA beads for 20 min (10 $\mu$l per sample). Beads were resuspended in 50 $\mu$l of Antibody buffer D (20 mM Tricine KOH pH 7.8, 0.1% Triton X-100, 25 mM KCI, 250 mM sucrose, 150 mM NaCl, 2 mM EDTA, 0.1 % BSA, 1X protease inhibitor cocktail, 0.5 mM spermidine) per sample, distributed into 0.2 ml tube strips and 1 $\mu$g of the primary antibody was added unless stated otherwise. This was incubated overnight at 4°C. The next day, the buffer was removed. Beads were again resuspended in 100 $\mu$l of Antibody buffer D (20 mM Tricine KOH pH 7.8, 0.1% Triton X-100, 25 mM KCI, 250 mM sucrose, 150 mM NaCl, 2 mM EDTA, 0.1 % BSA, 1X protease inhibitor cocktail, 0.5 mM spermidine). 1 $\mu$g of the secondary antibody was added and incubated for 45 min. 100 $\mu$l of a 1:250 pA-Tn5 dilution (Diagenode, #C01070001) in Buffer D300 (20 mM Tricine KOH pH 7.8, 0.1% Triton X-100, 25 mM KCI, 250 mM sucrose, 300 mM NaCl, 1X protease inhibitors cocktail, 0.5 mM spermidine) was added and incubated at 4°C for 1h. Unbound pA-Tn5 was washed away in 200 $\mu$l of the same buffer. Beads were resuspended in 200 $\mu$l of Tagmentation Buffer D300 (20 mM Tricine KOH pH 7.8, 0.1% Triton X-100, 25 mM KCI, 250 mM sucrose, 300 mM NaCl, 15 mM MgCl$_2$, 1X protease inhibitors cocktail, 0.5 mM spermidine) and incubated at 37°C for 1h. Input samples were included at this step. Tagmentation was stopped by adding 0.5 M EDTA, 10% SDS and proteinase K, and the following protein digestion took place at 55°C according to the manufacturer's instructions. A phenol-chloroform extraction with subsequent ethanol precipitation was performed and DNA was dissolved in 10 mM Tris-HCl pH 8 1 mM EDTA. The resulting DNA solution was used in a PCR with NEBNext High-Fidelity 2x PCR master mix (New England Biolabs, #M0541) and barcoded i5 and i7 sequencing primers. Samples were subjected to PCR (Table 9).

*Table 9: PCR protocol for the CUT&Tag protocols disclosed herein.*

| Step | Temperat ure | Duration |
|---|---|---|
| Gap filling | 72°C | 5 min |
| Initial denaturation | 98°C | 30 sec |
| 8 - 18 cycles of: | | |
| | 98°C | 10 sec |
| | 63°C | 10 sec |
| Final elongation | 72°C | 1 min |
| Hold | 4°C | |

An intermediate quantification of the samples was performed using the KAPA Library Quantification kit (see below). After the PCR and a potential re-amplification, samples were purified using AMPure XP beads (Beckman Coulter, #A63880) and resuspended in 10 mM Tris-HCl pH8. An enrichment qPCR was performed for most samples to test enrichment of known loci (see below). Samples were run on Fragment Analyzer using the NGS kit (Agilent Technologies, #DNF-473-0500), pooled in an equimolar ratio and sequenced.

All steps after the mentioned distribution into 0.2 ml tube strips took place in these tubes until a transfer in new 0.2 ml tubes for the PCR step. All wash steps were performed with 0.2 ml of the respective buffer.

Intermediate library quantification in CUT&Tag. To evaluate the success of a CUT&Tag experiment, a library quantification using the kit KAPA Library Quantification Kit (Roche, #07960140001) was performed. An aliquot of 0.5 - 1.0 $\mu$l was taken from the CUT&Tag samples and mixed in a 1:1000 dilution with library dilution buffer (10 mM Tris-HCl, pH 8.0- 8.5, 0.05% Tween$^®$ 20). These dilutions were vortexed at full speed for 10 seconds. A qPCR was conducted using the sample dilutions and DNA standards of known concentrations (20 pM to 0.0002 pM) and the samples' concentration was calculated according to the manufacturer's instructions. The KAPA SYBR$^®$ FAST qPCR Master Mix (2X) contains primers matching to sequencing adapters (included in the kit). Therefore, the qPCR amplifies fragments which contain the Illumina sequencing primers. If necessary, the samples could be re-amplified according to the manufacturer's instructions to reach a sufficient concentration high to fulfil the sequencing requirements, and then purified with AMPure XP beads as described above.

**Table 10: Antibodies and enzymes used in CUT&Tag and ChIP.**

| Antibody name | Supplier and catalogue number | Host | Experiment type |
|---|---|---|---|
| H3K27me3 | Diagenode, #C15410195 | Rabbit | CUT&Tag, ChIP |
| H3K27me3 | Merck Millipore, #07-449 | Rabbit | CUT&Tag |
| CTCF | Diagenode, #C15410210 | Rabbit | CUT&Tag, ChIP |
| SUZ12 | Abcam, #ab12073 | Rabbit | CUT&Tag, ChIP |
| JARID2 | Novus Biologicals, #NB100-2214 | Rabbit | CUT&Tag |
| IgG | Diagenode, #C15410206 | Rabbit | CUT&Tag |
| pA-Tn5 | Diagenode, #C01070001 | N/A | CUT&Tag |
| Proteinase K | ThermoFisher, #AM2546 | N/A | CUT&Tag |

Enrichment qPCR and the used primers: After the CUT&Tag protocol disclosed herein was completed, a qPCR targeting expected loci was performed. For this, 5 μl of 1:10 dilutions of the CUT&Tag samples were mixed with 10 μl of 2x KAPA SYBR FAST (Merck Millipore, #KK4601), 1 μl of the desired primer pair solution containing 5 mM per primer, and 4 μl of ultrapure water. The following PCR program was applied (Table 11):

**Table 11: Enrichment PCR in CUT&Tag protocol disclosed herein**

| Step | Temperature | Duration |
|---|---|---|
| Initial denaturation | 95°C | 5 min |
| 40 cycles of: | | |
| | 95°C | 30 sec |
| | 60°C | 30 sec |
| | 72°C (single acquisition) | 30 sec |
| Final elongation | 72°C | 10 sec |
| Melting curve | Specific for the used qPCR instrument | |

Relative enrichment was calculated using the following formulas:

$$delta\ Ct = Ct\ negative\ locus - Ct\ positive\ locus$$

$$relative\ enrichment = 2(delta\ Ct)$$

Since the exact relative enrichment value is highly dependent on the positive and negative loci examined, CUT&Tag IgG samples were included as a negative control for the enrichment values for each primer pair (Table 12). A value below 3 was considered as background.

**Table 12: List of primer pairs used in CUT& Tag and ChIP**

| Primer name | Primer sequence or supplier |
|---|---|
| Mouse T(Bra)_FW | GCAGCATGCGTTCCAACAAT |
| Mouse T(Bra)_RV | AGCGACCCTAGTAGGAGGTG |
| Mouse Actb_FW | GCCTAGTAACCGAGACATTGA |
| Mouse Actb_RV | AGAAAGCGAGATTGAGGAAG |
| Mouse C-MYC1_FW | ACAAATCCGAGAGCCACAAC |
| Mouse C-MYC1_RV | AACACCAAGAGCCACCAATC |
| Mouse myoglobin exon 2 pair | Diagenode, #C17021010 |
| Mouse TSH2B pair | Diagenode, #C17021042 |
| Mouse GAPDH pair | Diagenode, #C17021 045 |

Preparation of an input sample for input normalization:

50,000 cells per sample were collected, optionally in triplicates, and centrifuged at 500 g for 10 minutes at RT. The supernatant was discarded and cells were resuspended in RT 1X DPBS (100 μL per sample). Samples were spit into 1.5 ml tubes (50,000 each =100 μL) and centrifuged at 600 g for 3 minutes at RT. The supernatant was discarded and cells were resuspended in 100 μL of XL GenDNA Digestion Buffer (Diagenode, # C03030020) supplied with 0.5 proteinase K per sample. The samples were incubated in a thermomixer at 55°C for ½ hour, at 600 rpm. Samples were purified using DiaPure columns (500 μl Binding buffer was added per sample). Optionally, between the two wash steps, one step of adding 1 μl of RNAse directly onto the filter for 15 minutes was added. Samples were then eluted in 30 μl of elution buffer. The samples were quantified by Qubit and kept until the tagmentation step at +4°C. Optionally, the samples were further quantified by FA/FEMTO with a gDNA kit. For the tagmentation step, 270 μl of tagmentation buffer with pA-Tn5 diluted 1:100 (calculated as in total volume of 300 μl) per sample was added and incubated in a thermomixer at 37°C for 1 hour, set at 800 rpm. The tagmentation reaction was stopped and the samples were further treated as described for conventional CUT&Tag.

Sequencing of CUT&Tag samples. CUT&Tag samples were sequenced in 2x50 bp on an Illumina NovaSeq 6000 flow cell, running NovaSeq Control Software 1.7.5, RTA v3.4.4 and bcl2fastq 2.20 v2.20.0.422.

Bioinformatics analysis of CUT&Tag: Quality control of sequencing reads was performed using FastQC. Trimming of the adaptors was performed with cutadapt. Trimmed reads were aligned to the reference genome (mm10) obtained from the UCSC genome browser using BWA software v.0.7.5a. Samples were filtered for regions blacklisted by the ENCODE project. Multimapping reads were removed using samtools (Li, H., Handsaker, B., Wysoker, A., Fennell, T., Ruan, J., Homer, N., Marth, G., Abecasis, G., Durbin, R., 1000 Genome Project Data Processing Subgroup, 2009. The Sequence Alignment/Map format and SAMtools. Bioinformatics 25, 2078-2079. https://doi.org/10.1093/bioinformatics/btp352) while PCR duplicates were kept. Alignment coordinates were converted to BED format using BEDTools v.2.17. Peak calling was performed using MACS2 without the broad option, with a q-value of $1e^{-05}$. Differential binding analysis was performed with the R/Bioconductor package: DiffBind. Heatmaps around TSS were generated using computeMatrix functions from deepTools using the bigwig files. For the generating of screenshots of genome landscapes, the USCS genome browser and the IGV server have been used.

## Results

### a) Detection of SUZ12, CTCF and H3K27me3 using 50,000 cells

The CUT&Tag protocol as described herein was performed using two different antibodies against H3K27me3 (Diagenode, #C15410195; Merck Millipore, #07-449), CTCF, SUZ12 and IgG in fresh WT mESCs and Suz12$^{-/-}$ mESCs, each in duplicates, except CTCF and IgG. One input sample was processed per cell model. 50,000 freshly harvested cells were used per reaction. To quantify the Tn5 bias, a NOCOA (peaks not overlapping with ChIP, but overlapping with ATAC-seq) pipeline described by Susami et al., 2022 (Susami, K., Ikeda, S., Hoshino, Y., Honda, S., Minami, N., 2022. Genome-wide profiling of histone H3K4me3 and H3K27me3 modifications in individual blastocysts by CUT&Tag without a solid support (NON-TiE-UP CUT&Tag). Sci. Rep. 12, 11727), although here, only peaks in common among replicates were used in the analysis.

The preparation of sequencing libraries was successful for all samples, as shown by the library concentration values obtained (Figure 16A). Samples were re-amplified if necessary and purified.

Using qPCR, enrichment at target loci over negative loci was observed for all samples in SUZ12 WT cells (Figure 16B). As expected, no enrichment at these loci was observed in Suz12$^{-/-}$ cells, with the exception of CTCF, which was used as a SUZ12 KO-independent control.

Differential binding analysis using DiffBind was performed for all four H3K27me3 CUT&Tag samples. Without normalization, the pairwise correlation of the samples based on binding site occupancy between the H3K27me3 CUT&Tag samples of the two antibodies used against H3K27me3 was approximately 0.5 (Figure 17). There was one replicate with a lower correlation of 0.3. Normalization with IgG or input increased the correlation to 0.7 for most replicates.

Moreover, differential binding analysis was performed for the two SUZ12 CUT&Tag samples using 50,000 WT mESCs. A high correlation value of 0.8 regardless of normalization was observed (Figure 18), indicating good reproducibility.

The removal of peaks following normalisation with input or IgG can be visualised in genome tracks at the example of H3K27me3 CUT&Tag samples in WT and *Suz12*$^{-/-}$ cells (Figure 19). Without normalization, the signal at the HOXA cluster in Suz12$^{-/-}$ cells was still considered a peak, while the signal did not exceed the peak calling threshold after normalization with IgG or input. In contrast, the signal in SUZ12 WT cells was strong enough to persist normalization. Finally, as in the previous experiment, CTCF showed a similar signal in WT and Suz12$^{-/-}$ cells (Figure 19).

To quantify the Tn5 bias known from the previous experiment and to assess the influence of countermeasures, a pipeline was developed as described in Susami et al., 2022 (Susami, K., Ikeda, S., Hoshino, Y., Honda, S., Minami, N., 2022. Genome-wide profiling of histone H3K4me3 and H3K27me3 modifications in individual blastocysts by CUT&Tag without a solid support (NON-TiE-UP CUT&Tag). Sci. Rep. 12, 11727). The authors take the number of peaks not overlapping with reference ChIP-seq peaks, but that overlap with ATAC-seq peaks. A false positive rate is calculated by dividing this number by the total number of peaks. With this technique, (Susami et al., 2022) achieve a false positive rate between 12% and 25% for their CUT&Tag protocol on H3K27me3.

A similar approach was chosen here. In contrast to the disclosure of Susami et al., 2022, common peaks between replicates were identified first and then the peaks that did not overlap with the SUZ12 ChIP-seq reference data were identified. Of the resulting set of peaks, the peaks that overlapped with ATAC-seq were identified and divided by the total number of peaks. Interestingly, normalization with IgG or input reduced the number of peaks that did not overlap with SUZ12 ChIP-seq (NOC) from 54% to 21% and 15%, respectively. Of these, peaks overlapping with ATAC-seq (NOCOA) were reduced from 19% to 5% with both IgG and input normalization (Figure 20). Since the normalization effect is similar between IgG and input, the following analyses are shown only using IgG normalization.

Since normalization with IgG and input had a similar effect, the following analysis was performed with IgG normalization only.

Genome tracks at the HOXA cluster revealed successful detection of SUZ12 and H3K27me3 independent of the antibody used (Figure 21). All samples in WT mESC cells showed the expected pattern at the HOXA cluster. The signal of SUZ12 samples obtained using CUT&Tag as described herein were even twice as high as for the ChIP reference data. As expected, none of the SUZ12 KO samples showed enrichment and thus, no peak. Furthermore, H3K27me3 and SUZ12 peaks obtained for wt mESCs were plotted on their respective set of target genes (Figure 22A). In all cases, the KO signal was found to be similar to the IgG signal. Both antibodies against H3K27me3 showed similar signal intensity in WT cells. Taken together, this indicates a strong positive signal in WT samples and mere background signal in KO samples at these sites. In addition, a strong overlap between the peaks of the H3K27me3 repeats was obtained with Merck Millipore's antibody and normalized with IgG (Figure 22B). Boxplots of the peaks' sites shows that the signal at the common, but also at the specific sites is of a similar intensity (Figure 22C). Thus, potential peaks were not called due to a mere threshold setting and are likely to be true common peaks, which in turn indicates high reproducibility. Approximately 5,000 shared H3K27me3 peaks between WT and Suz12$^{-/-}$ cells were reported in non-normalized datasets (Figure 23A). This number decreased to 40 after normalization with IgG. In addition, upon normalization with IgG the total number of peaks in Suz12$^{-/-}$ cells decreased from almost 9,000 to 331, compared to 4,000 peaks detected in IgG-normalized WT cells. In contrast to the previous experiment, none of the sites of IgG-normalized peaks showed an increased ATAC-seq signal (Figure 23B). The same trend was observed for the detected gene sets (Figure 23C). It can be concluded that the normalization successfully filters out the non-specific peaks of the Tn5 bias, while the antibody-specific signal remains.

However, when IgG normalization was applied to the H3K27me3 CUT&Tag samples of Example 3, the number of detected peaks and genes in WT cells dropped to 853 and 708, respectively, while a large number of peaks (18,800) and genes (2,000) were detected in the KO samples (Figure 23D). This highlights that while the protocol of Example 3 resulted in the detection of H3K27me3 targets, it also produced false positive results and normalization could not compensate for the relatively low data quality. One explanation for the relatively low data quality could be the addition of MgCl$_2$ prior to the tagmentation step, which was carried out in Example 3 in deviation from the conventional CUT&Tag protocols. In contrast, the data quality could be significantly improved by normalization when using the experimental design and protocol described herein in which MgCl$_2$ was not added prior to the tagmentation step.

**Conclusions:**

To conclude, the CUT&Tag protocol disclosed herein reliably detected SUZ12 and H3K27me3 target sites when using 50,000 mESCs. Replicates were similar to each other, indicating a high reproducibility. *De novo* peaks in KO cells, many unexpected peaks in open chromatin for WT and KO cells and hence a large overlap between WT and KO cells, were not observed.

Normalization using the input protocol disclosed herein and IgG was systemically validated. Nonspecific peaks were removed by normalization, while peaks in target regions remained. No ATAC-seq signal was observed at the sites of IgG-normalized peaks or other evidence of transcriptionally active chromatin. Thus, the problem of false-positive peaks due to a Tn5 bias was solved.

Applying the normalization strategy to the data set obtained in Example 3, it became clear that in addition to the successful detection of true peaks and promising signals in some target genes in WT mESCs, a high number of false-positive peaks were also detected.

In summary, a robust CUT&Tag protocol has been developed that is suitable for detecting non-histone proteins, such as the PRC2 core protein SUZ12, when using cell numbers of 50,000 cells

**b) Detection of SUZ12, JARID2 and H3K27me3 using 5,000 cells**

**Results**

The protocol disclosed herein was further applied to determine binding of SUZ12, JARID2 and H3K27me3 in 5,000 mESCs. mESCs were harvested, cross-linked with 0.1% formaldehyde, permeabilized with Antibody buffer D and bound to ConA beads. 0.5 μg of antibodies against H3K27me3, SUZ12, JARID2 or IgG were used per reaction.

Despite the low cell number of 5,000 cells, high library concentrations (Figure 25A) and enrichment at the target loci were achieved (Figure 25B).

When looking at genome tracks (Figure 26), the signal of the CUT&Tag samples on H3K27me3, SUZ12 and JARID2 on 5,000 mESCs was very similar to ChIP-seq data for H3K27me3 and SUZ12 in mESCs.

Peak calling was performed using MACS with default parameters and broad option. Despite obvious enrichment, peak calling using MACS had issues to recognize peaks and thus further optimization is recommended. Nevertheless, the peaks that were called, matched target genes of PRC2 proteins and H3K27me3 when functional analysis using Enrichr was performed (Figure 27).

**Conclusions**

Thus, the CUT&Tag protocol disclosed herein showed a high reproducibility in a large number of loci for all samples when using low cell number of 5,000 mESCs. Peak calling may be further optimized as described in Example 6 below.

Example 6: Peak callers for analysing CUT&Tag

**Introduction**

The most commonly used peak callers for chromatin profiling are MACS2 and epic2. However, these were developed and optimized for ChIP-seq. While ChIP-seq and CUT&Tag are similar in their basic principles, the differences in sample preparation and fragmentation mechanisms may require different approaches for peak calling. ChIP-seq often results in high background levels caused by cross-linking and shearing as well as high sequencing depth. Peak callers developed for ChIP-seq often rely on background models to account for these high background levels and detect antibody-specific enrichment against this background. In contrast, the chromatin fragments excised by Tn5 transposase result in narrower, more distinct peaks and a much lower background signal. The use of standard peak calling in CUT&Tag may therefore be overly sensitive to the small amounts of background signal and lead to the invocation of false positive peaks. As shown in Example 5, the use of standard peak callers suitable for ChIP-seq, such as MACS, may not always be the best choice for CUT&Tag. Therefore, the use of special peak callers and settings further improves the analysis of CUT&Tag data.

The Sparse Enrichment Analysis for CUT&RUN (SEACR) was developed for CUT&RUN and uses the genome-wide background noise as calibration. This enables accurate differentiation between true and false-positive peaks. GoPeaks was developed for CUT&Tag and was suggested to improve peak detection in variable peak profiles compared to GoPeaks, especially when narrow peaks, as observed for H3K4me3 and transcription factors, are used as input data. In addition, the CUT&RUNTools2.0 were developed to analyze CUT&RUN and CUT&Tag data at the single cell and bulk level using an end-to-end pipeline with MACS2 for the peak calling step.

In this example, the use of different peak callers and settings for analyzing data generated with the CUT&Tag protocol disclosed herein will be compared.

**Results**

CUT&Tag samples obtained for H3K27me3, or IgG as control, in mES WT cells and in *Suz12*[-/-] cells using the CUT&Tag protocol disclosed herein were analyzed using to different peak callers and settings without or upon normalization using IgG (Table 13). For GoPeaks, all results shown used IgG normalization as this is required for this peak caller.

*Table 13: CUT&Tag samples used in different peak calling algorithms and settings.*

| Cell line | Antibody + replicate | Signal expected? |
|---|---|---|
| mESC WT | H3K27me3 A | Yes |
| mESC WT | H3K27me3 B | Yes |
| mESC *SUZ12*[-/-] | H3K27me3 A | No |
| mESC *SUZ12*[-/-] | H3K27me3 B | No |
| mESCWT | IgG A | No |

(continued)

| Cell line | Antibody + replicate | Signal expected? |
|---|---|---|
| mESC *SUZ12*$^{-/-}$ | IgG B | No |

Without normalization, a considerable number of peaks was observed when SEACR was applied using relaxed settings and when applying CUT&RUNTools2.0, which uses MACS2 (Figure 28). As expected, with IgG normalization, the number of peaks decreased for all peak callers. Previous results of example x have shown that the peaks removed by IgG normalization are mainly non-specific peaks. Therefore, a reduction in the number of peaks after normalization does not mean that true peaks are lost. This effect was particularly strong when the relaxed settings of the SEACR algorithm were used. In addition, a considerable number of peaks were called in Suz12$^{-/-}$ cells, with the lowest number of peaks in SUZ12$^{-/-}$ cells (900 - 1,400) being achieved with SEACR regardless of the relaxed or strict settings.

However, the proportion of common peaks between the H3K27me3 replicates in mES-WT cells was surprisingly low regardless of normalization (Table 14). With IgG normalization, epic2 showed the highest number of shared peaks with 88.40 %, followed by GoPeaks with 48.95 %. With the exception of epic2, these numbers are too low for peak calling to be used reliably.

**Table 14: Proportion of shared peaks between H3K27me3 replicates in WT mESCs with or without IgG normalization.**

| Peak caller | Without IgG normalisation | With IgG normalisation |
|---|---|---|
| epic2 | 98.69 | 88.40 |
| SEACR_relaxed | 12.68 | 0.32 |
| SEACR_stringent | 0.35 | 0.32 |
| GoPeaks_default | N/A | 30.13 |
| GoPeaks_broad | N/A | 48.95 |
| CUT&RUNTools2.0 | 38.24 | 35.19 |

Next, the proportion of NOCOA peaks (CUT&Tag peaks that do not overlap with ChIP-seq peaks but overlap with ATAC-seq peaks) was estimated for all peak callers and conditions. Given the low proportion of common peaks between H3K27me3 replicates for most peak callers except epic2, the proportion of NOCOA peaks was estimated at the individual sample level. Again, normalization improved peak calling by reducing the proportion of NOCOA (Figure 29). It should be noted that the NOCOA fraction of SUZ12 using IgG normalization and epic2 was 5 %. SEACR irrespective of applied settings and GoPeaks at default settings achieved the lowest NOCOA levels for H3K27me3 in mES-WT cells (at around 1.4 - 2 %). CUT&RUNTools2.0 achieved 2.4%.

The low level of common peaks between the replicates for all peak callers except epic2 and the high NOCOA rate for epic2 showed that there is still a need for further optimization of the peak calling parameters.

In a further experiment, parameters using MACS2 and epic2 peak callers were aimed to be optimized on MTF2 CUT&Tag samples. For this purpose, mESCs carrying a dTAG construct targeting MTF2 were generated and treated with DMSO, i.e. not resulting in the degradation of MTF2, and dTAG dissolved in DMSO, i.e. resulting in the degradation of MTF2, respectively. CUT&Tag using an antibody against MTF2, as well as IgG as control and fur the purpose of downstream normalization, was performed in DMSO- and dTAG-treated samples (Table 15).

**Table 15: List of CUT&Tag samples used for optimization of MACS2 and epic2 peak calling parameters.**

| Cell line | Antibody and replicate | MTF2 signal expected? |
|---|---|---|
| mESC clone on dTAG | MTF2, A | No |
| mESC clone on dTAG | MTF2, B | No |
| mESC clone on DMSO | MTF2, A | Yes |
| mESC clone on DMSO | MTF2, B | Yes |
| mESC clone on dTAG | IgG, A | No |
| mESC clone on dTAG | IgG, B | No |
| mESC clone on DMSO | IgG, A | No |
| mESC clone on DMSO | IgG, B | No |

First, default settings of epic2 and MACS2 (Table 16) were tested for enrichment at the HOXA cluster and *Alx1,* either using IgG samples for normalization during peak calling or not (Figure 30).

*Table 16 Default settings of epic2 and MACS2 as applied for the analysis of CUT&Tag MTF2 samples.*

|  | epic2 | MACS2 |
|---|---|---|
| Window size | 5,000 | -- broad |
| Gap | 6 | -- broad-cutoff: 0.01 (q-value) |
| e-value | 1,000 |  |

Optimal peak calling parameters are expected to detect only a few peaks in IgG and MTF2 KO samples, but be able to detect true MTF2 peaks in DMSO-treated control cells that resemble WT conditions. The first test was performed with the default settings of each peak caller (Table 16).

Without normalization, both epic2 and MACS2 default settings identified peaks in the MTF2-KO samples (Figure 30A). In addition, MACS2 called peaks in the IgG condition and epic2 identified regions broader than the enrichment. With IgG normalization, hardly any peaks were identified in the KO samples at the site of narrow enrichment with either peak caller (Figure 30B). However, many peaks were called around the large HOXA cluster. Similarly, as without normalization, peaks called with epic2 were broader than the actual enrichment. Therefore, neither peak caller was suitable for identifying CUT&Tag peaks using default settings.

Next, different epic2 settings were tested. While the number of peaks and the overlap between replicates remained high, the number of peaks identified in the KO samples remained at a similar level to the number of peaks identified for the WT samples for all conditions tested. In general, the number of peaks called with epic2 decreased with increasing gap values, as narrow peaks were increasingly combined into broader peaks. The same trend was observed when the window size was increased. In contrast, decreasing the FOR had little effect on the number of peaks identified. Window sizes of 200, 500, 1,000 and 2,000 resulted in many narrow peaks for both WT and KO cells that did not reflect the signal. At a window size of 5,000 and a gap of 6, the peaks were too broad and caused peak calling in the KO cells. The same window size of 5,000 with a gap of 2 resulted in better defined peaks, while peaks were still detected in the KO cells.

Ultimately, a window size of 5,000 combined with a gap of 1 resulted in the best peak calling when using epic2 at the two loci examined (Figure 31). Nevertheless, there were still peaks called at the larger HOXA cluster in the MTF2-KO cells, likely due to the higher background at this locus. In addition, the peaks were still broader than the region of enrichment.

Various settings for peak calling with MACS2 were also investigated. In example 5, peaks with the broad option and a q-value of 0.01 were called for H3K27me3 CUT&Tag samples. Here the peaks were called without the broad option and with q-values of either 0.01 or 1e-5 with IgG normalization. The number of peaks in the MTF2-KO cells was reduced when a more stringent q value was applied, although this reduction was also observed in the mESC-WT cells (Figure 32A). A considerable number of peaks were observed in KO cells at all settings. This may be attributed to the incomplete degradation of MTF2 upon treatment with dTAG (Figure 32B-C).

Optimized peak calling parameters for MACS2 were further confirmed to result in successful peak calling for H3K27me3 and SUZ12 in WT and *Suz12*$^{-/-}$ cells (Figure 33).

**Conclusion**

Using MACS2 without the broad option, a q-value of 1e-05 and using IgG normalization was identified as a suitable peak calling strategy for data generated with CUT&Tag, providing the best results compared to other peak callers and settings tested. It should be noted that there were still some very narrow peaks detectable in samples on KO cells. If in doubt, the peaks could be filtered according to their width so that the true peaks remain and the very narrow peaks in KO samples are no further taken into account.

A successful peak calling strategy for CUT&Tag targeting different chromatin-associated proteins has been identified.

Example 7

**Introduction**

MLL-AF9 is a fusion protein resulting from a translocation of chromosomes 9 and 11 in humans and 4 and 9 in mice. When expressed in hematopoietic stem and progenitor cells (HSPCs), it can lead to acute myeloid leukemia (AML) in humans and mice. Therefore, MLL-AF9 has been extensively studied in the context of leukemia development in mouse models. Previous research has shown that MLL-AF9 leads to gene activation through the abnormal recruitment of DOT1-like histone H3K79 methyl transferase and SEC. Through interactions with Polycomb complexes, MLL-AF9 causes upregulation of HOXA9 and EZH2 expression, among others, which promotes proliferation while inhibiting differentiation and apoptosis through Cdkn2a repression. While MLL-AF9's activity in hematological differentiation has been described, little is known about its role in embryonic differentiation is.

To this end, using the CUT&Tag protocol disclosed herein, this example aims to investigate the role of the oncogenic fusion protein MLL-AF9 in mESCs during their development to embryonic bodies and the interaction of MLL-AF9 with Polycomb repressive complexes (PRC).

To study MLL-AF9 in embryonic differentiation, the differentiation of embryonic stem cells into embryoid bodies, i.e. three-dimensional structures containing cells from the three germ layers (mesoderm, endoderm and ectoderm), was chosen as a model because it resembles the differentiation of mESC *in vivo.* Differentiation of embryoid bodies allows a comprehensive study of cell differentiation and tissue development and provides insights into the role of relevant factors in differentiation in a controlled *in vitro* environment, thus providing a physiologically relevant model to study MLL-AF9 in embryonic differentiation.

Moreover, combining CUT&Tag with mRNA-seq and ATAC-seq analyses was aimed to gain a comprehensive understanding of the mechanisms involved in EB differentiation in the context of MLL-AF9 expression. The combination of CUT&Tag on PRC proteins with mRNA-seq will help to correlate changes in PRC activity with changes in gene expression and to investigate how MLL-AF9 activity and its interaction with PRC complexes affect both chromatin accessibility (e.g. through chromatin condensation by H3K27me3) and gene expression during EB differentiation. For example, when SUZ12 is lost at a promoter, it is expected to abrogate its repressive function and increase gene expression. Furthermore, the combination of CUT&Tag with ATAC-seq will allow to link these changes to chromatin accessibility. This should lead to a more comprehensive understanding of the consequences of MLL-AF9 activity and may help to identify key genes and pathways affected by MLL-AF9 activity in EB differentiation.

**Materials & Methods**

Culture of mouse embryonic stem cells (mESCs). ES-E14TG2a were cultured in Glasgow's Minimal Essential Medium (GMEM, Gibco, 11710035), supplemented with 20% FBS (ES cell qualified, FisherScientific, #15946942), 1% Glutamax (FisherScientific, #11574466), 1% non-essential amino acids (FisherScientific #12084947), 1 mM sodium pyruvate (FisherScientific, #11530396), 1 mM 2-Mercaptoethanol (ThermoFisher, #21985023), 100 U Penicillin-Streptomycin (ThermoFisher, #15140122), 1,000 U/ml mouse Leukaemia Inhibitory Factor (mLIF, Merck Millipore, #ESG1106), on flasks coated with 0.1% gelatine.

Differentiation from mESC to mEB. Two clones of mESC cells expressing the MLL-AF9 fusion protein (B121C, D1C) and one WT mESC strain first were cultured routinely as described above. The cells were passaged in IMDM medium (still containing mLIF and the other described reagents) in gelatine-coated flasks twice before the start of the differentiation protocol. On day 0, cells were passaged in EB medium of IMDM, supplemented with 15% FBS, 1% GlutaMax, 50 μg/ml ascorbic acid, 0.15 mM MTG and 0.18 mg/ml, on flasks without gelatine coating to induce differentiation. EBs were collected by transferring the cell suspension into 15 ml tubes and letting it sit for 3 min. The supernatant was discarded. The cell pellet was washed with 10 ml PBS. 1 ml trypsin-EDTA was added and incubated at 37°C for 5 min. Cells were aspirated in 9 ml IMDM+10% FBS and released towards the tube wall. Cells were centrifugated at 180 x g for 5 min and the supernatant was discarded. Cells were resuspended in 1 ml IMDM+10%FBS, then additional 4 ml were added. The cells were passed through a cell strainer of 40 μm and counted. Samples for CUT&Tag, ATAC-seq and RNA-seq were taken at day 0 (mESC) and day 5 (mEB).

ATAC-seq. ATAC-seq was performed on samples of mESC and mEB. All cell conditions (Table 17) were processed in duplicates.

*Table 17 List of cell conditions processed with ATAC-seq and RNA-seq*

| List of cell conditions processed with ATAC-seq and RNA-seq |
| --- |
| WT mESC |
| WT mEB |
| MLL-AF9 clone B121C mESC |
| MLL-AF9 clone B121C mEB |
| MLL-AF9 clone D1C mESC |
| MLL-AF9 clone D1C mEB |

ATAC-seq samples were processed using the ATAC-seq kit (Diagenode, #C01080002) and following the manufacturer's instructions. Quality was checked on Fragment Analyzer using the NGS kit (Agilent Technologies, #DNF-473-0500). Sequencing was performed in paired-end 50 bp on an Illumina NovaSeq6000 flow cell. Quality control of sequencing reads was performed using FastQC (Andrews, 2010). Trimming of the adaptors was performed with Trim_Galore!. Trimmed reads were aligned to the reference genome (mm10) obtained from the UCSC genome browser (Rosenbloom et al., 2015) using Bowtie2. Samples were filtered for regions blacklisted by the ENCODE project (Hoffman et al., 2013). PCR duplicates and multimapping reads were removed using samtools (Li et al., 2009). Alignment coordinates were converted to BED format using BEDTools v.2.17 (Quinlan and Hall, 2010) and peak calling was performed using MACS2 (Zang et al., 2009).

RNA-seq. RNA-seq was performed on samples of mESC and mEB. Each cell condition was processed in duplicates. First, total RNA was extracted of the samples using the miRNeasy mini kit (Qiagen, #217004) and following the manufacturer's instructions. Ribosomal RNA has been depleted using the NEBNext rRNA Depletion Kit v2 (New England Biolabs, #E7400). Subsequently, the D-Plex Total RNA-seq Kit (Total RNA library preparation kit for Illumina® sequencing, Diagenode, #C05030031) was used for the library preparation of the isolated RNA molecules. Using this approach, not only mRNA, but also noncoding RNAs are captured. However, for the further processing of the data in the multi-omics analysis, only mRNAs were included. The noncoding RNAs could be processed and integrated in a future project using the MGCount tool (Hita et al., 2022). Sequencing of the samples was performed on an Illumina NovaSeq6000 instrument producing 50 bp paired-end reads running Control Software 1.7.0. The sequenced reads were controlled for quality of sequencing with FastQC (Andrews, 2010) and trimmed using Cutadapt v3.5 (Martin, 2011). Remaining reads were aligned to the reference genome mm10 using the STAR aligner version 2.7.9a (Dobin et al., 2013). Alignments associated with PCR clones were identified and filtered out with a UMI-based deduplication approach. RNA abundance was estimated with MGCount (Hita et al., 2022). Further analysis consider only features with counts per million (CPM) above 5 for at least 6 samples.

Integration of CUT&Tag, ATAC-seq and mRNA-seq. For RNA-seq, solely protein-coding genes were considered for the analysis. Additionally, genes not reaching a counts per million (CPM) expression above 5 for at least 2 samples were filtered out. For CUT&Tag and ATAC-seq, peaks called at least in both replicates of any sample type were considered for the analysis. Subsequently, following DiffBind pipeline, overlapping peaks were recentred at the summit and homogenised to 400 bp window length symmetrically defined around the submit. The number of alignments was then quantified on the recentred peaks for all samples (samples where peak was not called included) resulting into the enrichment matrix. All peaks included in the analysis were annotated with annotatR package to the following database entries: mm10_genes intergenic, mm10_genes_1to5kb, mm10_genes_introns, mm10_genes_exons, mm10_genes_promoters, mm10_enhancers_fantom. Variance-stabilising transformation was applied to all RNA-seq, ATAC-seq and Cut&Tag matrix datasets before any unsupervised analysis, following the DeSeq2 pipeline. To compare the signal measured by the different assays (CUT&Tag, ATAC-seq, RNA- seq), differential analysis results from the different technologies were compared against each other by considering a promoter region as the point of merge, e.g., a gene expression measurement is paired to ATAC-Seq and CUT&Tag peaks overlapping with its promoter, defined as 1000 bp window preceding the gene TSS. ATAC-Seq and CUT&Tag were paired if coinciding in the same promoter. The same analysis was performed for CUT&Tag and ATAC-seq signal co-localisation at enhancers.

*Table 18: Antibodies and enzymes used in CUT&Tag and ChIP*

| Antibody name | Supplier and catalogue number | Host |
|---|---|---|
| H3K27me3 | Diagenode, #C15410195 | Rabbit |
| H2AK119ub | Cell Signaling Technology, #8240 | Rabbit |
| RING1B | MBL, #D139-3 | Mouse |
| SUZ12 | Abcam, #ab12073 | Rabbit |
| JARID2 | Novus Biologicals, #NB100-2214 | Rabbit |
| H3K79me2 | Diagenode, #C15410051 | Rabbit |
| Mel18 / PCGF2 | Santa Cruz, #sc-515329 | Mouse |
| RYBP / DEDAF | Merck Millipore, #AB3637 | Rabbit |
| FLAG (against MLL-AF9) | Merck Millipore, #F1804 | Mouse |
| IgG | Diagenode, #C15410206 | |
| pA-Tn5 | Diagenode, #C01070001 | N/A |

(continued)

| Antibody name | Supplier and catalogue number | Host |
|---|---|---|
| Proteinase K | ThermoFisher, #AM2546 | N/A |

## Results

Three strains of embryonic stem cells, one WT strain and two clones expressing Flag-tagged MLL-AF9, referred to as B121C and D1C, were cultured in ES medium (Figure 34). The FLAG tag allows unambiguous identification of MLL-AF9 using an antibody against the FLAG tag and avoids confusion with endogenous, MLL and AF9 proteins in the cell. Samples for CUT&Tag, mRNA-seq and ATAC-seq were taken before switching to medium that induces differentiation into embryoid bodies, i.e. on day 0. On day 5, the embryoid bodies were harvested and samples were again taken for CUT&Tag, mRNA-seq and ATAC-seq analysis.

The CUT&Tag protocol disclosed in Example 5 was performed using antibodies against different PRC1 and PRC2 proteins as well as against MLL-AF9 itself.

| Antibody / chromatin mark | Function / complex | Note |
|---|---|---|
| H3K27me3 | Result of PRC2 activity | |
| H2AK119ub | Result of PRC1 activity | |
| RING1B | PRC1 core | |
| RYBP (DEDAF) | Part of ncPRC1 | |
| Mel18 (PCGF2) | Part of PRC1.2 | |
| SUZ12 | PRC2 core | |
| H3K79me2 | Result of MLL-AF9 activity | Expected to be deposited at MLL-AF9 target genes (in addition to usual target genes) |
| FLAG | Targets FLAG-tag on MLL-AF9 | Shows where MLL-AF9 binds in the genome (in addition to other effects) |

Using principal component analysis of the CUT&Tag data against PRC proteins RING1B and SUZ12, all WT and MLL-AF9 clones (mESC) displayed a similar pattern at day 0, but a distinct pattern at day 5 (mEB) (Figure 35). This clearly shows that PRC proteins (here investigating RING1B and SUZ12 as examples) were affected by differentiation and expression of MLL-AF9. Interestingly, the two MLL-AF9 clones formed slightly different patterns, indicating that there are some differences between them and compared to WT.

Next, gene expression in mESCs and mEBs was analyzed. For the MLL-AF9 clones, the expression data was shown as a logarithmic ratio of MLL-AF9 clone/WT. Here, a positive value indicates higher expression in the MLL-AF9 clones compared to WT, while a negative value indicates lower expression in the MLL-AF9 clones compared to WT. First, the stem cell markers Nanog, Oct4 and Sox2 were analyzed during differentiation of mESCs (green) to mEBs (red) in wt and MLL-AF9 clones (Figure 36). While WT clones differentiated as expected, showing a decrease in mRNA expression of Nanog, Oct4 and Sox2 (Figure 36A), clones expressing MLL-AF9 had difficulties with the downregulation of these pluripotency markers compared to WT cells (Figure 36B). This is consistent with the known consequences of MLL-AF9 expression in leukemic cells. It was further observed that clones expressing MLL-AF9 express germ layer markers of the mesoderm and endoderm lineages to a lesser extent than WT cells (Figure 37). Surprisingly, this is not true for the ectoderm lineage, where the MLL-AF9 clones in embryonic bodies expressed typical genes associated with ectoderm development, such as Nestin, Pax6 and NeuroD1, to a higher degree than WT cells. It appears that expression of MLL-AF9 induces cells to partially follow the ectoderm lineage, although most stem cell markers indicate that MLL-AF9 impedes proper differentiation. One hypothesis is that MLL-AF9 has some different or additional targets in EBs than in HSPCs. Interestingly, Flk-1, a marker for hematopoietic development also known as Kdr and VEGFR-2, as well as Brachyury and Eomes, were found to be downregulated compared to WT cells.

In embryonic stem cells, both the WT and the two MLL-AF9 clones showed a comparable gene expression profile and similar chromatin accessibility at day 0 of the protocol (Figure 38A and B, left panels). When these cells undergo differentiation into embryoid bodies, the WT cells begin to express lineage-specific genes while reducing the expression of stem ness-related genes, resulting in a different pattern observed in both ATAC-seq and mRNA-seq data (Figure 38A and, right panel). In contrast, the clones expressing the MLL-AF9 fusion protein showed a different pattern during the EB differentiation process that more closely resembles their ESC state in addition to other changes (Figure 38B). The same trend was observed for chromatin accessibility, which is consistent with open chromatin being correlated with gene expression. These results are consistent with the previous observation that differentiation is delayed upon MLL-AF9 expression.

Since the expression of MLL-AF9 caused more changes in the mEBs compared to mESCs, the analysis was further focused on mEBs and the consequences of MLL-AF9 activity in these cells. Looking more closely at examples of differentially expressed genes in the MLL-AF9 clones in mEBs, the excerpt of a heatmap of mRNA expression in mESCs and mEBs in WT and MLL-AF9 clones showed that differentiation is dysregulated in clones expressing MLL-AF9 (Figure 39).

In addition, some of these differentially expressed genes have been linked to carcinogenesis. One example is *Pim2* (upregulated in the MLL-AF9 clones), a proto-oncogene with serine/threonine kinase activity that is involved in cell survival and proliferation and is a known inhibitor of apoptosis (Fox et al., 2003). When dysregulated, proto-oncogenes become oncogenes and can lead to cancer development. It has been described to stimulate growth factor-independent proliferation through the phosphorylation of cell cycle regulators, e.g. CDKN1A and CDKN1B. In addition, PIM2 has been shown to stabilize p21, which despite its inhibitory effect on cell cycle progression has also been shown to inhibit apoptosis, promote proliferation and is upregulated in various cancers (Wang et al., 2010). Another gene found to be upregulated was *Enpp2*. It was shown to be involved in cell proliferation and promotes tumor progression in multiple myeloma (Li et al., 2022). In addition, expression of some developmental genes such as *Hand1*, *Hand2* and *Kdr* was found to be upregulated during differentiation of WT mESCs into EBs, but not (yet) in MLL-AF9 clones. These genes encode proteins involved in angiogenesis and other cardiovascular development (Nakao et al., 2011; Riley et al., 1998; Vazana-Netzarim et al., 2023). *Hoxb6* was downregulated in MLL-AF9 clones and has been described to be downregulated in MLL-rearranged leukaemia (Giampaolo et al., 2002) and colorectal cancer (Mo et al., 2019). Interestingly, *Hand2* has been described to be downregulated upon knockdown of DPF3 (Fu et al., 2018). These results further show that MLL-AF9 clones seem stuck in the ESC stage of differentiation, but have up-regulated genes involved in proliferation which is often an indication for cancer formation.

In AML, MLL-AF9 was shown to cause overexpression of *Hoxa9, Ezh2* and repression of *Cdkn2a,* which in turn was found to lead to inhibition of differentiation and promotion of cell proliferation and self-renewal (Tan et al., 2011; Thiel et al., 2013). Interestingly, the results showed that this is not the case in EB differentiation. *Hoxa9* and *Hoxa10* were repressed in both WT and MLL-AF9 clones in ESC and in EBs (Figure 40A). The counts were too low to perform statistical analysis in a normalised log2fold calculation and were therefore presented as counts. At the same time, *Cdnk2a* was found to be overexpressed in MLL-AF9 clones, particularly in EBs, while *Ezh2* was downregulated compared to WT cells (Figure 40B). This could indicate that the activity of MLL-AF9 in EB differentiation may not the same as in HSPCs. In addition, the cell of origin and/or the exact time that expression is initiated may be relevant. Further experiments are required to fully understand the interaction with HOXA9 and the consequences for *Cdkn2a.*

In addition, heatmaps of CUT&Tag results for PRC2 complexes and their associated histone modifications H3K27me3 or H2AK119ub, as well as H3K79me2 were generated. These provided a comprehensive overview of these chromatin proteins during differentiation of WT mESCs. In addition, the binding of MLL-AF9 was assessed. Similar to the mRNA expression patterns, more differences in binding of PRC2 were observed at the EB stage between clones expressing MLL-AF9 and WT than in mSCSs.

For these heatmaps, the top enrichments were chosen and there was a large proportion of high H3K27me3 peaks. The green circled region indicates some interesting changes in MLL-AF9 clones:

- Binding of RYBP and RING1B, i.e. ncPRC1, was gained are several sites
- H2AK119ub was found to be a little more enriched
- Mel18, despite having issues in one replicate, was found to be slightly more enriched
- A large region enriched for H3K27me3 in WT is not enriched in the MLL-AF9 clones and resembled more the ESC state.
- Both SUZ12 depleted and slightly enriched sites observed in WT resembled more ESC state in MLL-AF9 clones
- MLL-AF9 (FLAG) binding was increased in EBs
- Some other sites gained binding of RING1B in addition to the already existing SUZ12 in EBs

CUT&Tag peaks falling within promoters of genes were further integrated with the expression data of these genes. It was found that binding of MLL-AF9 to a promoter does not often lead to direct gene activation in mEBs (Figure 42).

One of the MLL-AF9-bound genes that was less expressed in both MLL-AF9 clones was *Cdh4.* Cadherin-4 (also known as R-cadherin) is a cell adhesion protein of the cadherin protein family. While cadherin-1 (E-cadherin) and cadherin-2 (N-cadherin) from the same family are known for their role in epithelial-mesenchymal transition (EMT), a normal developmental process that, when disrupted, is critical for metastasis (Loh et al., 2019), cadherin-4 is less well known. It was shown to be downregulated in various tumor types such as breast cancer cell lines and invasive ductal carcinomas (Agiostratidou et al., 2009), but has not been described in the context of MLL-AF9-driven leukemias or other types of leukemia. It has been ascribed a tumor-repressive function, and downregulation was found to contribute to epithelial suppression and progression to metastasis. The downregulation of *Cdh4* observed here raises the question of its contribution to pathogenesis associated with the expression of MLL-AF9 in the form of increased proliferation and reduced differentiation.

Interestingly, SUZ12 binding to *Cdh4* was more enriched in MLL-AF9 clones than in WT (Figure 42C, D). One hypothesis is that MLL-AF9 recruits SUZ12 to the promoter of *Cdh4* and both bind, causing a decrease in *Cdh4 expression.* An alternative explanation is that SUZ12 remains bound to the *Cdh4* promoter during EB differentiation with MLL-AF9 preventing SUZ12 from detaching.

CUT&Tag peaks falling within promoters of genes were further integrated with the expression data of these genes and their ATAC-seq signal in mEBs.

A general, natural trend was observed: In the upper half (i.e. with an increased ATAC-seq signal, i.e. higher chromatin accessibility), genes tend to be associated with increased expression and vice versa. Interestingly, some genes were found to have lower chromatin accessibility but were still more highly expressed than the WT and *vice versa.*

Binding of RYBP was gained at the *Cacnalc* gene. Furthermore, it was found to be associated with a more condensed chromatin and a lower expression according to ATAC-seq and mRNA-seq data (Figure 43A). Cacna1c is known to be part of the voltage-gated calcium channel regulating blood pressure (Fu et al., 2011; Moosmang, 2003). Furthermore, binding of RYBP and RING1B was gained at *Zfp442,* a gene encoding for a zinc finger protein with unknown function. As most zinc finger proteins comprise a DNA binding capacity, such activity is also expected for ZFP442. Despite increased ncPRC1 binding and a higher chromatin accessibility, its expression was not affected (Figure 43A, B). Moreover, binding of RYBP, RING1B and MEL18 was lost from *Esrrb's* promoter which, which was found to be increasingly expressed in the B121C clone (Figure 43A, B, C). *Esrrb* has been reported to be a transcriptional regulator involved in self-renewal of embryonic stem cells by activating OCT4 (Percharde et al., 2012; X. Zhang et al., 2008). Together with KLF4, it was reported as marker in colorectal cancer cells (Cho et al., 2023). Further, binding of RYBP and MEL18 but apparently not RING1B, was lost from *Glis3* whose expression was found to be increased (Figure 43A, B, C). It was reported to be overexpressed in several cancers, promoting proliferation (Zhang et al., 2023). Finally, binding of RYBP, RING1B and MEL18 was lost from *Bcas3* in the MLL-AF9 clone B121C, but less in the MLL-AF9 clone D1C (Figure 43A, B, C). In both clones, the PRC1 loss at *Bcas3* was associated with a higher expression as expected, but surprisingly with a more condensed chromatin. This protein has been shown to be strongly expressed in blood vessels (Siva and Inamdar, 2006) and overexpressed in breast cancer (Corrêa et al., 2017).

All this leads to the observation that MLL-AF9 induces PRC1 to regulate genes towards proliferation. The changes in PRC1 are reflected in the deposition of H2AK119ub, as it shows a similar set of genes that have been upregulated and downregulated (Figure 43D).

Binding of SUZ12 was observed to be gained and lost at similar genes as PRC1 proteins (Figure 44A). These include *Glis3* in D1C clone and *Bcas3* in most samples as well as *Zfp442, Cacna1c,* but not *Esrrb.*

In addition, binding of SUZ12 and H3K27me3 were gained at *Bnip3l* whose gene expression and chromatin accessibility were both found to be decreased. *Bnip3l* has been reported as a mitochondrial protein which can induce apoptosis. RYBP was also gained at this site, but not RING1B or Mel18. A role of mitochondrial apoptosis-related genes in the development of AML and its pathogenesis has been reported (Meng et al., 2023). These results indicate how MLL-AF9 inhibits apoptosis.

The binding of SUZ12 and H3K27me3 was enriched at *Hdac4*, which encodes histone deacetylase 4. mRNA-seq and ATAC-seq data showed that the expression of *Hdac4* was decreased and the surrounding chromatin was more condensed, most likely due to enhanced binding of SUZ12 and H3K27me3.

## Conclusions

Principle component analysis of RING1B and SUZ12 CUT&Tag patterns allowed distinguishing cell lines and differentiation state. Moreover, expressing MLL-AF9 during EB differentiation was found to cause a disruption in the expression of pluripotency and germ layer genes.

The newly developed and validated CUT&Tag protocol was applied to several PCR1/2 proteins, the oncofusion protein MLL-AF9 and associated histone modifications during the differentiation of mouse embryonic stem cells into embryoid bodies. This was combined with mRNA-seq and ATAC-seq to gain a more comprehensive understanding of the changes in the cells. The antibodies used in CUT&Tag performed surprisingly well. The antibody against Mel18 proved to be less reproducible, as some differences were visible between replicates in most experiments. Furthermore, pA-Tn5 could also be used for monoclonal antibodies raised in mice, as shown by the good results of the RING1B antibody.

A few deregulated genes were identified, as *Cdh4, Pim2, Ksr1.* Some had already been associated with leukemia and some with other cancer types, many were associated with regulating proliferation and differentiation.

Not many mechanisms of MLL-AF9-mediated recruitment are known. In mouse HSPCs, MLL-AF9 was reported to recruit DOT1L to a set of target genes where DOT1L deposits H3K79me2, thereby activating these target genes (Ottersbach et al., 2018). Consistent with this mechanism, H3K79me2 was found on several upregulated genes. On the other side, the downregulated genes found in this project could be the result of an MLL-AF9-directed recruitment of PRC1 and PRC2 as previously described in HSCPs and/or the result of other MLL-AF9-induced changes in the cells. For example, PRC2 was found to bind *Hdac4*, whose resulting repression can cause inactivation of its target genes.

Here, binding of MLL-AF9 and binding of some PRC1/2 proteins were associated with deregulation of some genes that are likely the driving force for inhibiting differentiation into embryoid bodies and promoting proliferation, possibly leading to transformation into cancer cells when they are differentiated for a longer period of time.

## Claims

1.  A method for determining at least one chromatin binding site of a chromatin-associated factor of interest in a cell, comprising:

    (a) permeabilizing the cell;
    (b) contacting the permeabilized cell with a first antibody that specially binds the chromatin-associated factor of interest and with a second antibody that specifically binds to the first antibody;
    (c) contacting the permeabilized cell with a transposome that is linked to a specific binding agent that specifically binds the first and/or second antibody wherein the transposome comprises a transposase and a first and second DNA molecule;
    (d) activating the transposase, thereby excising a DNA segment comprising at least one chromatin binding site of a chromatin-associated factor of interest and tagging the DNA with the first and second DNA molecule;
    (e) determining the sequence of the excised and tagged DNA segment; and
    (f) based on the determined sequence of the excised and tagged DNA segment, determining the at least one chromatin binding site of the chromatin-associated factor of interest in the cell;

    wherein the steps of contacting the permeabilized cell with the transposome and activating the transposase are performed in presence a crowding agent.

2.  A method for determining at least one chromatin binding site of a chromatin-associated factor of interest in a cell, comprising the following steps:

    (a) permeabilizing the cell in a first replicate;
    (b) purifying DNA from the cell in a second replicate;
    (c) contacting the permeabilized cell in the first replicate with a first antibody that specially binds the chromatin-associated factor of interest and with a second antibody that specifically binds to the first antibody;
    (d) contacting both the permeabilized cell in the first replicate and the purified DNA in the second replicate with a transposome that is linked to a specific binding agent that specifically binds the first and/or second antibody wherein the transposome comprises a transposase and a first and second DNA molecule;
    (e) activating the transposase in both the first and second replicate, thereby excising a DNA segment comprising at least one chromatin binding site of a chromatin-associated factor of interest and tagging the DNA with the first and second DNA molecule;
    (f) determining the sequence of the excised and tagged DNA segment in both the first and second replicate; and
    (g) based on the determined sequence of the excised and tagged DNA segment in the first replicate, determining the at least one chromatin binding site of the chromatin-associated factor of interest in the cell, wherein the sequence of the excised and tagged DNA segment determined in the second replicate is used as a normalization control.

3.  The method of claim 2, wherein the steps of contacting both the permeabilized cell in the first replicate and the purified DNA in the second replicate with the transposome and activating the transposase in both the first and second replicate are performed in presence a crowding agent.

4.  The method of any claims 1 to 3, wherein the steps of contacting the permeabilized cell with the transposome and activating the transposase are performed in presence of a Good's buffer.

5.  The method of claim 4, wherein the Good's buffer is selected from Tricine, MES, ADA, PIPEs, ACES, MPOSO, Cholamine chloride, MOPS, BES, TES, DIPSO, TASO, Acetamidoglycine, POPSO, Acetamidoglycine, POPSO, HEPPSO, HEPPS, Tris, Glycinamide, Glycylglycine, Bicine and TAPS, optionally wherein the Good's buffer is Tricine.

6. The method of any of the preceding claims, wherein the steps of contacting the permeabilized cell with the transposome and activating the transposase are performed at a salt concentration of $\leq 300$ mM NaCl.

7. The method of any of the preceding claims, wherein prior to the permeabilization step the cells are subjected to mild cross-linking conditions, optionally wherein the mild cross-linking conditions are 0.1 % formaldehyde for $\leq 15$ min, $\leq 10$ min or $\leq 5$ min.

8. The method of any of the preceding claims, wherein $MgCl_2$ is not added prior to activating the transposase.

9. The method of any of the preceding claims, wherein the chromatin-associated factor of interest is a non-histone protein, optionally a transcription factor or a histone reader protein.

10. The method of any of the preceding claims, wherein the number of cells is $\leq 100,000$, optionally $\leq 50,000$, $\leq 10,000$, $\leq 5,000$ or $\leq 1,000$.

11. The method of any of the preceding claims, wherein the cell is permeabilized using Triton-X-100, optionally at a concentration of 0.1%.

12. The method of any of the preceding claims, wherein the crowding agent is selected from a group comprising sucrose, hexylene glycol and glycerol, optionally wherein the crowding agent is sucrose.

13. The method of any of the preceding claims, wherein prior to the permeabilization step, the cells are bound to beads, optionally magnetic beads.

14. The method of any of the preceding claims, wherein all method steps are performed using total buffer volumes of $\leq 200$ $\mu$l.

15. The method of any of the preceding claims, wherein the amount of transposase that is linked to a specific binding agent is $\leq 1: 250$ of the reaction volume.

16. The method of any of the preceding claims, wherein the amount of first and/or second antibody per reaction is $\leq 1$ $\mu$g.

17. The method of claim 1 or any claims 4 to 16, comprising performing steps (a) to (f) in a first replicate and the following steps in a second replicate:

    - purifying DNA from the cell,
    - contacting the purified DNA with the transposome;
    - activating the transposase;
    - determining the sequence of the excised and tagged DNA in the second replicate;

    wherein the sequence of the excised and tagged DNA segment determined in the second replicate is used as a normalization control in the step of determining the at least one chromatin binding site of the chromatin-associated factor of interest in the cell determined in step (f) in the first replicate.

18. The method of claim 1 or any claims 4 to 16, wherein for a first portion of the cell population an antibody targeting the chromatin-associated factor of interest is used as first antibody and for a second portion of the cell population IgG is used as first antibody, and wherein the sequence of the excised DNA determined for the portion of the cell population using IgG as first antibody is used for normalization in the step of determining the sequence of the excised DNA for the portion of cell population using the antibody targeting the chromatin-associated factor of interest as first antibody.

19. A method for assessing the quality of the method of any of the preceding claims by determining the percentage of false positives resulting from off-target transposase activity, comprising

    (i) conducting steps (a) to (f) of the method of claim 1 or steps (a) to (g) of the method of claim 2 for determining at least one chromatin binding site of the chromatin-associated factor of interest at least twice,

(ii) identifying the chromatin binding sites determined in each step (f) of the method of claim 1 or in each step (g) of the method of claim 2 that overlap;

(iii) identifying a subset of the chromatin binding sites identified in step (ii) that does not overlap with a reference set of chromatin binding sites determined for the chromatin-associated factor of interest identified using ChIP-seq,

(iv) identifying a subset of the subset of chromatin binding sites identified in step (iii) that overlaps with a reference set of chromatin sites identified using ATAC-seq; and

(v) determining the number of chromatin binding sites identified in step (iv) and dividing said number with the number of chromatin binding sites identified in step (ii) in order to obtain the percentage of false positives resulting from off-target activity of the transposase.

20. The method of claim 19, wherein the at least one chromatin binding site is present in closed chromatin.

21. The method of claim 19 or claim 20, wherein the first antibody is an antibody against H3K27me3 or H3K9me3.

22. Method for generating a normalization control suitable for use in the method of claim 1 or any of claims 4 to 18, comprising the following steps:

- purifying DNA from the cell;
- contacting the purified DNA with a transposome that is linked to a specific binding agent suitable for binding a first and/or second antibody wherein the transposome comprises a transposase and a first and second DNA molecule;
- activating the transposase; and
- determining the sequence of the excised and tagged DNA.

Fig. 1

Fig. 2

Fig. 3

EP 4 613 871 A1

# Fig. 4

# Fig. 5

# Fig. 6

# Fig. 7

EP 4 613 871 A1

Fig. 8

Fig. 9

EP 4 613 871 A1

# Fig. 10

Fig. 11

Fig. 12

Fig. 13A

## Fig. 13B

EP 4 613 871 A1

# Fig. 13C,D

C

D

# Fig. 14

A  SUZ12 WT A          SUZ12 KO A

6,643      3,325    1,766

B  Suz12 levels in the overlapping peak sets

C  6,643 WT only

REGIONS
PROMOTER (63.6%)
GENIC (18.6%)
INTERGENIC (17.8%)

3,325 common

REGIONS
PROMOTER (78.3%)
GENIC (9.3%)
INTERGENIC (12.3%)

1,766 KO only

REGIONS
PROMOTER (45%)
GENIC (26.5%)
INTERGENIC (28.5%)

EP 4 613 871 A1

# Fig. 15

A

SUZ12 WT A          SUZ12 KO A

5,180    3,816    1,058

B

WT-only          Common genes          KO-only

C

D

EP 4 613 871 A1

Fig. 16

# Fig. 17

Fig. 18

Fig. 19

# Fig. 20

# Fig. 21

EP 4 613 871 A1

# Fig. 22A

# Fig. 22B, C

Fig. 23

Fig. 24

Fig. 25

Fig. 26

A                                          B

EP 4 613 871 A1

# Fig. 27

# Fig. 28

# Fig. 29

# Fig. 30

A

B

Fig. 31

Fig. 32

Fig. 33

Fig. 34

Fig. 35

Fig. 36

# Fig. 37

EP 4 613 871 A1

Fig. 38A

# Fig. 38B

EP 4 613 871 A1

Fig. 39

Fig. 40

Fig. 41A

Fig. 41B

Fig. 42

EP 4 613 871 A1

# Fig. 43A

# Fig. 43B

RING1B_B121C_EB_vs_WT_EB

RNA log2 Fold-Change   -3 -2 -1 0 1 2 3

RING1B_D1C_EB_vs_WT_EB

RNA log2 Fold-Change   -3 -2 -1 0 1 2 3

EP 4 613 871 A1

Fig. 43C

Fig. 43D

# Fig. 44A

SUZ12_B121C_EB_vs_WT_EB

SUZ12_D1C_EB_vs_WT_EB

# Fig. 44B

H3K27me3_B121C_EB_vs_WT_EB

H3K27me3_D1C_EB_vs_WT_EB

EP 4 613 871 A1

102

Fig. 45

Europäisches
Patentamt

European
Patent Office

Office européen
des brevets

**EUROPEAN SEARCH REPORT**

**Application Number**

EP 24 17 7784

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | HATICE S. KAYA-OKUR ET AL: "CUT&Tag for efficient epigenomic profiling of small samples and single cells", NATURE COMMUNICATIONS, vol. 10, no. 1, 29 April 2019 (2019-04-29), XP055719803, DOI: 10.1038/s41467-019-09982-5 * the whole document * | 1-22 | INV. C12Q1/6804 |
| X | Kaya-Okur Hatice S. ET AL: "Bench Top CUT&Tag V.3", , 15 February 2020 (2020-02-15), XP093199615, DOI: 10.17504/protocols.io.bcuhiwt6 Retrieved from the Internet: URL:https://www.protocols.io/view/bench-top-cut-amp-tag-kqdg34qdpl25/v3?version_warning=no [retrieved on 2024-08-28] * the whole document * | 1-22 | |
| X | EP 4 215 618 A1 (BGI SHENZHEN [CN]) 26 July 2023 (2023-07-26) * claims 1-31 * | 1-22 | TECHNICAL FIELDS SEARCHED (IPC) C12Q |
| X | US 2020/299678 A1 (JELINEK MARY ANNE [US] ET AL) 24 September 2020 (2020-09-24) * paragraph [0189] * | 22 | |

-/--

The present search report has been drawn up for all claims

| Place of search | Date of completion of the search | Examiner |
|---|---|---|
| Munich | 12 November 2024 | Ulbrecht, Matthias |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

......................................................................................

& : member of the same patent family, corresponding
document

EPO FORM 1503 03.82 (P04C01)

page 1 of 2

| | Europäisches Patentamt European Patent Office Office européen des brevets | **EUROPEAN SEARCH REPORT** | **Application Number** EP 24 17 7784 |
|---|---|---|---|

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | HANDA TETSUYA ET AL: "Chromatin integration labeling for mapping DNA-binding proteins and modifications with low input", NATURE PROTOCOLS, NATURE PUBLISHING GROUP, GB, vol. 15, no. 10, 17 August 2020 (2020-08-17), pages 3334-3360, XP037256907, ISSN: 1754-2189, DOI: 10.1038/S41596-020-0375-8 [retrieved on 2020-08-17] * table 1 * * figure 1 * | 1-22 | |
| A | JENS NIKLAS ET AL: "Selective permeabilization for the high-throughput measurement of compartmented enzyme activities in mammalian cells", ANALYTICAL BIOCHEMISTRY, ACADEMIC PRESS, AMSTERDAM, NL, vol. 416, no. 2, 24 May 2011 (2011-05-24), pages 218-227, XP028237513, ISSN: 0003-2697, DOI: 10.1016/J.AB.2011.05.039 [retrieved on 2011-05-30] * abstract * | 1,11 | |
| A | Anonymous: "Good's buffers - Wikipedia", , 26 October 2023 (2023-10-26), XP093199722, Retrieved from the Internet: URL:https://en.wikipedia.org/wiki/Good's_buffers [retrieved on 2024-08-28] * the whole document * | 1-5 | TECHNICAL FIELDS SEARCHED (IPC) |

The present search report has been drawn up for all claims

| Place of search | Date of completion of the search | Examiner |
|---|---|---|
| Munich | 12 November 2024 | Ulbrecht, Matthias |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

    .....................................................................................
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 17 7784

This annex lists the patent family members relating to the patent documents cited in the above-mentioned European search report.
The members are as contained in the European Patent Office EDP file on
The European Patent Office is in no way liable for these particulars which are merely given for the purpose of information.

12-11-2024

| Patent document cited in search report | | Publication date | Patent family member(s) | | Publication date |
|---|---|---|---|---|---|
| EP 4215618 | A1 | 26-07-2023 | CN | 115279917 A | 01-11-2022 |
| | | | EP | 4215618 A1 | 26-07-2023 |
| | | | US | 2024018512 A1 | 18-01-2024 |
| | | | WO | 2022056704 A1 | 24-03-2022 |
| US 2020299678 | A1 | 24-09-2020 | US | 2016115474 A1 | 28-04-2016 |
| | | | US | 2020299678 A1 | 24-09-2020 |
| | | | US | 2022356465 A1 | 10-11-2022 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9523875 A
- WO 2003052095 A
- WO 2000062855 A
- US 5179022 A

**Non-patent literature cited in the description**

- **COLEGIO et al.** *J. Bacteriol*, 2001, vol. 183, 2384-8
- **KIRBY C et al.** *Mol. Microbiol*, 2002, vol. 43, 173-86
- **DEVINE** ; **BOEKE**. *Nucleic Acids Res.*, 1994, vol. 22, 3765-72
- **CRAIG, N L**. *Science*, 1996, vol. 271, 1512
- **CRAIG, N L**. *Curr Top Microbiol Immunol*, 1996, vol. 204, 27-48
- **KLECKNER N et al.** *Curr Top Microbiol Immunol*, 1996, vol. 204, 49-82
- **LAMPE D J et al.** *EMBO J.*, 1996, vol. 15, 5470-9
- **PLASTERK R H**. *Curr. Topics Microbiol. Immunol*, 1996, vol. 204, 125-43
- **GLOOR, G B**. *Methods Mol. Biol*, 2004, vol. 260, 97-114
- **ICHIKAWA** ; **OHTSUBO**. *J Biol. Chem.*, 1990, vol. 265, 18829-32
- **OHTSUBO** ; **SEKINE**. *Curr. Top. Microbiol. Immunol.*, 1996, vol. 204, 1-26
- **BROWN et al.** *Proc Natl Acad Sci USA*, 1989, vol. 86, 2525-9
- **BOEKE** ; **CORCES**. *Annu Rev Microbiol.*, 1989, vol. 43, 403-34
- **ZHANG et al.** *PLoS Genet.*, 16 October 2009, vol. 5, el000689
- **WILSON C. et al.** *J. Microbiol. Methods*, 2007, vol. 71, 332-5
- **GUPTA N**. DNA Extraction and Polymerase Chain Reaction. *J Cytol.*, April 2019, vol. 36 (2), 116-117

- **PREETHA J SHETTY**. *The Evolution of DNA Extraction Methods*, 2020, vol. 8 (1)
- **ROSENBLOOM, K.R.** ; **ARMSTRONG, J.** ; **BARBER, G.P.** ; **CASPER, J.** ; **CLAWSON, H.** ; **DIEKHANS, M.** ; **DRESZER, T.R.** ; **FUJITA, P.A.** ; **GURUVADOO, L.** ; **HAEUSSLER, M.** The UCSC Genome Browser database: 2015 update. *Nucleic Acids Res.*, 2015, vol. 43, D670-D681, https://doi.org/10.1093/nar/gku1177
- **LANDT, S.G.** ; **MARINOV, G.K.** ; **KUNDAJE, A.** ; **KHERADPOUR, P.** ; **PAULI, F.** ; **BATZOGLOU, S.** ; **BERNSTEIN, B.E.** ; **BICKEL, P.** ; **BROWN, J.B.** ; **CAYTING, P.** ChIP-seq guidelines and practices of the ENCODE and modENCODE consortia. *Genome Res.*, 2012, vol. 22, 1813-1831, https://doi.org/10.1101/gr.136184.111
- **LI, H.** ; **HANDSAKER, B.** ; **WYSOKER, A.** ; **FENNELL, T.** ; **RUAN, J.** ; **HOMER, N.** ; **MARTH, G.** ; **ABECASIS, G.** ; **DURBIN, R.** 1000 Genome Project Data Processing Subgroup, 2009. The Sequence Alignment/Map format and SAMtools. *Bioinformatics*, vol. 25, 2078-2079, https://doi.org/10.1093/bioinformatics/btp352
- **SUSAMI, K.** ; **IKEDA, S.** ; **HOSHINO, Y.** ; **HONDA, S.** ; **MINAMI, N.** Genome-wide profiling of histone H3K4me3 and H3K27me3 modifications in individual blastocysts by CUT&Tag without a solid support (NON-TiE-UP CUT&Tag). *Sci. Rep.*, 2022, vol. 12, 11727